# EUROPEAN PATENT APPLICATION

(11) **EP 4 685 145 A1**
(43) Date of publication of application: **28.01.2026**
(21) Application number: 25190764.8
(22) Date of filing: 21.07.2025
(51) Int. Cl.: C07D 413/12, C07D 413/14, C07D 417/12, C07D 417/14, H10K 50/00

(54) **COMPOUND FOR ORGANIC ELECTRONIC DEVICE, ORGANIC ELECTRONIC DEVICE AND ELECTRONIC APPARATUS USING THE COMPOUND**

(30) Priority: 22.07.2024 KR 20240096553
(71) Applicant: Duk San Neolux Co., Ltd., Chungcheongnam-do 31027 (KR)
(72) Inventor: PARK, Ji Hyun, 31027 Cheonan-si, Chungcheongnam-do (KR); LEE, Se Hoon, 31027 Cheonan-si, Chungcheongnam-do (KR); LEE, Hyung Dong, 31027 Cheonan-si, Chungcheongnam-do (KR)
(74) Representative: Prüfer & Partner mbB Patentanwälte · Rechtsanwälte

(57) **Abstract**

The present invention provides a compound represented by Formula 1 and a method for recovering the compound, a material for an organic electronic device containing Formula 1 and Formula **I,** as well as an organic electronic device comprising a first electrode, a second electrode, and an organic layer between the first and second electrodes, and an electronic apparatus comprising the organic electronic device. By including a compound represented by Formula 1 or a mixture of compounds represented by Formula 1 and Formula I in the organic layer, the driving voltage of the organic electronic device can be lowered, and the luminous efficiency and lifetime of the organic electronic device can be improved.

## Description

### [TECHNICAL FIELD]

The present invention relates to a compound for an organic electronic device, an organic electronic device and an electronic apparatus comprising the compound.

### [BACKGROUND]

In general, organic electroluminescence refers to a phenomenon in which electrical energy is converted into light energy by an organic material. An organic electronic device utilizing organic electroluminescence typically includes an anode, a cathode, and an organic layer interposed therebetween. In many cases, the organic layer has a multilayered structure comprising different materials, respectively, in order to improve the efficiency and stability of an organic electronic device. For example, the organic layer may comprise a hole injection layer, a hole transport layer, an emission layer, an electron transport layer, and an electron injection layer.

The materials used in the organic layer of an organic electronic device may be classified, according to their functions, into an emissive material and charge-transporting materials such as a hole-injecting material, a hole-transporting material, an electron-transporting material, and an electron-injecting material. Further, the emissive material may be classified into a high molecular weight type and a low molecular weight type according to molecular weight, and may also be classified into a fluorescent material, which emits light from an excited singlet state, and a phosphorescent material, which emits light from an excited triplet state, according to its light-emission mechanism. Further, the emissive material may be classified, according to its emission color, into blue, green, and red emissive materials, and yellow and orange emissive materials, which are required for improved natural color reproduction.

Meanwhile, when a single material is used as an emissive material, problems may occur, such as a shift in the maximum emission wavelength toward a longer wavelength due to intermolecular interactions, a deterioration in color purity, or a reduction in luminous efficiency, thereby resulting in decreased efficiency of the corresponding device. Accordingly, a host/dopant system may be employed as the emissive material in order to enhance color purity and improve luminous efficiency through energy transfer. This is based on the principle that, when a small amount of a dopant having a smaller energy band gap than that of a host forming the emission layer is mixed into the emission layer, excitons generated in the emission layer are transferred to the dopant, thereby enabling light emission with high efficiency. Here, since the emission wavelength of the host is shifted to the wavelength region of the dopant, light having a desired wavelength can be obtained depending on the type of the dopant.

Currently, the portable display market is expanding with the adoption of large-area displays, which require greater power consumption than conventional portable displays. Accordingly, power consumption has become a critical factor for portable displays that operate with limited battery power, and issues related to efficiency and lifespan must also be addressed.

Efficiency, lifespan, driving voltage, and the like are interrelated. An increase in efficiency may lead to a decrease in driving voltage, which in turn may reduce the crystallization of organic materials caused by Joule heating generated during device operation. As a result, the lifespan of the device may be extended. However, efficiency cannot be maximized solely by improving the organic layer. This is because both long lifespan and high efficiency can be simultaneously achieved only when an optimal combination is established among the energy levels, T₁ values, and intrinsic material properties (e.g., charge mobility, interfacial characteristics, etc.) of the respective layers constituting the organic layer.

Therefore, it is necessary to develop an emitting material that exhibits high thermal stability and can efficiently achieve charge balance in the emission layer. That is, in order to fully realize the excellent characteristics of an organic electronic device, the materials constituting the organic layer, for example, hole-injecting materials, hole-transporting materials, emissive materials, electron-transporting materials, and electron-injecting materials etc. must be based on materials that are both stable and efficient. In particular, it is necessary to develop host material for an emission layer.

### [DETAILED DESCRIPTION OF THE INVENTION]

### [Technical Problem]

An object of the present invention is to provide a compound capable of lowering the driving voltage of a device and improving the luminous efficiency and lifespan of the device, an organic electronic device and an electronic apparatus employing the compound.

### [Technical Solution]

In one aspect, the present invention provides a compound represented by the following Formula 1.

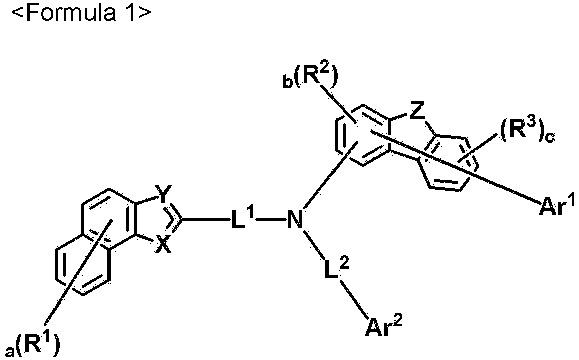

In another aspect, the present invention provides a material for an organic electronic device comprising a compound represented by Formula 1 and a compound represented by Formula I.

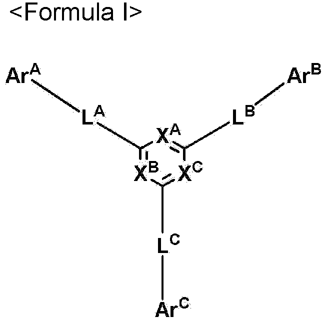

In another aspect, the present invention provides an organic electronic device comprising compound of the above Formula 1 or material for an organic electronic device containing compounds of Formula 1 and Formula I, and an electronic apparatus comprising the same.

In another aspect, the present invention provides a method for recovering the compound represented by the above Formula 1.

### [ADVANTAGEOUS EFFECTS]

By using the compound according to an embodiment of the present invention as material for an organic electronic device, the driving voltage of a device can be reduced, and the emission efficiency and lifespan can be improved.

### [BRIEF DESCRIPTION OF THE DRAWINGS]

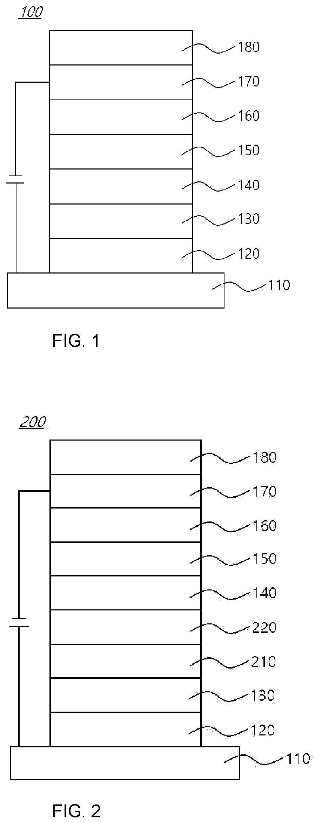

Figures 1 to 3 illustrate an example of organic electroluminescent device according to the present invention.

### [DETAILED DESCRIPTION OF THE EMBODIMENTS]

Unless otherwise stated, the term "aryl group" or "arylene group" as used herein refers to a group having 6 to 60 carbon atoms, but is not limited thereto. The aryl group or arylene group in the present invention may comprise a monocyclic ring, polycyclic and condensed ring, and the like.

As used herein, the term "fluorenyl group" refers to a fluorenyl moiety that may be substituted or unsubstituted, and the term "fluorenylene group" refers to a fluorenylene moiety that may be substituted or unsubstituted. The fluorenyl group or fluorenylene group employed in the present invention may comprise a spiro compound in which R and R' are bonded to each other in the structure shown below, and may also comprise compounds in which adjacent R" groups are linked together. The terms "substituted fluorenyl group" and "substituted fluorenylene group" mean that at least one of R, R', or R" in the following structure is a substituent other than hydrogen. In the following structure, the number of R" groups may range from 1 to 8. Throughout this specification, the fluorenyl group and fluorenylene group may collectively be referred to as a "fluorene group" or "fluorene," regardless of their valence.

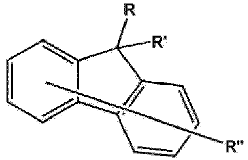

As used herein, the term "spiro compound" refers to a compound having a spiro linkage, which is a structure in which two rings are connected through a single common atom. The atom shared by the two rings is referred to as a "spiro atom," and the compound may be classified as a monospiro, dispiro, or trispiro compound depending on the number of spiro atoms present in the molecule.

As used herein, the term "heterocyclic group" comprises both aromatic rings, such as a "heteroaryl group" or a "heteroarylene group," and non-aromatic rings. Unless otherwise specified, the "heterocyclic group" refers to a ring structure containing one or more heteroatoms and having from 2 to 60 carbon atoms, but is not limited thereto. The term "heteroatom," as used herein, refers to atoms such as nitrogen (N), oxygen (O), sulfur (S), phosphorus (P), or silicon (Si), and may also include heteroatomic groups such as SO₂, P=O, and the like, which can replace a carbon atom in the ring structure as shown in the following compound.

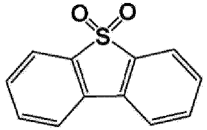

In addition, the heterocyclic group includes monocyclic, polycyclic, or condensed(fused) rings containing a heteroatom. In the case of condensed rings, if at least one of the rings in the condensed system contains a heteroatom, it is defined as a heterocycle. For example, the condensed ring systems where a heterocycle such as furan, dihydrofuran, thiophene, pyrrole, or pyridine is fused with an aromatic ring such as benzene, naphthalene, or phenanthrene, or with an alicyclic ring such as cyclopentane or cyclohexane fall under the category of heterocycles. Likewise, a spiro compound in which at least one ring contains a heteroatom is also considered heterocycle.

The term "aliphatic ring group" as used in this specification refers to a cyclic hydrocarbon excluding aromatic hydrocarbons. It includes monocyclic, polycyclic, fused ring, and spiro compounds. Unless otherwise specified, it generally refers to rings containing 3 to 60 carbon atoms, but is not limited thereto. In particular, an aliphatic ring (group) in this specification is defined as a hydrocarbon ring that does not contain any aromatic ring. Therefore, not only a saturated hydrocarbon ring such as cycloalkyl group, but also a ring containing one or more double bonds is considered aliphatic ring as long as it is not an aromatic hydrocarbon.

The term "fused ring (group)" or "condensed ring (group)" as used in the present specification refers, unless otherwise specified, to a ring formed by the fusion of an aliphatic ring and an aromatic hydrocarbon ring (aromatic ring or aryl ring), and, unless otherwise stated, it means a ring condensed of an aliphatic ring having 3 to 60 carbon atoms and an aromatic hydrocarbon having 6 to 60 carbon atoms.

In this specification, the 'group name' corresponding to an aryl group, an arylene group, a heterocyclic group, and the like, exemplified for each symbol and its substituent, may be expressed either as 'a functional group name reflecting the valence' or as 'the name of a parent compound'. For example, in the case of 'phenanthrene,' which is a type of aryl group, it may be described as 'phenanthryl' when referring to a monovalent group, and as 'phenanthrylene' when referring to a divalent group. Alternatively, it may also be described by its parent compound name 'phenanthrene,' regardless of valence. Similarly, in the case of pyrimidine, it may be referred to as 'pyrimidine' regardless of its valence. Alternatively, it may be described by the name of the corresponding functional group, such as 'pyrimidinyl' for a monovalent group and 'pyrimidinylene' for a divalent group.

In addition, in the present specification, numerical and alphabetical indicators of positions may be omitted when describing the name of a compound or a substituent. For example, compounds such as pyrido[4,3-d]pyrimidine, benzofuro[2,3-d]pyrimidine, and 9,9-dimethyl-9H-fluorene may be described in a simplified manner as pyridopyrimidine, benzofurropyrimidine, and dimethylfluorene, respectively. Accordingly, both benzo[g]quinoxaline and benzo[f]quinoxaline may be generally referred to as benzoquinoxaline.

In addition, unless otherwise specified, the definitions of substituents in the Formula used in the present invention may be applied in accordance with the definitions of the index in the following Formula.

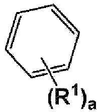

Here, when "a" is an integer of 0, it means that the substituent R¹ is absent. In other words, when a is 0, all carbon atoms forming the benzene ring are bonded to hydrogen atoms, and in this case, the hydrogen atoms bonded to the carbon atoms may be omitted in the depiction of the formula or compound. In addition, when "a" is an integer of 1, one substituent R¹ is bonded to any one of the carbon atoms forming the benzene ring. When "a" is an integer of 2 or 3, the substituents may be bonded as shown below, and when "a" is an integer from 4 to 6, the substituents are also bonded to the carbon atoms of the benzene ring in a similar manner. When "a" is an integer of 2 or more, the R¹ substituents may be the same or different from each other.

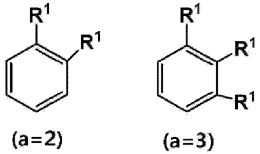

In addition, unless otherwise specified, the term "ring" as used in this specification refers to an aryl ring, a heteroaryl ring, a fluorene ring, an aliphatic ring, a fused ring, and the like. The expression "number-ring" denotes a fused(condensed) ring system, whereas "number-membered ring" may refer to the shape of the ring. For example, naphthalene corresponds to a fused ring consisting of two rings, anthracene corresponds to a fused ring consisting of three rings, thiophene and furan correspond to five-membered heterocycles, and benzene and pyridine corresponds to six-membered aromatic rings.

In addition, unless otherwise specified in the present specification, when adjacent groups are linked to each other to form a ring, the ring may be selected from the group consisting of a C₆-C₆₀ aromatic ring group, a fluorenyl group, a C₂-C₆₀ heterocyclic group containing at least one heteroatom selected from O, N, S, Si, and P, a C₃-C₆₀ aliphatic ring group, and a fused ring of a C₃-C₆₀ aliphatic ring and a C₆-C₆₀ aromatic ring. Here, the aromatic ring group may comprise an aryl ring, and the heterocyclic group may comprise a heteroaryl ring.

Unless otherwise specified, the term 'adjacent groups,' as used herein, comprises not only the relationships such as R₁ and R₂, R₂ and R₃, R₃ and R₄, and R₅ and R₆ but also R₇ and R₈ sharing a common carbon atom. It may further comprise cases substituents attached to different ring-forming atoms (e.g., carbon or nitrogen), such as R₁ and R₇, R₁ and R₈, or R₄ and R₅. That is, even when substituents are not directly adjacent on the same atom, one substituent may be considered adjacent to another substituent attached to a neighboring ring-forming atom. Additionally, substituents bonded to the same carbon atom forming the ring may also be regarded as adjacent groups. In the following Formula, when substituents such as R₇ and R₈, which are bonded to the same carbon atom, are connected to form a ring, a compound containing a spiro moiety may be generated.

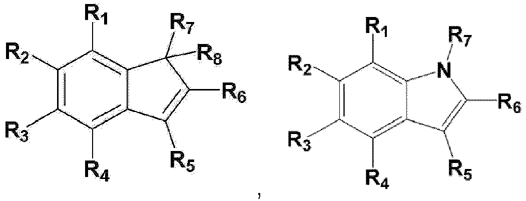

In addition, in the present specification, the expression 'adjacent groups may be linked to each other to form a ring' is used in the same sense as 'adjacent groups are selectively linked to each other to form a ring,' and refers to a case where at least one pair of adjacent groups may be bonded to form a ring structure.

In addition, in this specification, the phrase "adjacent(neighboring) groups can bond to each other to form a ring" means that the adjacent(neighboring) groups can ultimately form a ring, and it does not necessarily assume that substituents such as R¹ and R² contain unsaturated bonds like alkenyl or alkynyl groups.

In addition, unless otherwise specified in the present specification, substituents such as an aryl group, an arylene group, a fluorenyl group, a fluorenylene group, a heterocyclic group, an aliphatic ring group, an alkyl group, an alkenyl group, an alkynyl group, an alkoxyl group, an aryloxyl group, alkylthio group, arylthio group, etc., and a ring formed by adjacent groups may be each optionally substituted with one or more substituents selected from the group consisting of deuterium, halogen, a cyano group, a nitro group, siloxane group, a C₆-C₃₀ aryl group, a fluorenyl group, a C₂-C₃₀ heterocyclic group containing at least one heteroatom of O, N, S, Si and P, a C₃-C₃₀ aliphatic ring group, a fused ring of a C₃-C₆₀ aliphatic ring and a C₆-C₆₀ aromatic ring, a C₁-C₂₀ alkyl group, a C₂-C₂₀ alkenyl group, a C₂-C₂₀ alkynyl group, a C₁-C₂₀ alkoxyl group, a C₆-C₂₀ aryloxy group, a C₁-C₂₀ alkylthio group, a C₆-C₂₀ arylthio group, a silane group unsubstituted or substituted with a C₁-C₂₀ alkyl group or a C₆-C₂₀ aryl group, and a phosphine oxide group unsubstituted or substituted with a C₁-C₂₀ alkyl group or a C₆-C₂₀ aryl group.

Unless otherwise specified in this specification, the symbols "*" or " 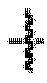 " represent the bonding sites.

Hereinafter, with reference to Figures 1 to 3, the laminated structure of an organic electronic device comprising the compound according to the present invention will be described.

In the designation of reference numerals for components in the respective drawings, it should be understood that the same elements are denoted by the same reference numerals, even if they appear in different drawings. Furthermore, in the following description of the present invention, detailed explanations of well-known functions and configurations will be omitted where they may unnecessarily obscure the essence of the invention.

Terms such as "first," "second," "A," "B," "(a)," "(b)," and the like may be used to describe various components of the present invention. These terms are merely intended to distinguish one component from another and do not imply any particular order, importance, or essential characteristics. Furthermore, it should be understood that when a component is described as being "connected," "coupled," or "joined" to another component, this may include both direct connections as well as indirect connections through one or more intervening components.

Additionally, it is to be understood that when an element such as a layer, film, region, or substrate is described as being "on" or "over" another element, it may be positioned directly on the other element or with one or more intervening layers therebetween. In contrast, the expression "directly on" indicates that no intervening elements are present between the two elements.

Figures 1 to 3 respectively illustrate examples of an organic electronic device according to embodiments of the present invention.

Referring to FIG. 1, an organic electronic device 100 according to an embodiment of the present invention comprises a first electrode 110, a second electrode 170, and an organic layer formed between the first electrode 110 and the second electrode 170 on a substrate (not shown), and an inorganic layer may also be comprised between the first electrode 110 and the second electrode 170.

For example, the first electrode 110 may be an anode (positive electrode), and the second electrode 170 may be a cathode (negative electrode). In an inverted organic electronic device, however, the first electrode may be a cathode, while the second electrode may be an anode.

The organic layer refers to a layer comprising at least one organic material. For example, the organic layer may include a hole injection layer 120, a hole transport layer 130, an emission layer 140, an electron transport layer 150, and an electron injection layer 160. In certain embodiments, the electron injection layer 160 may be an inorganic layer that does not contain any organic material.

Specifically, a hole injection layer 120, a hole transport layer 130, an emission layer 140, an electron transport layer 150, and an electron injection layer 160 may be sequentially formed on the first electrode 110.

Preferably, a layer for improving the luminous efficiency 180 may be formed on one side of either the first electrode 110 or the second electrode 170, which does not face the organic layer or the inorganic layer. When the layer for improving the luminous efficiency 180 is formed, the luminous efficiency of the organic electronic device can be enhanced.

For example, when the layer for improving the luminous efficiency 180 may be formed on the second electrode 170, in the case of a top-emission organic electroluminescent device, optical energy loss due to surface plasmon polaritons (SPPs) at the second electrode 170 may be reduced, and in the case of a bottom-emission organic electroluminescent device, the layer for improving the luminous efficiency 180 may function as a buffer layer for the second electrode 170.

A buffer layer 210 or an emission-auxiliary layer 220 may additionally be formed between the hole transport layer 130 and the emission layer 140, as will be described with reference to FIG. 2.

Referring to FIG. 2, an organic electronic device 200 according to another embodiment of the present invention may sequentially include a hole injection layer 120, a hole transport layer 130, a buffer layer 210, an emission-auxiliary layer 220, an emission layer 140, an electron transport layer 150, an electron injection layer 160, and a second electrode 170 on a first electrode 110, and a layer for improving the luminous efficiency 180 may be formed on the second electrode.

Although not illustrated in FIG. 2, an electron transport auxiliary layer may additionally be formed between the emission layer 140 and the electron transport layer 150.

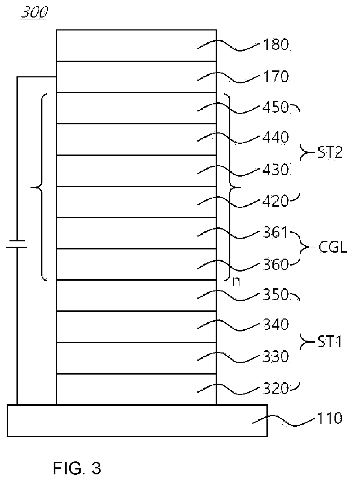

In addition, according to another embodiment of the present invention, an organic layer may be in the form of a plurality of stacks, each including a hole transport layer, an emission layer, and an electron transport layer. This will be described with reference to FIG. 3.

Referring to FIG. 3, an organic electronic device 300 according to another embodiment of the present invention may include two or more sets of stacks (ST1, ST2) of organic layers formed in multiple layers between the first electrode 110 and the second electrode 170, and a charge generation layer (CGL) may be formed between the stacks of the organic layers.

Specifically, the organic electronic device according to the embodiment of the present invention may comprise a first electrode 110, a first stack ST1, a charge generation layer CGL, a second stack ST2, and a second electrode 170 and a layer for improving light efficiency 180.

The first stack ST1 is an organic layer formed on the first electrode 110, and may comprise a first hole injection layer 320, a first hole transport layer 330, a first emission layer 340, and a first electron transport layer 350. The second stack ST2 may comprise a second hole injection layer 420, a second hole transport layer 430, a second emission layer 440, and a second electron transport layer 450. As such, the first stack and the second stack may have the same or different stacked structures of organic layers.

The charge generation layer CGL may be formed between the first stack ST1 and the second stack ST2. The charge generation layer CGL may comprise a first charge generation layer 360 and a second charge generation layer 361. It is formed between the first emission layer 340 and the second emission layer 440 to enhance the current efficiency of each emission layer and facilitate charge distribution.

The first emission layer 340 may comprise an emissive material that comprises a blue host doped with a blue fluorescent dopant, and the second emission layer 440 may comprise an emissive material that comprises a green host doped with both a greenishyellow dopant and a red dopant. However, the materials of the first emission layer 340 and the second emission layer 440 according to an embodiment of the present invention are not limited thereto.

In FIG. 3, n may be an integer from 1 to 5, and when n is 2, a charge generation layer (CGL) and a third stack may be additionally formed on the second stack ST2.

When a plurality of emission layers are formed in a multi-layer stack structure as shown in FIG. 3, it is possible to manufacture an organic electroluminescent element that emits not only white light but also various colors, where the white light is produced by the mixing of light emitted from each emission layer.

Compound represented by Formula 1 or a mixture of the compound of Formula 1 and the compound Formula I of the present invention may be included in an organic layer. For example, the compound represented by Formula 1 or a mixture of the compound of Formula 1 and the compound Formula I of the present invention can be used as a material for a hole injection layer 120, 320, 420, a hole transport layer 130, 330, 430, a buffer layer 210, an emission-auxiliary layer 220, an electron transport layer 150, 350, 450, an emission layer 140, 340, 440, and/or a light efficiency improving layer 180, more preferably, as a host material in the emission layers 140, 340, or 440.

Even if the cores of the compounds are identical or similar, their band gaps, electronic properties, and interfacial characteristics may vary depending on which substituents are bonded and at which positions. Therefore, it is necessary to study the selection of the core structure and the combination with sub-substituents attached to the core. In particular, both long lifespan and high efficiency can be achieved simultaneously when the optimal combination of energy levels, T1 values, and intrinsic material properties (such as mobility and interfacial characteristics) is realized among the layers of the organic structure.

Therefore, by using the compound represented by Formula 1 or a mixture of the compound of Formula 1 and the compound Formula I as a material for the emission layers 140, 340, and 440, it is possible to optimize the energy levels and T1 values, intrinsic material properties (such as mobility and interfacial characteristics) between the respective layers of the organic layer, as a result, the lifetime and efficiency of an organic electronic device can be simultaneously improved.

The organic electronic device according to an embodiment of the present invention may be fabricated using various deposition methods, comprising physical vapor deposition (PVD) or chemical vapor deposition (CVD). For example, the organic electronic device may be manufactured by forming the anode 110 on the substrate by depositing a metal, a conductive metal oxide, or a mixture thereof, then forming an organic layer comprising the hole injection layer 120, the hole transport layer 130, the emission layer 140, the electron transport layer 150, and the electron injection layer 160 thereon, and finally depositing a material that can be used as the cathode 170. In addition, an emission-auxiliary layer 220 may be formed between the hole transport layer 130 and the emission layer 140, and an electron transport auxiliary layer (not shown) may additionally be formed between the emission layer 140 and the electron transport layer 150. As described above, the organic layer may be formed in a stacked structure.

In addition, the organic layer may be manufactured with fewer layers by using various polymer materials through a solution process or solvent-based process, such as spin coating, nozzle printing, inkjet printing, slot coating, dip coating, roll-to-roll, doctor blading, screen printing, or thermal transfer, instead of deposition. Since the organic layer according to the present invention may be formed in various ways, the scope of protection of the present invention is not limited by the method of forming the organic layer.

The organic electronic device according to an embodiment of the present invention may be a top-emission type, a bottom-emission type, or a dual-emission type, depending on the materials used.

In addition, the organic electronic device according to an embodiment of the present invention may be selected from the group consisting of an organic electroluminescent device, an organic solar cell, an organic photoconductor, an organic transistor, a monochromatic illumination device, and a quantum dot display device.

Another embodiment of the present invention provides an electronic apparatus comprising a display device including the above-described organic electronic device and a control unit for controlling the display device. The electronic apparatus may be a wired or wireless communication terminal currently in use or to be developed in the future, and comprises all types of electronic devices, such as mobile communication terminals (e.g., cellular phones), navigation units, game players, various types of TVs, and computers.

Hereinafter, a compound according to one aspect of the present invention will be described.

A compound according to one aspect of the present invention is represented by Formula 1 below.

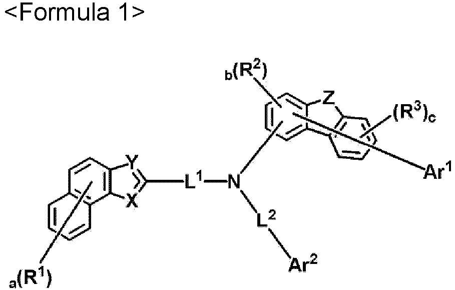

In Formula 1, each symbol may be defined as follows.

One of X and Y is N, and the other is O or S.

Z is O or S.

Ar¹ and Ar² are each independently selected from the group consisting of a C₆-C₆₀ aryl group, a fluorenyl group, C₂-C₆₀ heterocyclic group containing at least one heteroatom of O, N, S, Si and P, a C₃-C₆₀ aliphatic ring group, a fused ring of a C₆-C₆₀ aromatic ring and a C₃-C₆₀ aliphatic ring, and a C₁-C₂₀ alkyl group.

R¹ to R³ are each independently selected from the group consisting of hydrogen, deuterium, halogen, a cyano group, a nitro group, a C₆-C₆₀ aryl group, a fluorenyl group, a C₂-C₆₀ heterocyclic group containing at least one heteroatom of O, N, S, Si and P, a C₃-C₆₀ aliphatic ring group, a fused ring of a C₆-C₆₀ aromatic ring and a C₃-C₆₀ aliphatic ring, a C₁-C₂₀ alkyl group, a C₂-C₂₀ alkenyl group, a C₂-C₂₀ alkynyl group, a C₁-C₂₀ alkoxyl group, and a C₆-C₆₀ aryloxy group, and adjacent groups may be bonded to each other to form a ring.

When adjacent groups, for example, adjacent R¹s, adjacent R²s, or adjacent R³s are bonded to each other to form a ring, the ring may be selected from the group consisting of a C₆-C₆₀ aromatic ring group, a fluorenylene group, a C₃-C₆₀ aliphatic ring, a C₂-C₆₀ heterocyclic group containing at least one heteroatom of O, N, S, Si and P, and a fused ring of a C₃-C₆₀ aliphatic ring and a C₆-C₆₀ aromatic ring.

When an aromatic ring is formed by adjacent groups, the aromatic ring may be, for example, a C₆-C₂₀, a C₆-C₁₈, a C₆-C₁₆, a C₆-C₁₄, a C₆-C₁₃, a C₆-C₁₂, a C₆-C₁₀, a C₆, a C₁₀, a C₁₂, a C₁₄, a C₁₅, a C₁₆, or a C₁₈ aromatic ring, specifically, benzene, naphthalene, anthracene, phenanthrene, pyrene, etc.
a is an integer of 0 to 6, b is an integer of 0 to 3, c is an integer of 0 to 4, when they are integers of 2 or more, each of R¹, each of R² each of R³ are the same or different from each other.

L¹ is selected from the group consisting of a C₆-C₆₀ arylene group, a fluorenylene group, a C₂-C₆₀ heterocyclic group containing at least one heteroatom of O, N, S, Si and P, a C₃-C₆₀ aliphatic ring group, and a fused ring of a C₆-C₆₀ aromatic ring and a C₃-C₆₀ aliphatic ring.

L² is selected from the group consisting of a single bond, a C₆-C₆₀ arylene group, a fluorenylene group, a C₂-C₆₀ heterocyclic group containing at least one heteroatom of O, N, S, Si and P, a C₃-C₆₀ aliphatic ring group, and a fused ring of a C₆-C₆₀ aromatic ring and a C₃-C₆₀ aliphatic ring.

When at least one of Ar¹, Ar², R¹ to R³ is an aryl group, or at least one of L¹ and L² is an arylene group, the aryl group or the arylene group may be, for example, a C₆-C₃₀, a C₆-C₂₉, a C₆-C₂₈, a C₆-C₂₇, a C₆-C₂₆, a C₆-C₂₅, a C₆-C₂₄, a C₆-C₂₃, a C₆-C₂₂, a C₆-C₂₁, a C₆-C₂₀, a C₆-C₁₉, a C₆-C₁₈, a C₆-C₁₇, a C₆-C₁₆, a C₆-C₁₅, a C₆-C₁₄, a C₆-C₁₃, a C₆-C₁₂, a C₆-C₁₁, a C₆-C₁₀, a C₆, a C₁₀, a C₁₂, a C₁₃, a C₁₄, a C₁₅, a C₁₆, a C₁₇, a C₁₈, a C₁₉, a C₂₀, a C₂₁, a C₂₂, a C₂₃, a C₂₄, a C₂₅, a C₂₆, a C₂₇, a C₂₈, a C₂₉, a C₃₀ aryl group or arylene group, specifically, phenyl, biphenyl, naphthyl, terphenyl, phenanthrene, benzophenanthrene, triphenylene, chrysene, etc.

When at least one of Ar¹, Ar², R¹ to R³, L¹ and L² is a heterocyclic group, the heterocyclic group may be, for example, a C₂-C₃₀, a C₂-C₂₉, a C₂-C₂₈, a C₂-C₂₇, a C₂-C₂₆, a C₂-C₂₅, a C₂-C₂₄, a C₂-C₂₃, a C₂-C₂₂, a C₂-C₂₁, a C₂-C₂₀, a C₂-C₁₉, a C₂-C₁₈, a C₂-C₁₇, a C₂-C₁₆, a C₂-C₁₅, a C₂-C₁₄, a C₂-C₁₃, a C₂-C₁₂, a C₂-C₁₁, a C₂-C₁₀, a C₂-C₉, a C₂-C₈, a C₂-C₇, a C₂-C₆, a C₂-C₅, a C₂-C₄, a C₂-C₃, a C₂, a C₃, a C₄, a C₅, a C₆, a C₇, a C₈, a C₉, a C₁₀, a C₁₁, a C₁₂, a C₁₃, a C₁₄, a C₁₅, a C₁₆, a C₁₇, a C₁₈, a C₁₉, a C₂₀, a C₂₁, a C₂₂, a C₂₃, a C₂₄, a C₂₅, a C₂₆, a C₂₇, a C₂₈, a C₂₉ or a C₃₀ heterocyclic group, specifically, pyridine, pyrimidine, pyrazine, pyridazine, triazine, furan, pyrrole, indene, indole, phenyl-indole, benzoindole, phenyl-benzoindole, pyrazinoindol, quinoline, isoquinoline, benzoquinoline, pyridoquinoline, quinazoline, benzoquinazoline, dibenzoquinazoline, phenanthroquinazoline, quinoxaline, benzoquinoxaline, dibenzoquinoxaline, benzofuran, naphthobenzofuran, dibenzofuran, dinaphthofuran, phenanthrobenzofuran, thiophene, benzothiophene, dibenzothiophene, naphthobenzothiophene, dinaphthothiophene, phenanthrobenzothiophene, carbazole, phenyl-carbazole, benzocarbazole, phenylbenzocarbazole, naphthyl-benzocarbazole, dibenzocarbazole, indolocarbazole, benzofuropyridine, benzothienopyridine, benzofuropyridine, benzothienopyrimidine, benzofuropyrimidine, benzothienopyrazine, benzofuropyrazine, benzoimidazole, benzothiazole, benzosiloe, phenanthroline, dihydro-phenylphenazine, 10-phenyl-10H-phenoxazine, phenoxazine, phenothiazine, dibenzodioxin, benzodibenzodioxin, thianthrene, oxazole, benzooxazole, naphthooxazole, phenanthrooxazole, dibenzothienobenzoxazole, dibenzofurobenzoxazole, 9,9-dimethyl-9H-xanthene, 9,9-dimethyl-9H-thioxanthene, dihydrodimethylphenylacridine, spiro[fluorene-9,9'-xanthene] and the like.

When at least one of R¹ to R³, Ar¹ is an alkyl group, the alkyl group may be, for example, a C₁-C₂₀, a C₁-C₁₀, a C₁-C₄, a C₁, a C₂, a C₃, or a C₄ alkyl group, for example, methyl, ethyl, propyl group, isopropyl group, butyl group, t-butyl, etc.

The aryl group, the arylene group, the fluorenyl group, the fluorenylene group, the heterocyclic group, the aliphatic ring group, the fused ring, the alkyl group, the alkenyl group, the alkynyl group, the alkoxyl group, the aryloxyl group, and the ring formed by adjacent groups may be each substituted with one or more substituents selected from the group consisting of deuterium, halogen, a silane group unsubstituted or substituted with a C₁-C₂₀ alkyl group or a C₆-C₂₀ aryl group, a phosphine oxide substituted or unsubstituted with a C₁-C₂₀ alkyl group or a C₆-C₂₀ aryl group, a cyano group, a nitro group, C₁-C₂₀ alkylthio group, C₁-C₂₀ alkoxy group, C₆-C₃₀ aryloxy group, C₆-C₃₀ arylthio group, a C₁-C₂₀ alkyl group, a C₂-C₂₀ alkenyl group, a C₂-C₂₀ alkynyl group, a C₆-C₃₀ aryl group, a C₆-C₃₀ aryl group substituted with deuterium, a C₃-C₃₀ aliphatic ring, a fused ring of a C₆-C₃₀ aromatic ring and a C₃-C₃₀ aliphatic ring, and a C₂-C₃₀ heterocyclic group comprising at least one heteroatom selected from the group consisting of O, N, S, Si and P, the adjacent substituents may be bonded to each other to form a ring, and hydrogen of the substituents may be replaced with deuterium.

When at least one of the aryl group, the arylene group, the fluorenyl group, the fluorenylene group, the heterocyclic group, the aliphatic ring group, the fused ring, the alkyl group, the alkenyl group, the alkynyl group, the alkoxyl group, the aryloxyl group, and the ring formed by adjacent groups is substituted with an aryl group, the aryl group may be, for example, a C₆-C₃₀, a C₆-C₂₉, a C₆-C₂₈, a C₆-C₂₇, a C₆-C₂₆, a C₆-C₂₅, a C₆-C₂₄, a C₆-C₂₃, a C₆-C₂₂, a C₆-C₂₁, a C₆-C₂₀, a C₆-C₁₉, a C₆-C₁₈, a C₆-C₁₇, a C₆-C₁₆, a C₆-C₁₅, a C₆-C₁₄, a C₆-C₁₃, a C₆-C₁₂, a C₆-C₁₁, a C₆-C₁₀, a C₆, a C₁₀, a C₁₂, a C₁₃, a C₁₄, a C₁₅, a C₁₆, a C₁₇, or a C₁₈, a C₁₉, a C₂₀, a C₂₁, a C₂₂, a C₂₃, a C₂₄, a C₂₅, a C₂₆, a C₂₇, a C₂₈, a C₂₉ or a C₃₀ aryl group.

When at least one of the aryl group, the arylene group, the fluorenyl group, the fluorenylene group, the heterocyclic group, the aliphatic ring group, the fused ring, the alkyl group, the alkenyl group, the alkynyl group, the alkoxyl group, the aryloxyl group, and the ring formed by adjacent groups is substituted with a heterocyclic group, the heterocyclic group may be, for example, a C₂-C₃₀, a C₂-C₂₉, a C₂-C₂₈, a C₂-C₂₇, a C₂-C₂₆, a C₂-C₂₅, a C₂-C₂₄, a C₂-C₂₃, a C₂-C₂₂, a C₂-C₂₁, a C₂-C₂₀, a C₂-C₁₉, a C₂-C₁₈, a C₂-C₁₇, a C₂-C₁₆, a C₂-C₁₅, a C₂-C₁₄, a C₂-C₁₃, a C₂-C₁₂, a C₂-C₁₁, a C₂-C₁₀, a C₂-C₉, a C₂-C₈, a C₂-C₇, a C₂-C₆, a C₂-C₅, a C₂-C₄, a C₂-C₃, a C₂, a C₃, a C₄, a C₅, a C₆, a C₇, a C₈, a C₉, a C₁₀, a C₁₁, a C₁₂, a C₁₃, a C₁₄, a C₁₅, a C₁₆, a C₁₇, a C₁₈, a C₁₉, a C₂₀, a C₂₁, a C₂₂, a C₂₃, a C₂₄, a C₂₅, a C₂₆, a C₂₇, a C₂₈, a C₂₉ or a C₃₀ heterocyclic group.

Formula 1 may be represented by one of Formula 1-1 to Formula 1-34 below.

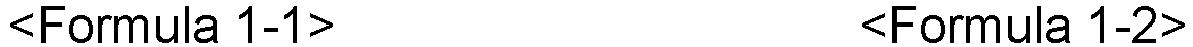

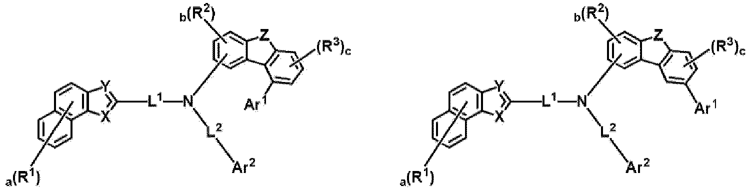

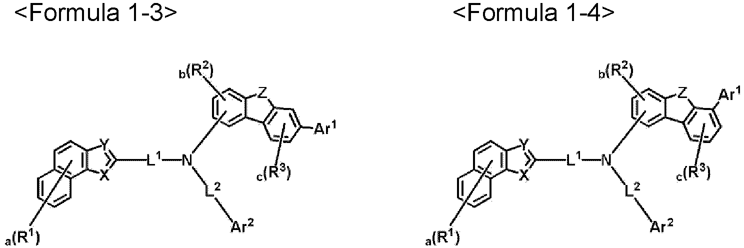

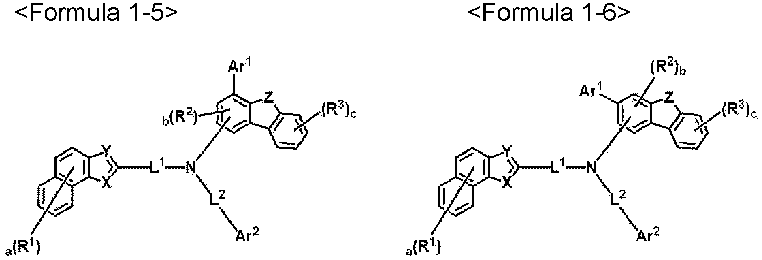

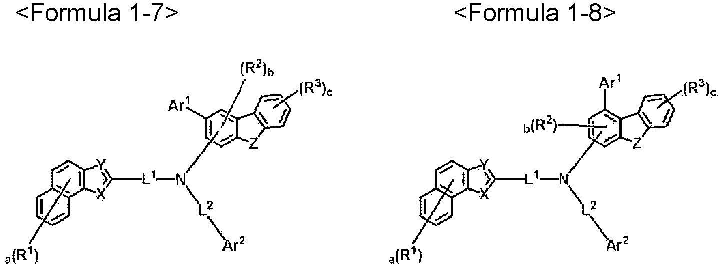

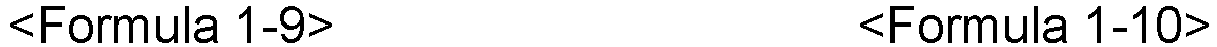

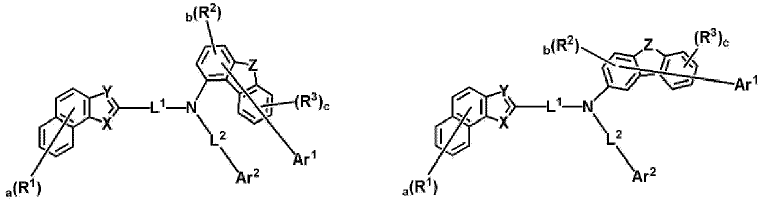

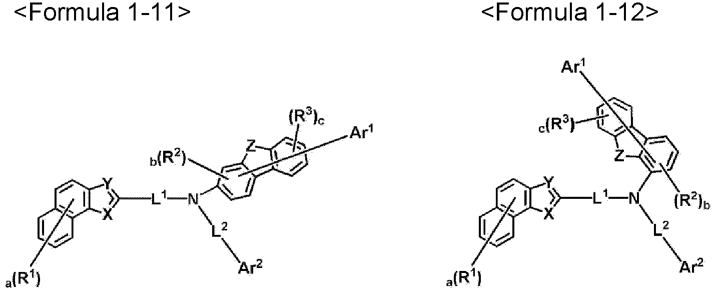

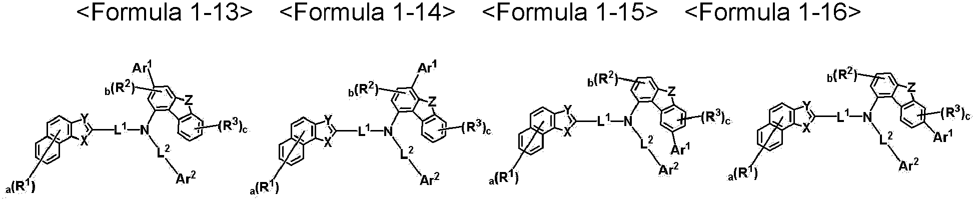

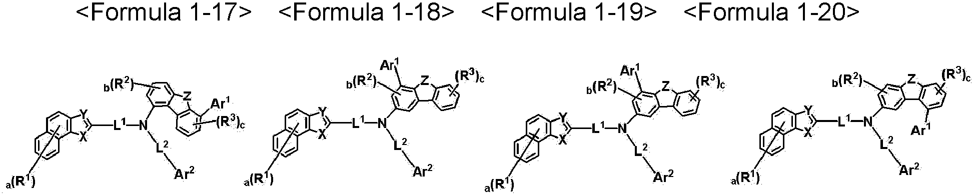

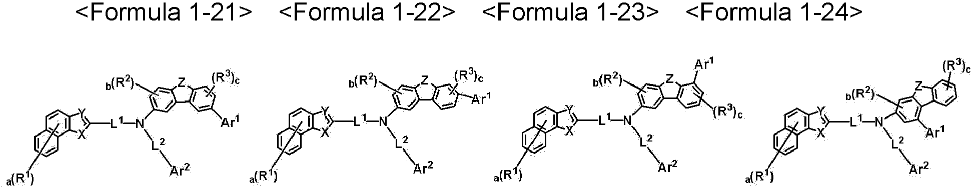

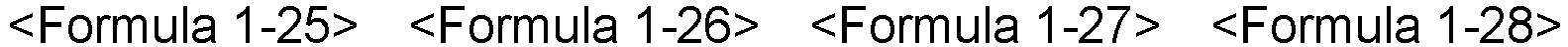

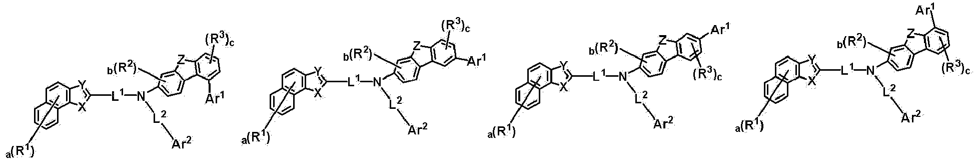

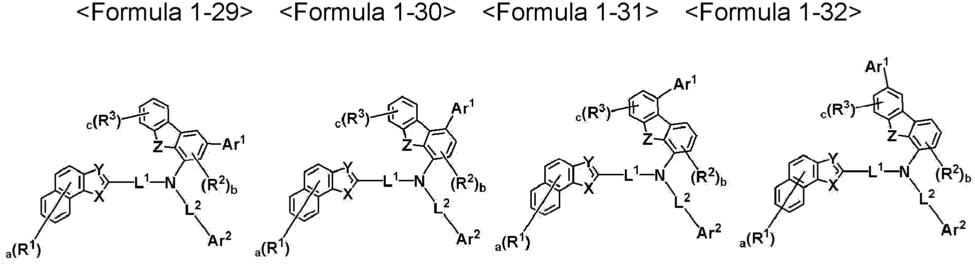

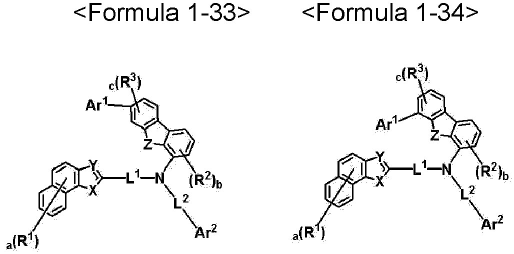

In Formula 1-1 to Formula 1-34, X, Y, Z, R¹ to R³, L¹, L², Ar¹, Ar², a, b and c are the same as defined for Formula 1. With the proviso that in Formula 1-5 to Formula 1-8, Formula 13, Formula 14, Formula 18, Formula 19, Formula 24, Formula 29, Formula 30, Formula 34, b is an integer of 0 to 2, and in Formula 1-1 to Formula 1-4, Formula 15 to Formula 17, Formula 20 to Formula 23, Formula 25 to Formula 28, Formula 31 to Formula 33, c is an integer of 0 to 3.

At least one of L¹ and L² may be selected from the group consisting of the following Formula a-1 to Formula a-12, but is not limited thereto.

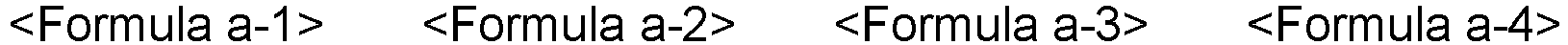

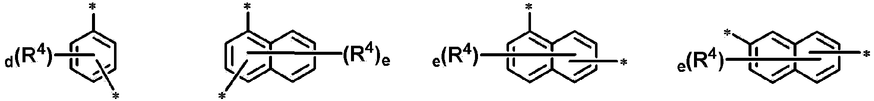

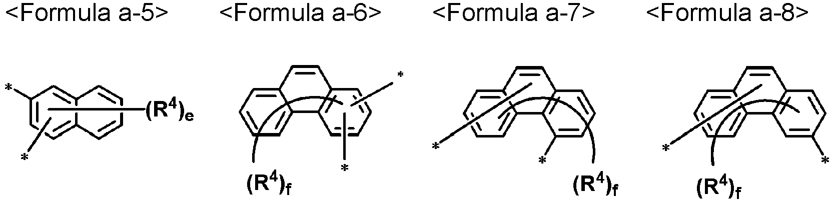

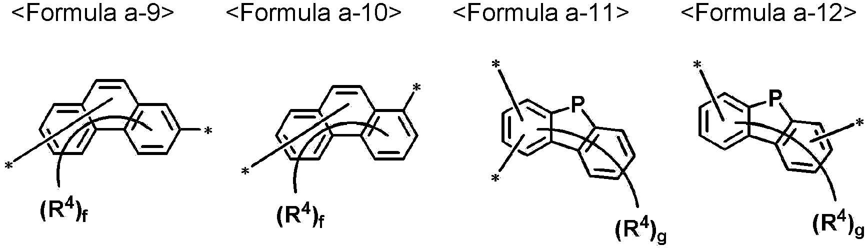

In Formula a-1 to Formula a-12, each symbol may be defined as follows.

P is O or S.

R⁴ is selected from the group consisting of hydrogen, deuterium, halogen, a silane group unsubstituted or substituted with a C₁-C₂₀ alkyl group or a C₆-C₂₀ aryl group, a phosphine oxide substituted or unsubstituted with a C₁-C₂₀ alkyl group or a C₆-C₂₀ aryl group, a cyano group, a nitro group, C₁-C₂₀ alkylthio group, C₁-C₂₀ alkoxy group, C₆-C₃₀ aryloxy group, C₆-C₃₀ arylthio group, a C₁-C₂₀ alkyl group, a C₂-C₂₀ alkenyl group, a C₂-C₂₀ alkynyl group, a C₆-C₃₀ aryl group, a C₆-C₃₀ aryl group substituted with deuterium, a C₃-C₃₀ aliphatic ring, a fused ring of a C₆-C₃₀ aromatic ring and a C₃-C₃₀ aliphatic ring, and a C₂-C₃₀ heterocyclic group comprising at least one heteroatom selected from the group consisting of O, N, S, Si and P, the adjacent groups may be bonded to each other to form a ring.

d is an integer of 0 to 4, e and g are each an integer of 0 to 6, f is an integer of 0 to 8, and when they are integers of 2 or more, each of the plurality of R⁴ groups may be the same or different from each other.

At least one of Ar¹ and Ar² may be selected from the group consisting of Formulae b-1 to b-11, but is not limited thereto.

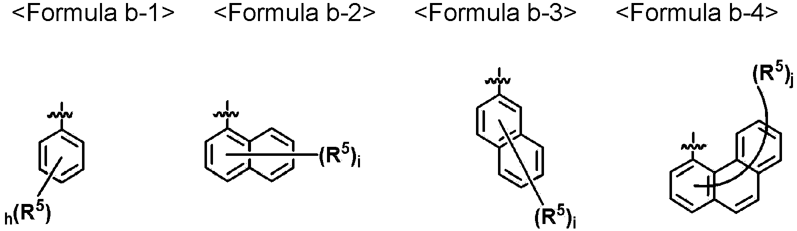

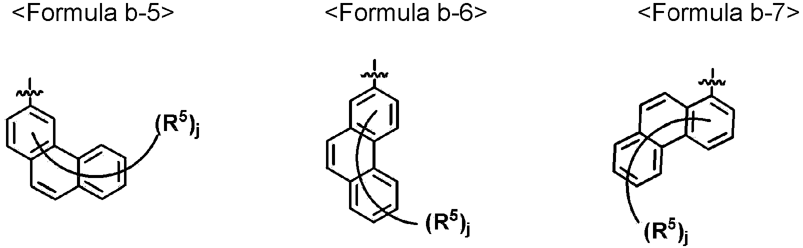

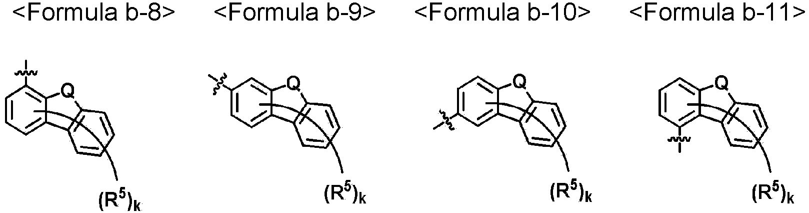

In Formula b-1 to Formula b-11, each symbol may be defined as follows.

Q is O or S.

R⁵ is selected from the group consisting of hydrogen, deuterium, halogen, a silane group unsubstituted or substituted with a C₁-C₂₀ alkyl group or a C₆-C₂₀ aryl group, a phosphine oxide substituted or unsubstituted with a C₁-C₂₀ alkyl group or a C₆-C₂₀ aryl group, a cyano group, a nitro group, C₁-C₂₀ alkylthio group, C₁-C₂₀ alkoxy group, C₆-C₃₀ aryloxy group, C₆-C₃₀ arylthio group, a C₁-C₂₀ alkyl group, a C₂-C₂₀ alkenyl group, a C₂-C₂₀ alkynyl group, a C₆-C₃₀ aryl group, a C₆-C₃₀ aryl group substituted with deuterium, a C₃-C₃₀ aliphatic ring, a fused ring of a C₆-C₃₀ aromatic ring and a C₃-C₃₀ aliphatic ring, and a C₂-C₃₀ heterocyclic group comprising at least one heteroatom selected from the group consisting of O, N, S, Si and P, the adjacent groups may be bonded to each other to form a ring.

h is an integer of 0 to 5, i and k are each an integer of 0 to 7, j is an integer of 0 to 9, and when they are integers of 2 or more, each of the plurality of R⁵ groups may be the same or different from each other.

Specifically, the compound represented by Formula 1 may be one of the following compounds, but is not imited thereto.

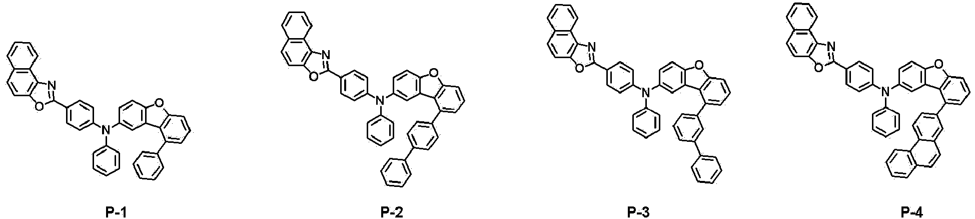

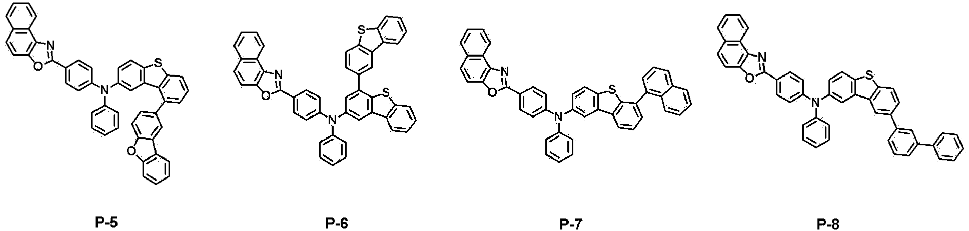

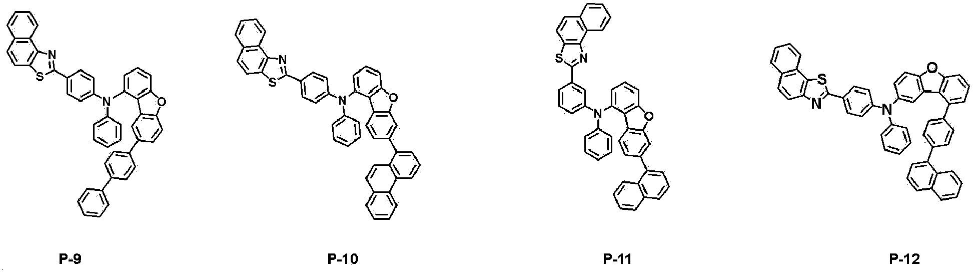

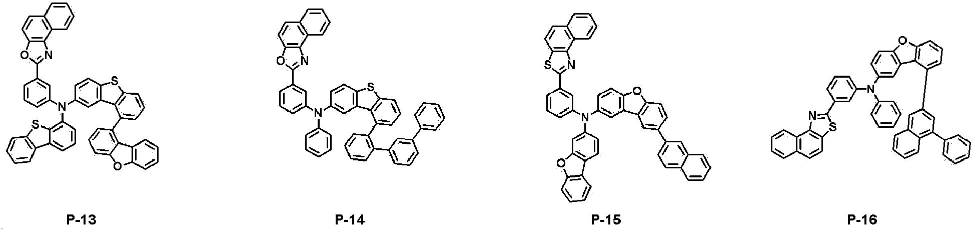

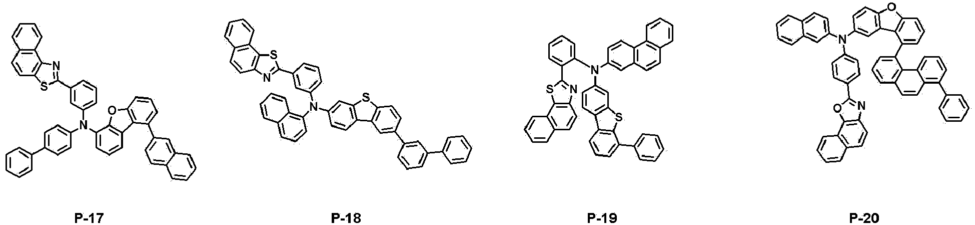

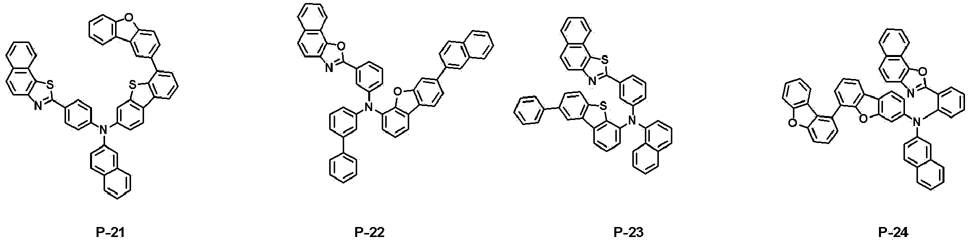

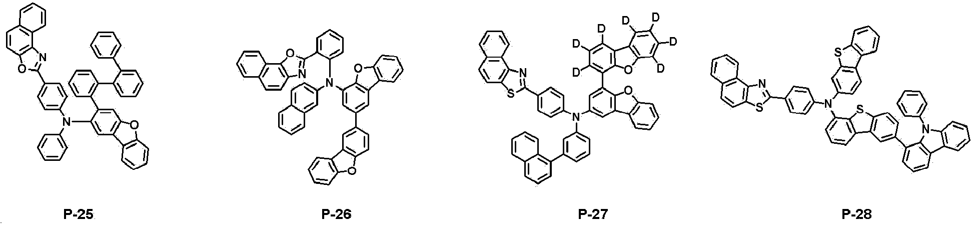

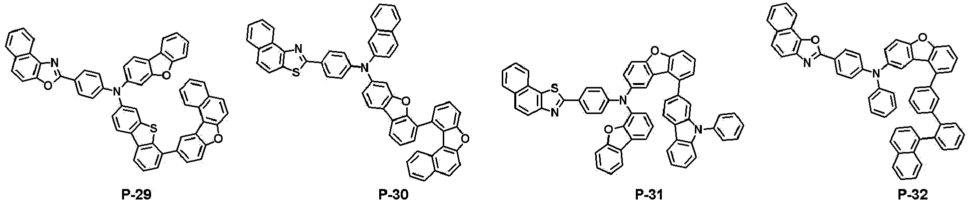

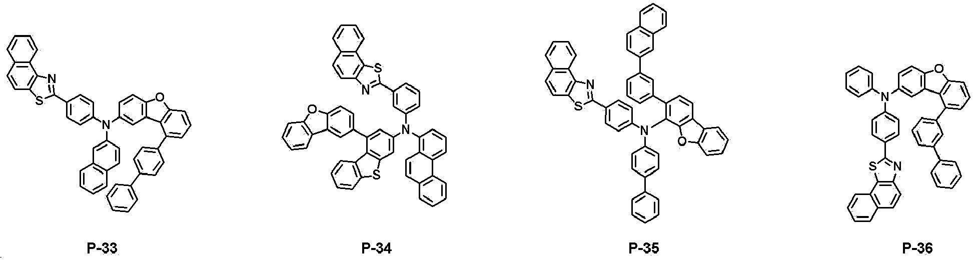

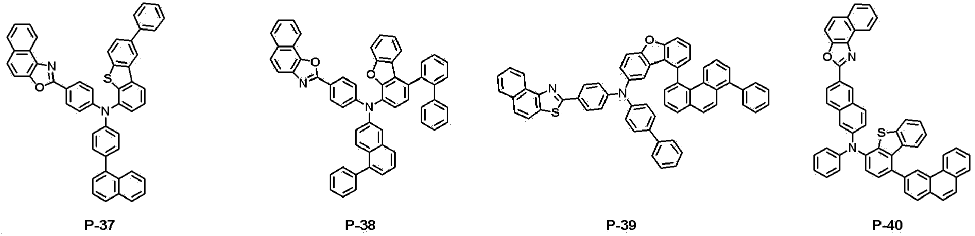

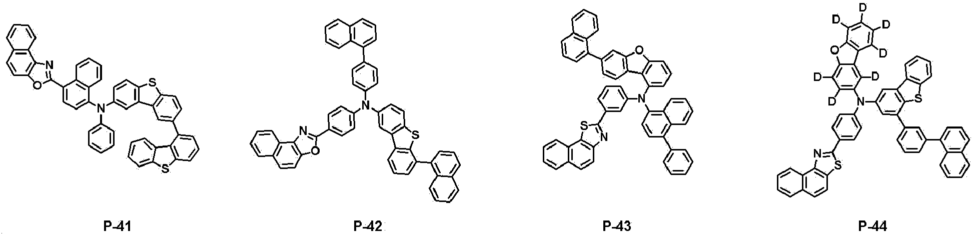

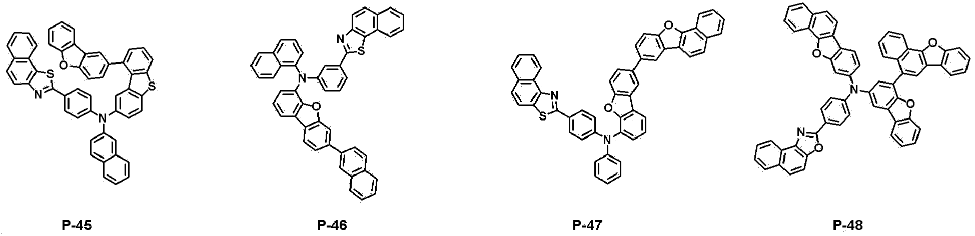

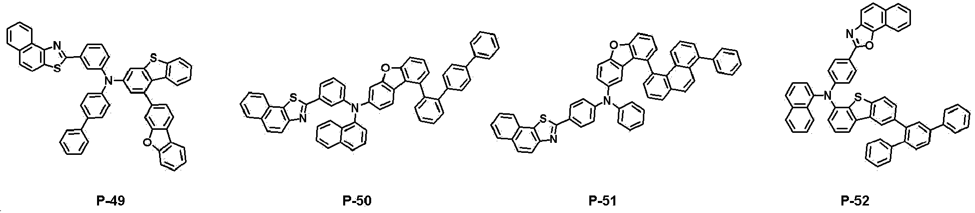

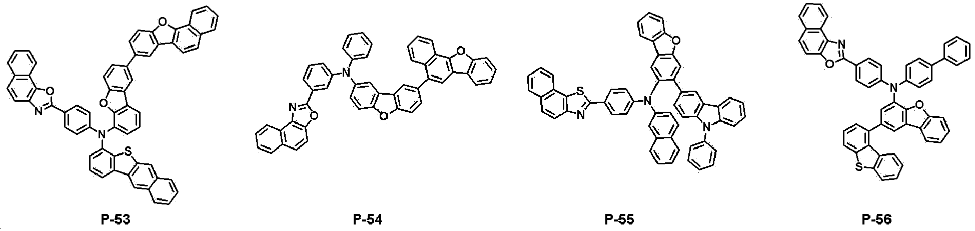

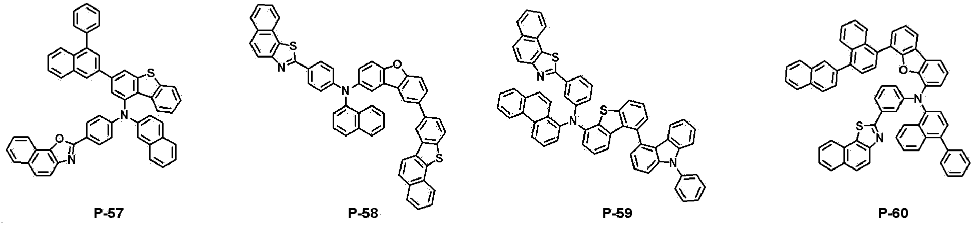

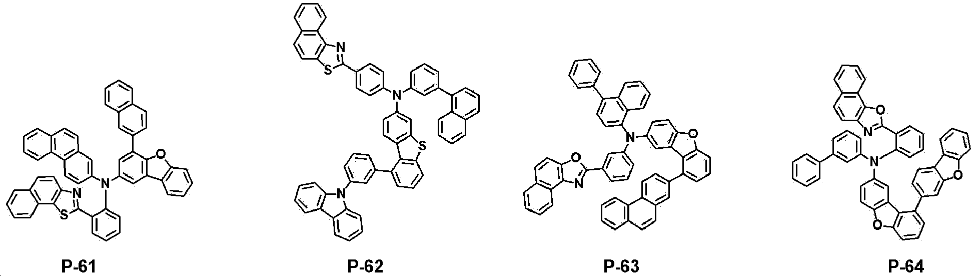

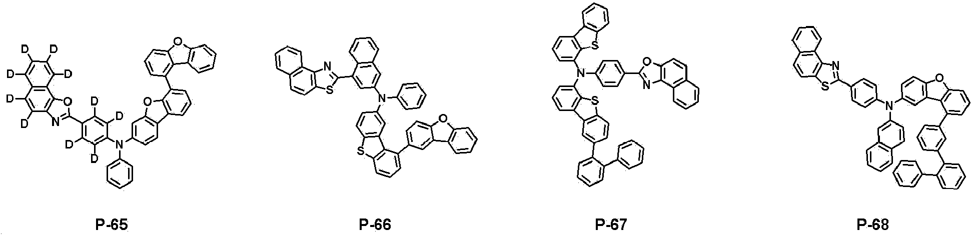

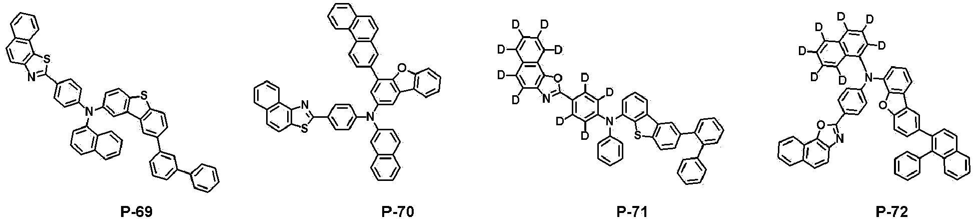

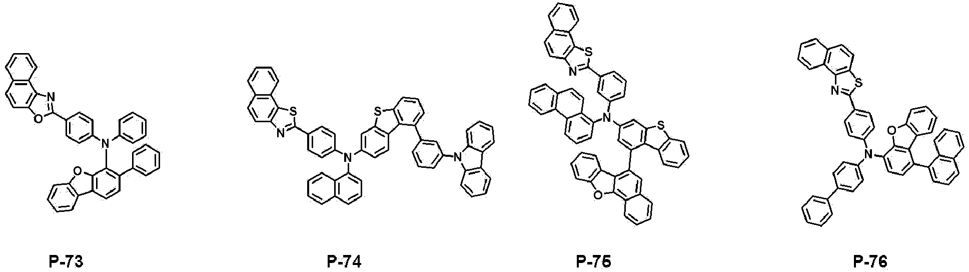

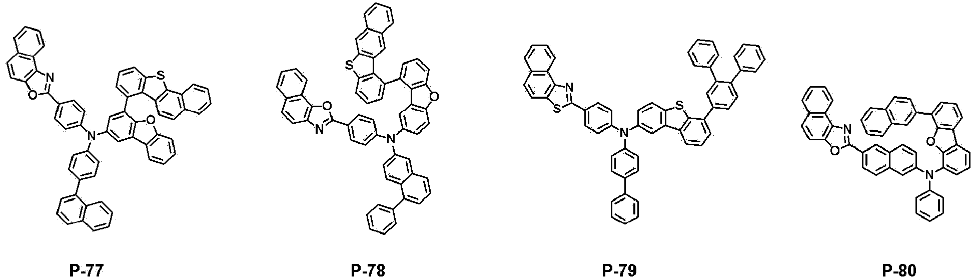

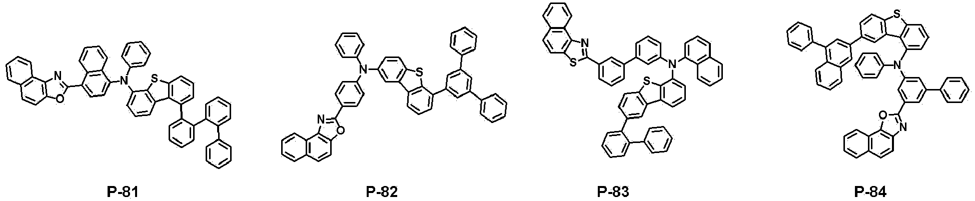

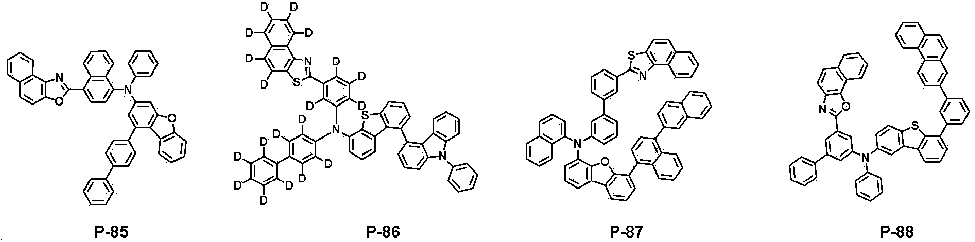

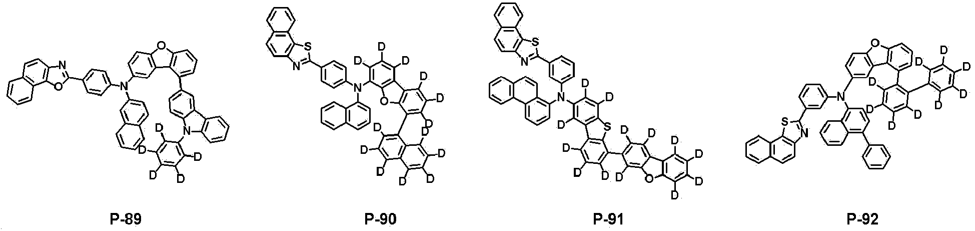

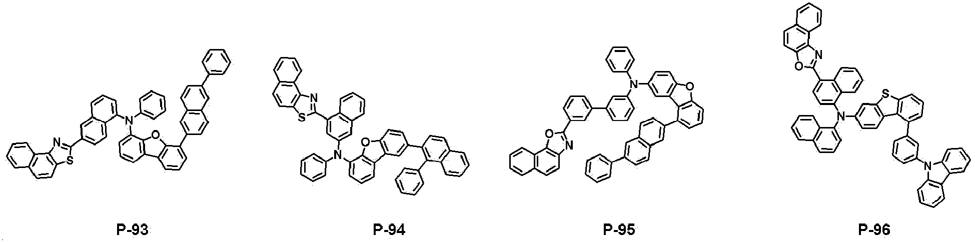

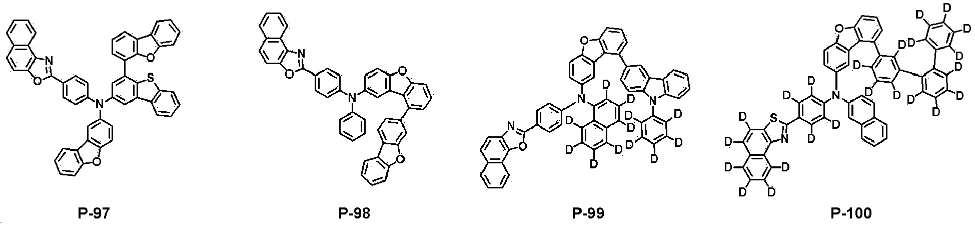

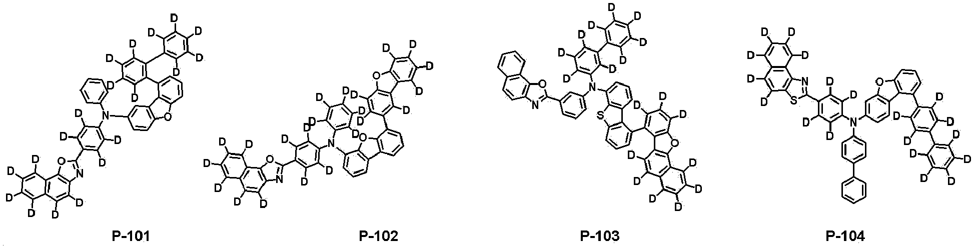

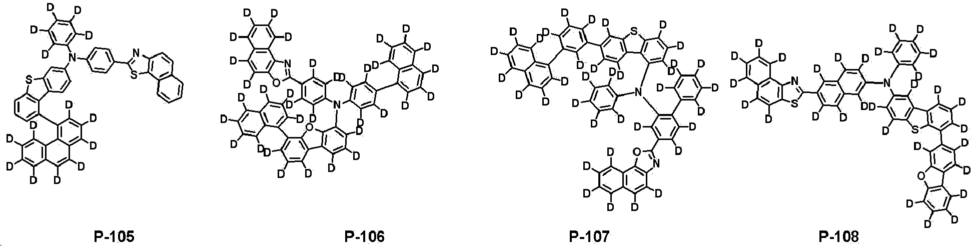

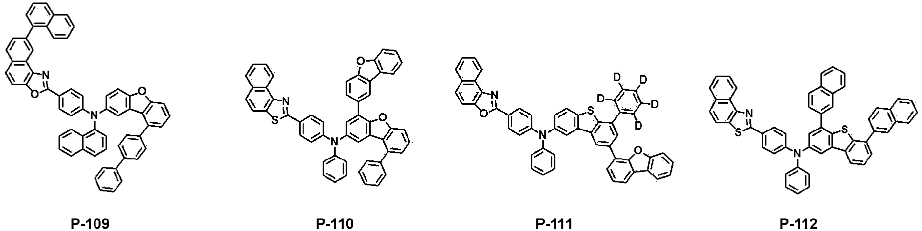

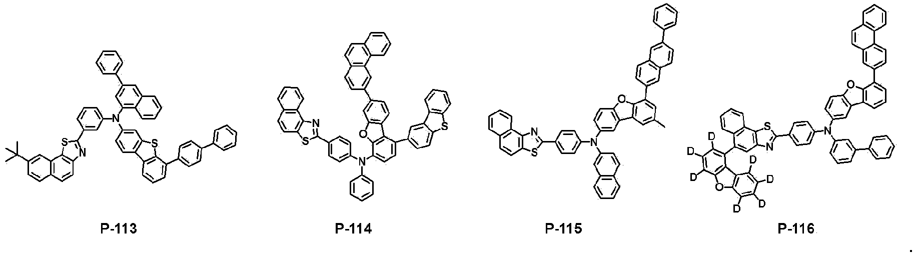

In another aspect, the present invention provides a material for an organic electronic device comprising a compound of Formula 1 and a compound represented by Formula I. Preferably, the material for the organic electronic device is a host material for an emission layer. That is, the mixture of the compound represented by Formula 1 and the compound represented by Formula I may be used as a host in an organic layer.

Hereinafter, Formula I will be described in detail.

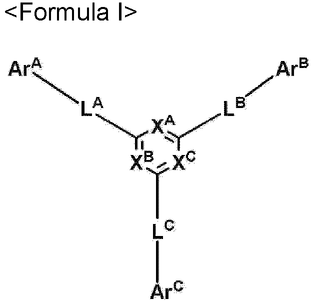

In Formula I, each symbol may be defined as follows.

X^{A} to X^{C} are each N or C(R'), and at least two of them are N. For example, a ring containing X^{A} to X^{C} may be pyrimidine or triazine.

Ar^{A} to Ar^{C} are each independently selected from the group consisting of a C₆-C₆₀ aryl group, a fluorenyl group, C₂-C₆₀ heterocyclic group containing at least one heteroatom of O, N, S, Si and P, a C₃-C₆₀ aliphatic ring group, a fused ring of a C₃-C₆₀ aliphatic ring and a C₆-C₆₀ aromatic ring, and a C₁-C₃₀ alkyl group.

L^{A} to L^{C} are each independently selected from the group consisting of a single bond, a C₆-C₆₀ arylene group, a fluorenylene group, a C₂-C₆₀ heterocyclic group containing at least one heteroatom of O, N, S, Si and P, a C₃-C₆₀ aliphatic ring group, and a fused ring of a C₃-C₆₀ aliphatic ring and a C₆-C₆₀ aromatic ring.

R' is selected from the group consisting of hydrogen, deuterium, halogen, a cyano group, a nitro group, a C₆-C₆₀ aryl group, a fluorenyl group, a C₂-C₆₀ heterocyclic group containing at least one heteroatom of O, N, S, Si and P, a C₃-C₆₀ aliphatic ring group, a fused ring of a C₆-C₆₀ aromatic ring and a C₃-C₆₀ aliphatic ring, a C₁-C₂₀ alkyl group, a C₂-C₂₀ alkenyl group, a C₂-C₂₀ alkynyl group, a C₁-C₂₀ alkoxyl group, and a C₆-C₆₀ aryloxy group.

When at least one of Ar^{A} to Ar^{C}, R' is an aryl group, or at least one of L^{A} to L^{C} is an arylene group, the aryl group or the arylene group may be, for example, a C₆-C₃₀, a C₆-C₂₉, a C₆-C₂₈, a C₆-C₂₇, a C₆-C₂₆, a C₆-C₂₅, a C₆-C₂₄, a C₆-C₂₃, a C₆-C₂₂, a C₆-C₂₁, a C₆-C₂₀, a C₆-C₁₀, a C₆-C₁₈, a C₆-C₁₇, a C₆-C₁₆, a C₆-C₁₅, a C₆-C₁₄, a C₆-C₁₃, a C₆-C₁₂, a C₆-C₁₁, a C₆-C₁₀, a C₆, a C₁₀, a C₁₂, a C₁₃, a C₁₄, a C₁₅, a C₁₆, a C₁₇, or a C₁₈ aryl group or arylene group, specifically, phenyl, biphenyl, naphthyl, terphenyl, phenanthrene, triphenylene, or the like.

When at least one of Ar^{A} to Ar^{C}, R', L^{A} to L^{C} is a heterocyclic group, the heterocyclic group may be, for example, a C₂-C₃₀, a C₂-C₂₉, a C₂-C₂₈, a C₂-C₂₇, a C₂-C₂₆, a C₂-C₂₅, a C₂-C₂₄, a C₂-C₂₃, a C₂-C₂₂, a C₂-C₂₁, a C₂-C₂₀, a C₂-C₁₉, a C₂-C₁₈, a C₂-C₁₇, a C₂-C₁₆, a C₂-C₁₅, a C₂-C₁₄, a C₂-C₁₃, a C₂-C₁₂, a C₂-C₁₁, a C₂-C₁₀, a C₂-C₉, a C₂-C₈, a C₂-C₇, a C₂-C₆, a C₂-C₅, a C₂-C₄, a C₂-C₃, a C₂, a C₃, a C₄, a C₅, a C₆, a C₇, a C₈, a C₉, a C₁₀, a C₁₁, a C₁₂, a C₁₃, a C₁₄, a C₁₅, a C₁₆, a C₁₇, a C₁₈, a C₁₉, a C₂₀, a C₂₁, a C₂₂, a C₂₃, a C₂₄, a C₂₅, a C₂₆, a C₂₇, a C₂₈, or a C₂₉ heterocyclic group, specifically, pyridine, pyrimidine, pyrazine, pyridazine, triazine, furan, pyrrole, indene, indole, phenyl-indole, benzoindole, phenyl-benzoindole, pyrazinoindol, quinoline, isoquinoline, benzoquinoline, pyridoquinoline, quinazoline, benzoquinazoline, dibenzoquinazoline, phenanthroquinazoline, quinoxaline, benzoquinoxaline, dibenzoquinoxaline, benzofuran, naphthobenzofuran, dibenzofuran, dinaphthofuran, thiophene, benzothiophene, dibenzothiophene, naphthobenzothiophene, dinaphthothiophene, carbazole, phenyl-carbazole, benzocarbazole, phenylbenzocarbazole, naphthyl-benzocarbazole, dibenzocarbazole, indolocarbazole, benzofuropyridine, benzothienopyridine, benzofuropyridine, benzothienopyrimidine, benzofuropyrimidine, benzothienopyrazine, benzofuropyrazine, benzoimidazole, benzothiazole, benzooxazole, benzosiloe, phenanthroline, dihydro-phenylphenazine, 10-phenyl-10H-phenoxazine, phenoxazine, dibenzodioxin, benzodibenzodioxin, thianthrene, 9,9-dimethyl-9H-xanthene, 9,9-dimethyl-9H-thioxanthene, dihydrodimethylphenylacridine, spiro[fluorene-9,9'-xanthene] and the like.

When at least one of Ar^{A} to Ar^{C}, R' is a fluorenyl group, or when at least one of L^{A} to L^{C} is a fluorenylene group, the fluorenyl group or the fluorenylene group may be 9,9-dimethyl-9H-fluorene, 9,9-diphenyl-9H-fluorene, 9,9'-spirobifluorene, spiro[benzo[b]fluorene-11,9'-fluorene], benzo[b]fluorene, 11,11-diphenyl-11H-benzo[b]fluorene, or 9-(naphthalen-2-yl)-9-phenyl-9H-fluorene.

When at least one of Ar^{A} to Ar^{C}, R', L^{A} to L^{C} is an aliphatic ring group, the aliphatic ring group may be, for example, a C₃-C₃₀, a C₃-C₂₉, a C₃-C₂₈, a C₃-C₂₇, a C₃-C₂₆, a C₃-C₂₅, a C₃-C₂₄, a C₃-C₂₃, a C₃-C₂₂, a C₃-C₂₁, a C₃-C₂₀, a C₃-C₁₉, a C₃-C₁₈, a C₃-C₁₇, a C₃-C₁₆, a C₃-C₁₅, a C₃-C₁₄, a C₃-C₁₃, a C₃-C₁₂, a C₃-C₁₁, a C₃-C₁₀, a C₃-C₈, a C₃-C₆, a C₆, a C₁₀, a C₁₁, a C₁₂, a C₁₃, a C₁₄, a C₁₅, a C₁₆, a C₁₇ or a C₁₈ aliphatic ring group, specifically, a cyclohexanyl group, an adamantyl group, etc.

The aryl group, the arylene group, the fluorenyl group, the fluorenylene group, the heterocyclic group, the aliphatic ring group, the fused ring, the alkyl group, the alkenyl group, the alkynyl group, the alkoxyl group, and the aryloxyl group may be each substituted with one or more substituents selected from the group consisting of deuterium, halogen, a silane group unsubstituted or substituted with a C₁-C₂₀ alkyl group or a C₆-C₂₀ aryl group, a phosphine oxide substituted or unsubstituted with a C₁-C₂₀ alkyl group or a C₆-C₂₀ aryl group, a cyano group, a nitro group, C₁-C₂₀ alkylthio group, C₁-C₂₀ alkoxy group, C₆-C₃₀ aryloxy group, C₆-C₃₀ arylthio group, a C₁-C₂₀ alkyl group, a C₂-C₂₀ alkenyl group, a C₂-C₂₀ alkynyl group, a C₆-C₃₀ aryl group, a fluorenyl group, a C₃-C₃₀ aliphatic ring, a fused ring of a C₆-C₃₀ aromatic ring and a C₃-C₃₀ aliphatic ring, and a C₂-C₃₀ heterocyclic group comprising at least one heteroatom selected from the group consisting of O, N, S, Si and P, the adjacent substituents may be bonded to each other to form a ring, and hydrogen of the substituents may be replaced with deuterium.

When at least one of the aryl group, the arylene group, the fluorenyl group, the fluorenylene group, the heterocyclic group, the aliphatic ring group, the fused ring, the alkyl group, the alkenyl group, the alkynyl group, the alkoxyl group, the aryloxyl group, and the ring formed by adjacent groups is substituted with an aryl group, the aryl group may be, for example, a C₆-C₃₀, a C₆-C₂₉, a C₆-C₂₈, a C₆-C₂₇, a C₆-C₂₆, a C₆-C₂₅, a C₆-C₂₄, a C₆-C₂₃, a C₆-C₂₂, a C₆-C₂₁, a C₆-C₂₀, a C₆-C₁₉, a C₆-C₁₈, a C₆-C₁₇, a C₆-C₁₆, a C₆-C₁₅, a C₆-C₁₄, a C₆-C₁₃, a C₆-C₁₂, a C₆-C₁₁, a C₆-C₁₀, a C₆, a C₁₀, a C₁₂, a C₁₃, a C₁₄, a C₁₅, a C₁₆, a C₁₇, or a C₁₈ aryl group.

When at least one of the aryl group, the arylene group, the fluorenyl group, the fluorenylene group, the heterocyclic group, the aliphatic ring group, the fused ring, the alkyl group, the alkenyl group, the alkynyl group, the alkoxyl group, the aryloxyl group, and the ring formed by adjacent groups is substituted with a heterocyclic group, the heterocyclic group may be, for example, a C₂-C₂₀, a C₂-C₁₉, a C₂-C₁₈, a C₂-C₁₇, a C₂-C₁₆, a C₂-C₁₅, a C₂-C₁₄, a C₂-C₁₃, a C₂-C₁₂, a C₂-C₁₁, a C₂-C₁₀, a C₂-C₉, a C₂-C₈, a C₂-C₇, a C₂-C₆, a C₂-C₅, a C₂-C₄, a C₂-C₃, a C₂, a C₃, a C₄, a C₅, a C₆, a C₇, a C₈, a C₉, a C₁₀, a C₁₁, a C₁₂, a C₁₃, a C₁₄, a C₁₅, a C₁₆, a C₁₇, a C₁₈, a C₁₉, a C₂₀ heterocyclic group.

When at least one of the aryl group, the arylene group, the fluorenyl group, the fluorenylene group, the heterocyclic group, the aliphatic ring group, the fused ring, the alkyl group, the alkenyl group, the alkynyl group, the alkoxyl group, the aryloxyl group, and the ring formed by adjacent groups is substituted with an aliphatic ring group, the aliphatic ring group may be, for example, a C₃-C₃₀, a C₃-C₂₉, a C₃-C₂₈, a C₃-C₂₇, a C₃-C₂₆, a C₃-C₂₅, a C₃-C₂₄, a C₃-C₂₃, a C₃-C₂₂, a C₃-C₂₁, a C₃-C₂₀, a C₃-C₁₉, a C₃-C₁₈, a C₃-C₁₇, a C₃-C₁₆, a C₃-C₁₅, a C₃-C₁₄, a C₃-C₁₃, a C₃-C₁₂, a C₃-C₁₁, a C₃-C₁₀, a C₃-C₈, a C₃-C₆, a C₆, a C₁₀, a C₁₁, a C₁₂, a C₁₃, a C₁₄, a C₁₅, a C₁₆, a C₁₇ or a C₁₈ aliphatic ring group.

When at least one of the aryl group, the arylene group, the fluorenyl group, the fluorenylene group, the heterocyclic group, the aliphatic ring group, the fused ring, the alkyl group, the alkenyl group, the alkynyl group, the alkoxyl group, the aryloxyl group, and the ring formed by adjacent groups is substituted with a fluorenyl group, the fluorenyl group may be 9,9-dimethyl-9H-fluorene, 9,9-diphenyl-9H-fluorene, 9,9'-spirobifluorene, spiro[benzo[b]fluorene-11,9'-fluorene], benzo[b]fluorene, 11,11-diphenyl-11H-benzo[b]fluorene, or 9-(naphthalen-2-yl)-9-phenyl-9H-fluorene.

At least one of Ar^{A} to Ar^{C} may be selected from the group consisting of Formulae Ar-a to Ar-d, but is not limited thereto.

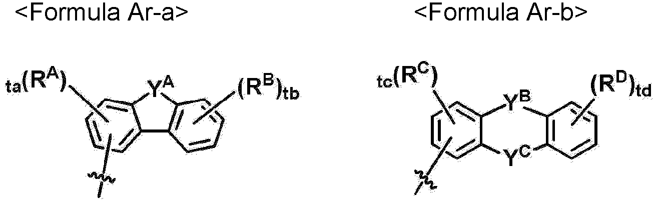

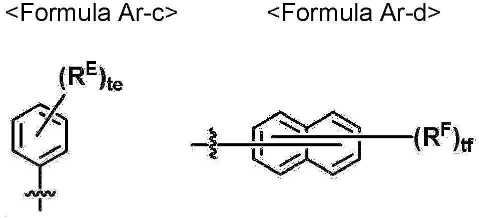

In Formula Ar-a to Formula Ar-d, each of symbols may be defined as follows.

Y^{A} to Y^{C} are each independently O, S, C(R₁)(R₂) or N(Ar₁).

R^{A} to R^{F}, R¹ and R₂ are each independently selected from the group consisting of hydrogen, deuterium, halogen, a silane group unsubstituted or substituted with a C₁-C₂₀ alkyl group or a C₆-C₂₀ aryl group, a phosphine oxide substituted or unsubstituted with a C₁-C₂₀ alkyl group or a C₆-C₂₀ aryl group, a cyano group, a nitro group, C₁-C₂₀ alkylthio group, C₁-C₂₀ alkoxy group, C₆-C₃₀ aryloxy group, C₆-C₃₀ arylthio group, a C₁-C₂₀ alkyl group, a C₂-C₂₀ alkenyl group, a C₂-C₂₀ alkynyl group, a C₆-C₃₀ aryl group, a fluorenyl group, a C₃-C₃₀ aliphatic ring, a fused ring of a C₆-C₃₀ aromatic ring and a C₃-C₃₀ aliphatic ring, and a C₂-C₃₀ heterocyclic group comprising at least one heteroatom selected from the group consisting of O, N, S, Si and P, the adjacent groups may be bonded to each other to form a ring.

When at least one pair of adjacent R^{A}s to adjacent R^{F}s are bonded to each other to form a ring, the ring may be selected from the group consistiong of a C₆-C₆₀ aryl ring, a fluorenyl group, a C₃-C₆₀ aliphatic ring group, C₂-C₆₀ heterocyclic group containing at least one heteroatom of O, N, S, Si and P, and a fused ring of a C₃-C₆₀ aliphatic ring and a C₆-C₆₀ aromatic ring.

When adjacent R₁ and R₂ are bonded to each other to form a ring, a spiro compound may be formed.

Ar₁ is selected from the group consisting of a C₁-C₂₀ alkyl group, a C₆-C₃₀ aryl group, a fluorenyl group, a C₃-C₃₀ aliphatic ring, a fused ring of a C₆-C₃₀ aromatic ring and a C₃-C₃₀ aliphatic ring, and a C₂-C₃₀ heterocyclic group comprising at least one heteroatom selected from the group consisting of O, N, S, Si and P.

ta and tc are each an integer of 0 to 3, tb and td are each an integer of 0 to 4, te is an integer of 0 to 5, tf is an integer of 0 to 7, and when they are integers of 2 or more, each of the plurality of R^{A} to each of the plurality of R^{F} may be the same or different from each other.

R^{A} to R^{F}, R₁, R₂ may be each substituted with one or more substituents selected from the group consisting of deuterium, halogen, a silane group unsubstituted or substituted with a C₁-C₂₀ alkyl group or a C₆-C₂₀ aryl group, a phosphine oxide substituted or unsubstituted with a C₁-C₂₀ alkyl group or a C₆-C₂₀ aryl group, a cyano group, a nitro group, C₁-C₂₀ alkylthio group, C₁-C₂₀ alkoxy group, C₆-C₃₀ aryloxy group, C₆-C₃₀ arylthio group, a C₁-C₂₀ alkyl group, a C₂-C₂₀ alkenyl group, a C₂-C₂₀ alkynyl group, a C₆-C₃₀ aryl group, a fluorenyl group, a C₃-C₃₀ aliphatic ring, a fused ring of a C₆-C₃₀ aromatic ring and a C₃-C₃₀ aliphatic ring, and a C₂-C₃₀ heterocyclic group comprising at least one heteroatom selected from the group consisting of O, N, S, Si and P, and hydrogen of the substituents may be replaced with deuterium.

The Formula Ar-a may be selected from the group consisting of Formulae Ar-a-1 to Ar-a-4, Formula Ar-b may be represented by Formula Ar-b-1 or Ar-b-2, and Formula Ar-d may be represented by Formula Ar-d-1 or Ar-d-2.

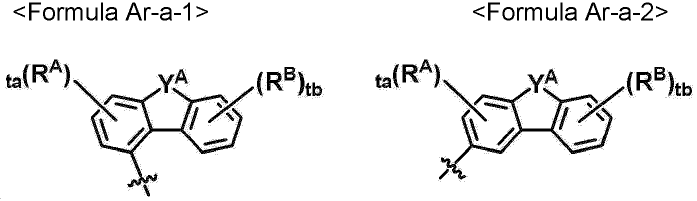

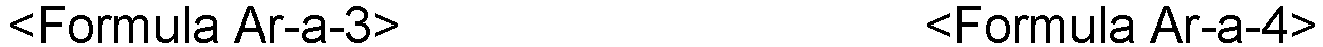

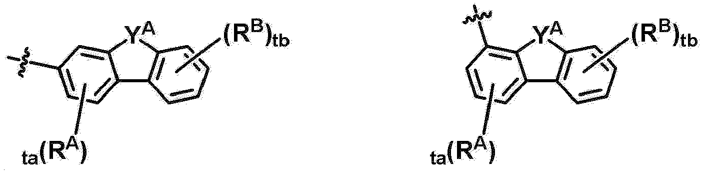

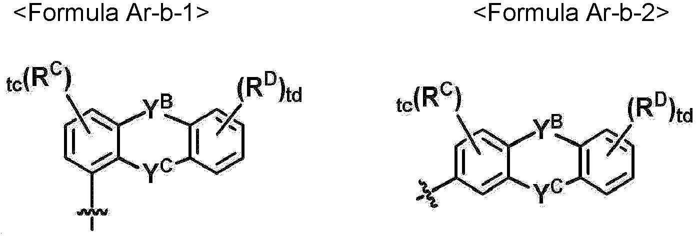

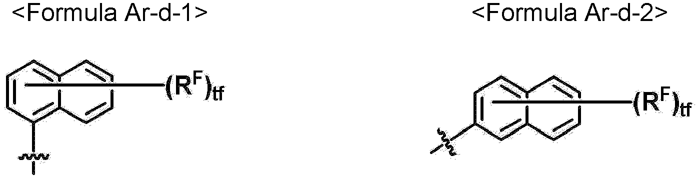

In Formula Ar-a-1 to Formula Ar-a-4, Y^{A}, R^{A}, R^{B}, ta, tb are the same as defined for Formula Ar-a, in Formula Ar-b-1 and Formula Ar-b-2, Y^{B}, Y^{C}, R^{C}, R^{D}, tc, td are the same as defined for Formula Ar-b, in Formula Ar-d-1 and Formula Ar-d-2, R^{F}, tf are the same as defined for Formula Ar-d.

At least one of L^{A} to L^{C} may be selected from the group consisting of a single bond and the following Formula b-1 to b-13, but is not limited thereto.

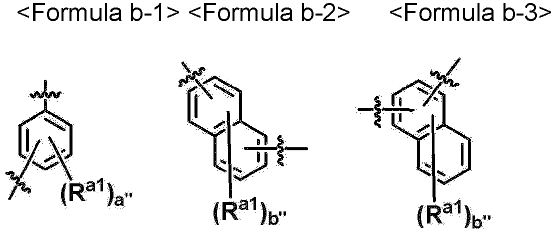

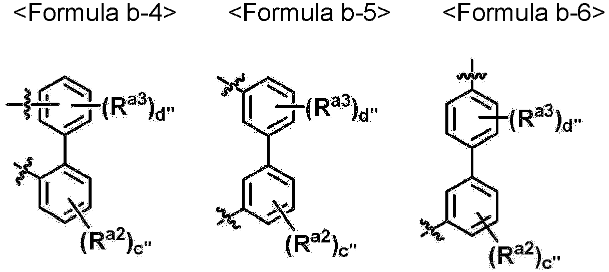

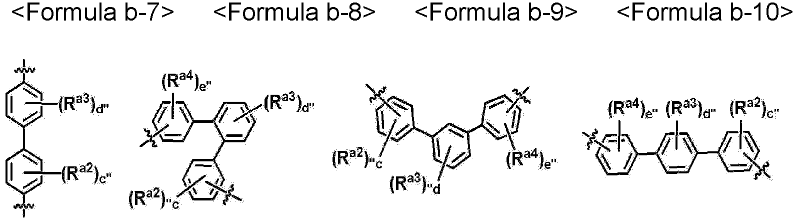

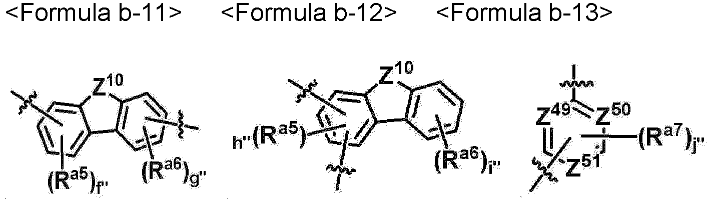

In Formula b-1 to Formula b-13, each symbol may be defined as follows.

Z¹⁰ is O, S, N(Ar₁₂) or C(R₁₁)(R₁₂).

Z⁴⁹, Z⁵⁰, Z⁵¹ are each independently N or C(R₁₃), and at least one of them is N.

R^{a1} to R^{a7}, R₁₁, R₁₂, R₁₃ are each independently selected from the group consisting of hydrogen, deuterium, halogen, a silane group unsubstituted or substituted with a C₁-C₂₀ alkyl group or a C₆-C₂₀ aryl group, a phosphine oxide substituted or unsubstituted with a C₁-C₂₀ alkyl group or a C₆-C₂₀ aryl group, a cyano group, a nitro group, C₁-C₂₀ alkylthio group, C₁-C₂₀ alkoxy group, C₆-C₃₀ aryloxy group, C₆-C₃₀ arylthio group, a C₁-C₂₀ alkyl group, a C₂-C₂₀ alkenyl group, a C₂-C₂₀ alkynyl group, a C₆-C₃₀ aryl group, a fluorenyl group, a C₃-C₃₀ aliphatic ring, a fused ring of a C₆-C₃₀ aromatic ring and a C₃-C₃₀ aliphatic ring, and a C₂-C₃₀ heterocyclic group comprising at least one heteroatom selected from the group consisting of O, N, S, Si and P, and and adjacent groups may be bonded to each other to form a ring.

When at least one pair of adjacent R^{a1}s to adjacent R^{a7}s are bonded to each other to form a ring, the ring may be selected from the group consistiong of a C₆-C₆₀ aryl ring, a fluorenyl group, a C₃-C₆₀ aliphatic ring group, C₂-C₆₀ heterocyclic group containing at least one heteroatom of O, N, S, Si and P, and a fused ring of a C₃-C₆₀ aliphatic ring and a C₆-C₆₀ aromatic ring, and when adjacent R₁₁ and R₁₂ are linked to form a ring, a spiro compound may be formed.

Ar₁₂ is selected from the group consisting of a C₁-C₂₀ alkyl group, a C₆-C₃₀ aryl group, a fluorenyl group, a C₃-C₃₀ aliphatic ring, a fused ring of a C₆-C₃₀ aromatic ring and a C₃-C₃₀ aliphatic ring, and a C₂-C₃₀ heterocyclic group comprising at least one heteroatom selected from the group consisting of O, N, S, Si and P.

a", c", d", and e" are each integers from 0 to 4, b" is an integer from 0 to 6, f" and g" are each integers from 0 to 3, h" is an integer from 0 to 2, and i" is an integer of 0 or 1, and when these are integers of 2 or more, each of the plurality of R^{a1} to R^{a7} may be the same or different from each other.

R^{a1} to R^{a7}, R₁₁, R₁₂, R₁₃, Ar₁₂ may be each substituted with one or more substituents selected from the group consisting of deuterium, halogen, a silane group unsubstituted or substituted with a C₁-C₂₀ alkyl group or a C₆-C₂₀ aryl group, a phosphine oxide substituted or unsubstituted with a C₁-C₂₀ alkyl group or a C₆-C₂₀ aryl group, a cyano group, a nitro group, C₁-C₂₀ alkylthio group, C₁-C₂₀ alkoxy group, C₆-C₃₀ aryloxy group, C₆-C₃₀ arylthio group, a C₁-C₂₀ alkyl group, a C₂-C₂₀ alkenyl group, a C₂-C₂₀ alkynyl group, a C₆-C₃₀ aryl group, a fluorenyl group, a C₃-C₃₀ aliphatic ring, a fused ring of a C₆-C₃₀ aromatic ring and a C₃-C₃₀ aliphatic ring, and a C₂-C₃₀ heterocyclic group comprising at least one heteroatom selected from the group consisting of O, N, S, Si and P, and hydrogen of the substituents may be replaced with deuterium.

Specifically, compound represented by Formula I may be one of the following compounds, but is not limited thereto.

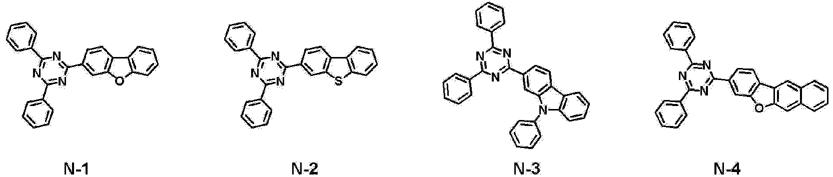

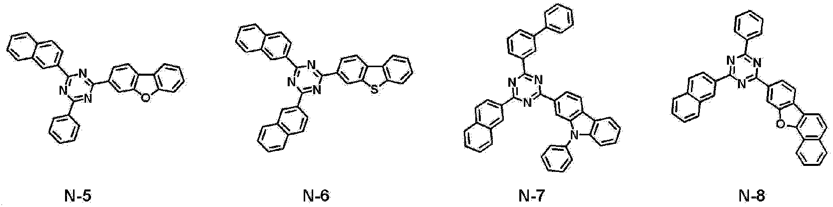

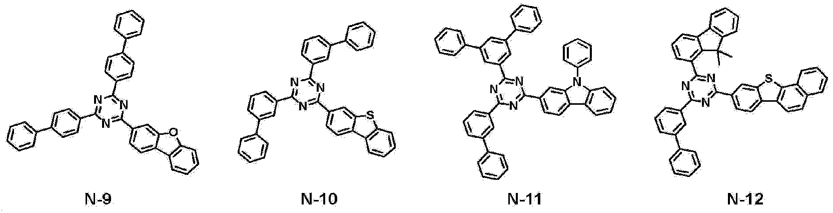

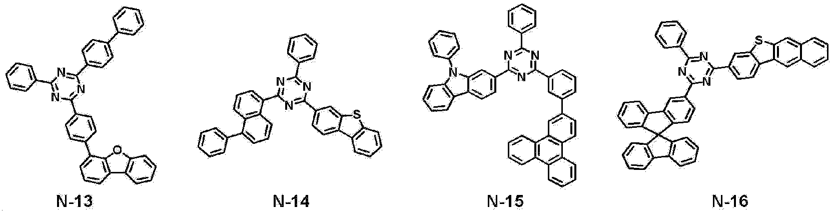

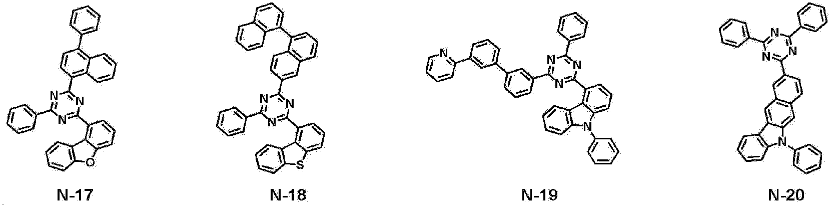

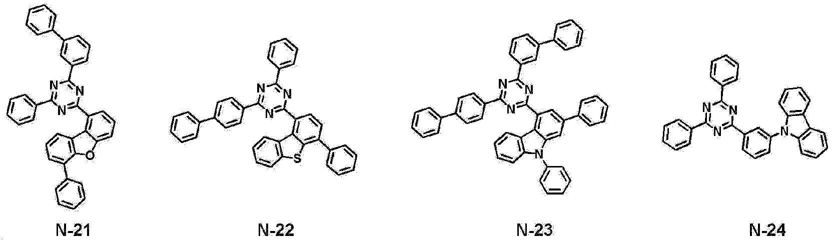

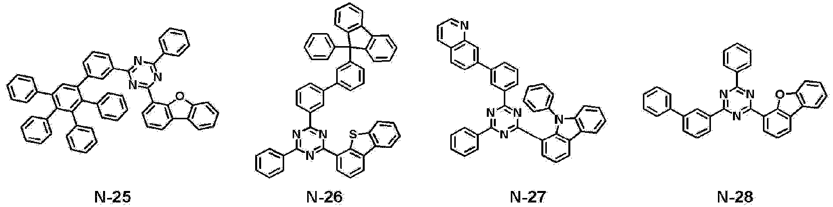

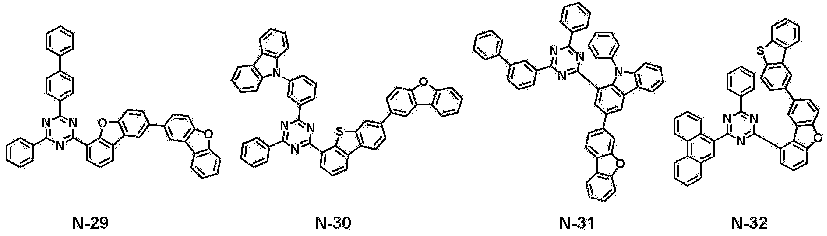

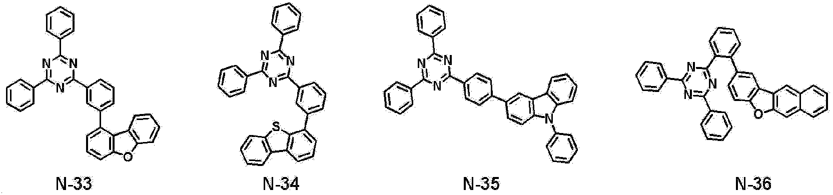

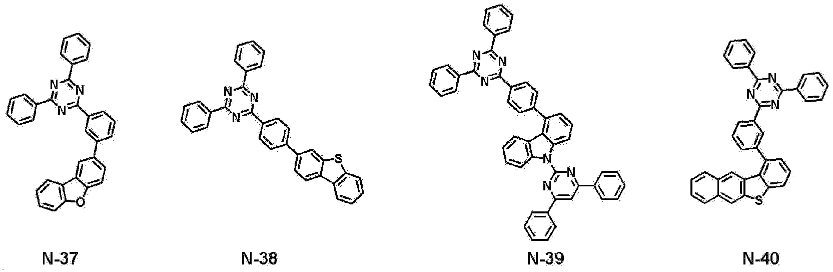

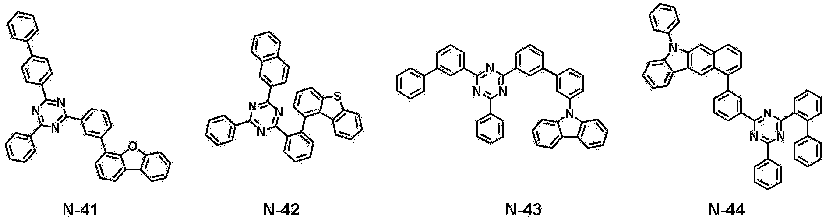

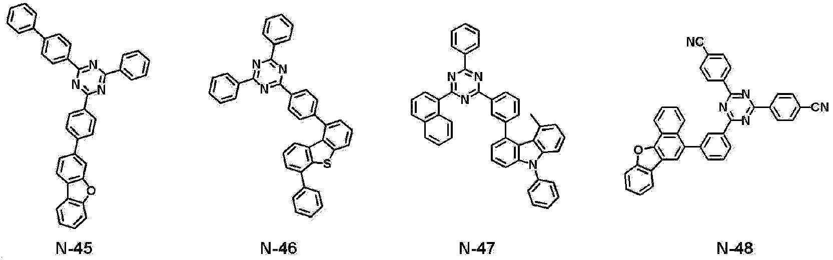

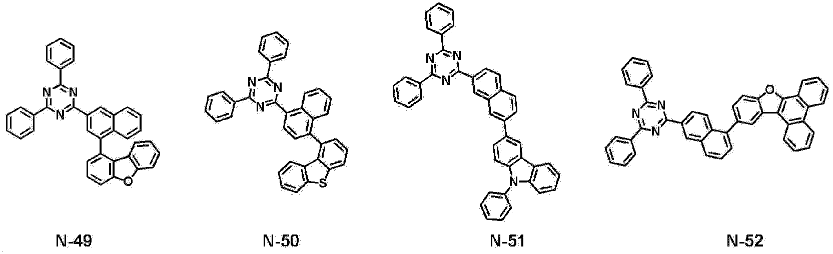

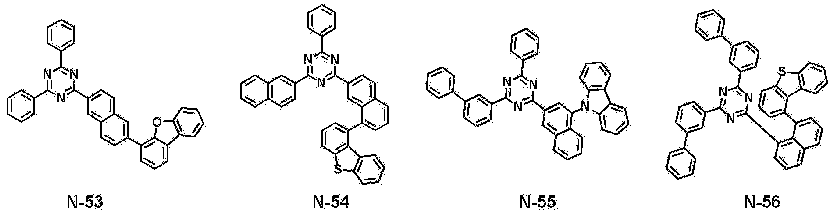

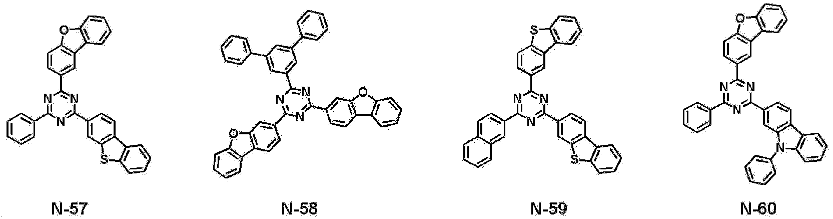

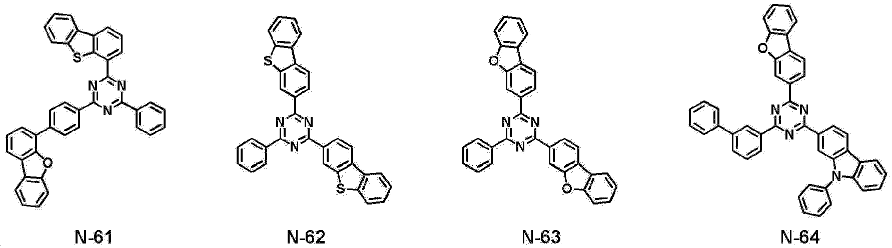

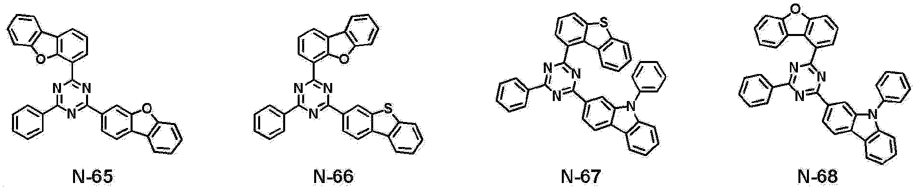

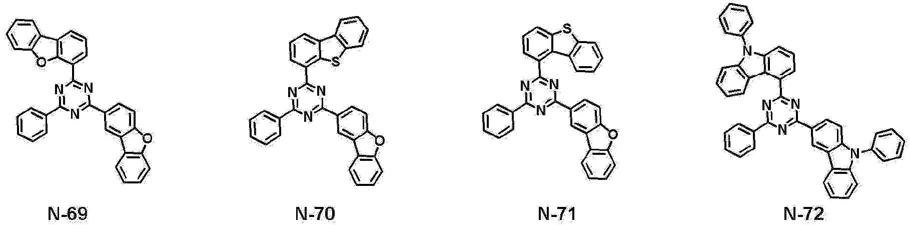

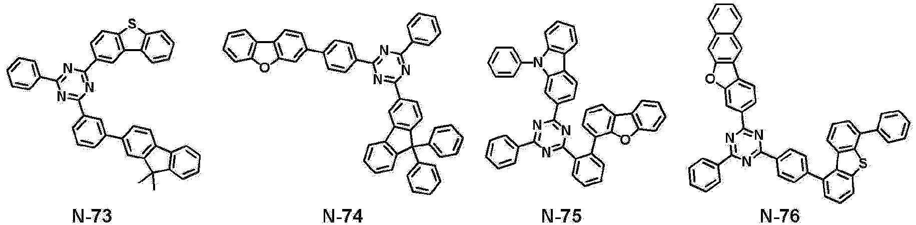

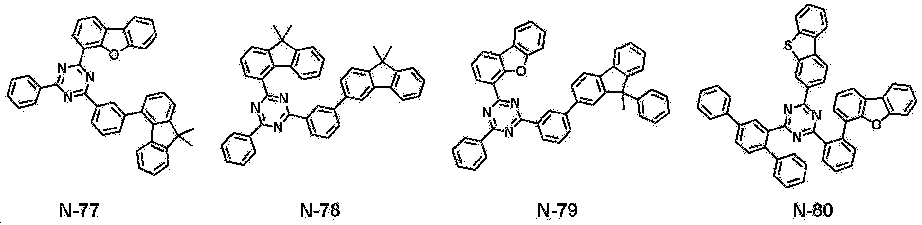

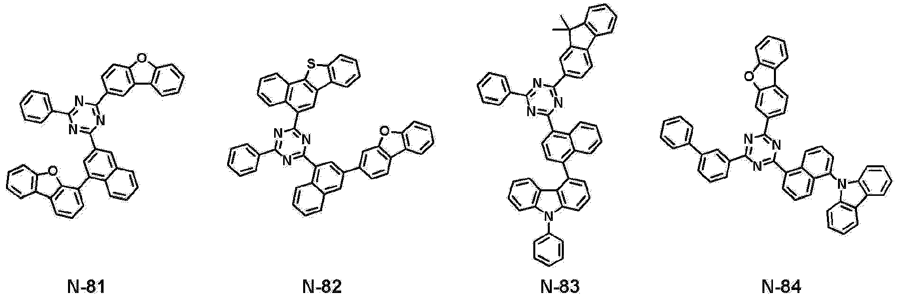

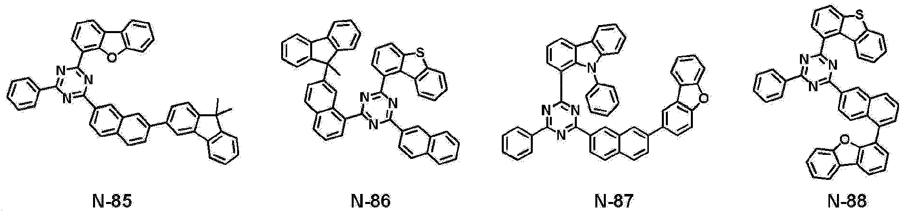

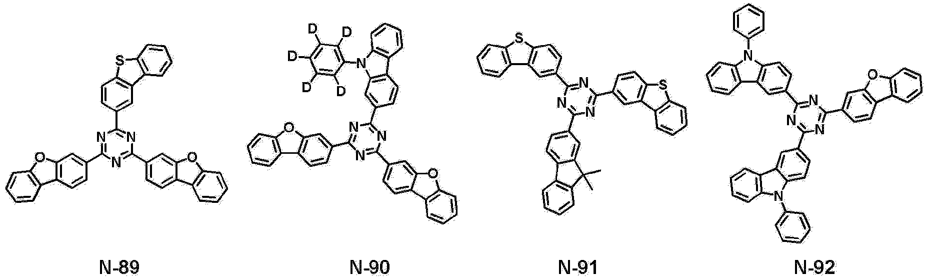

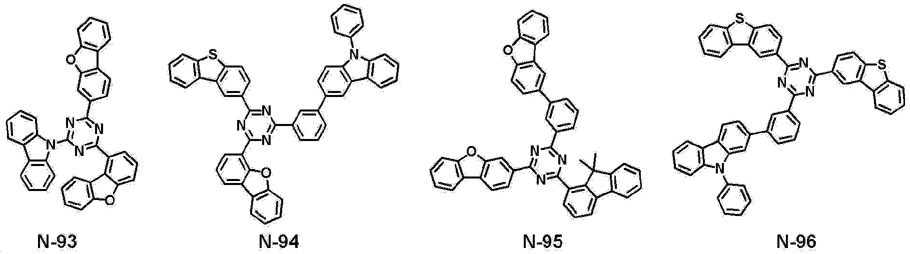

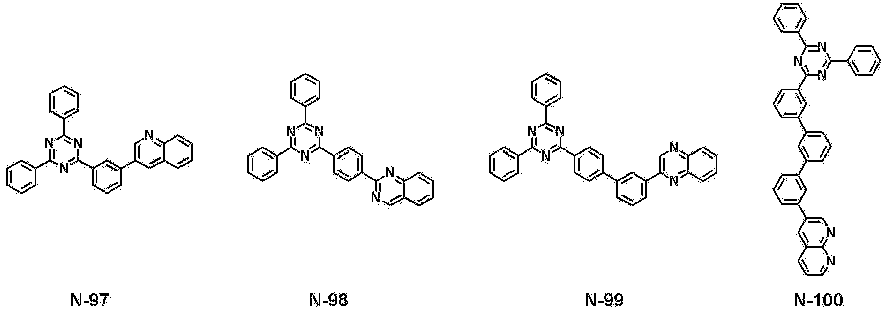

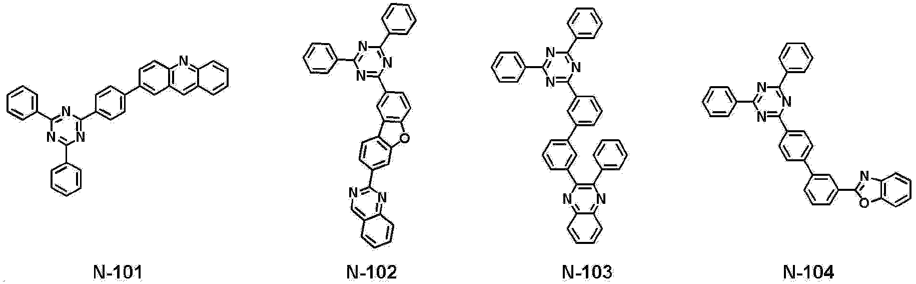

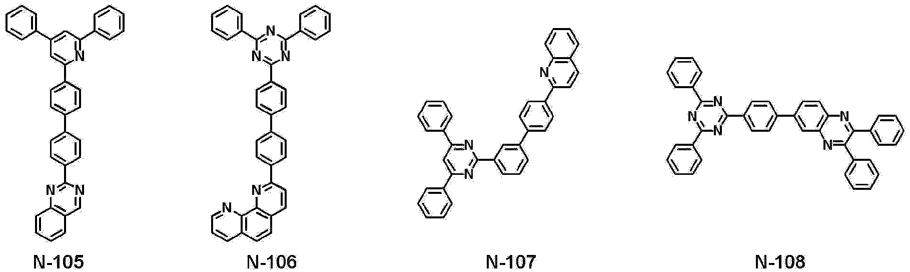

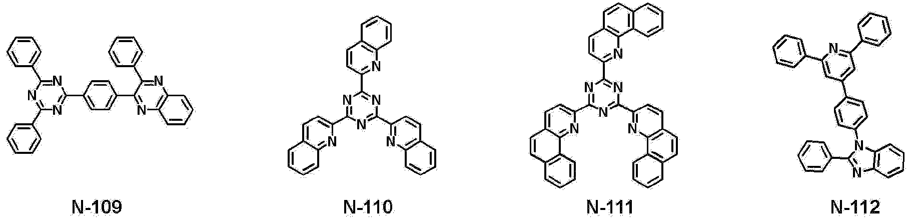

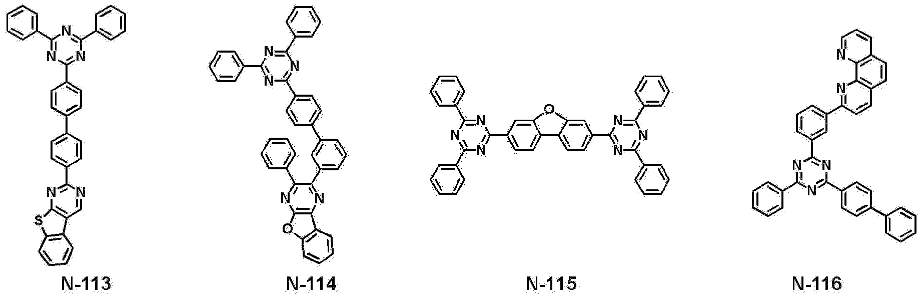

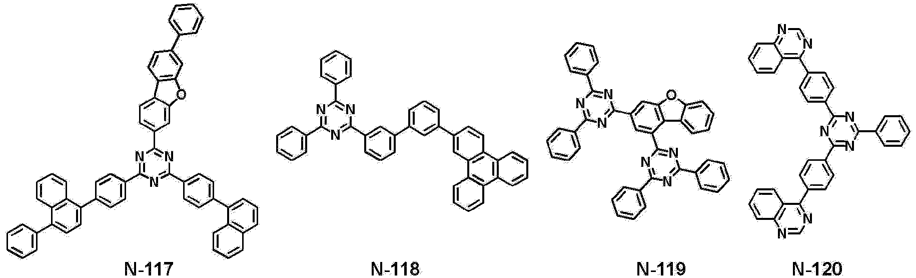

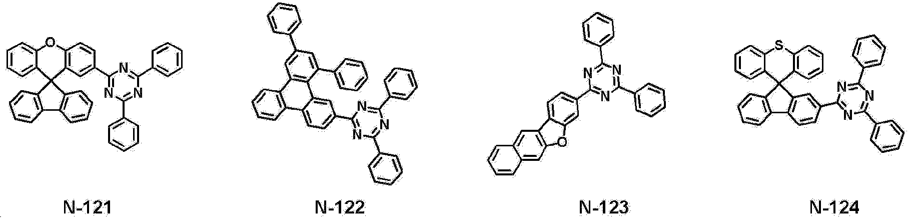

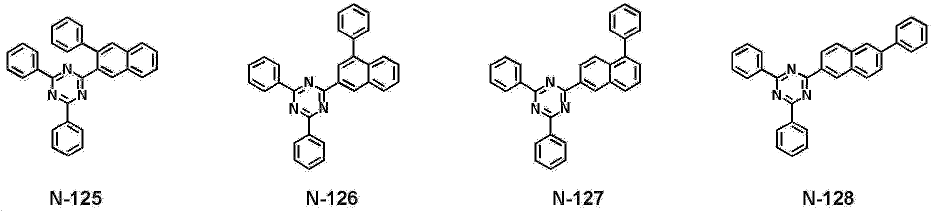

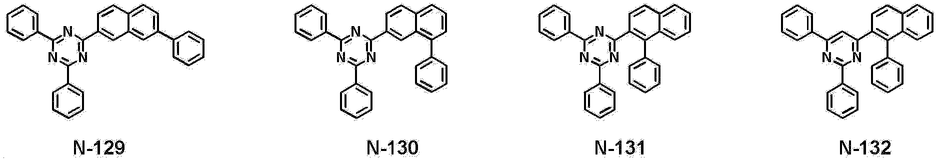

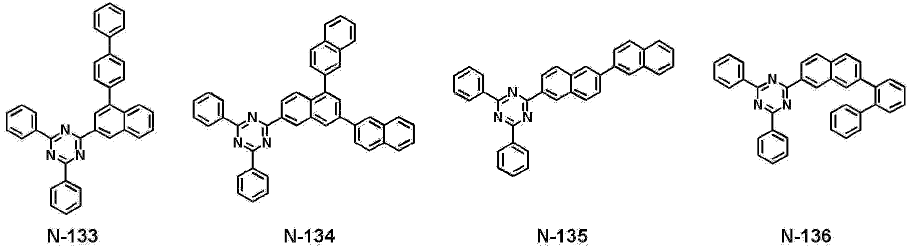

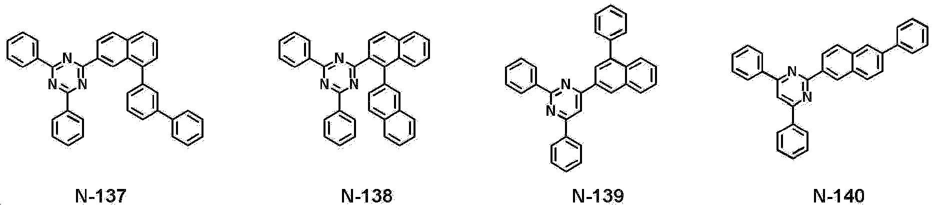

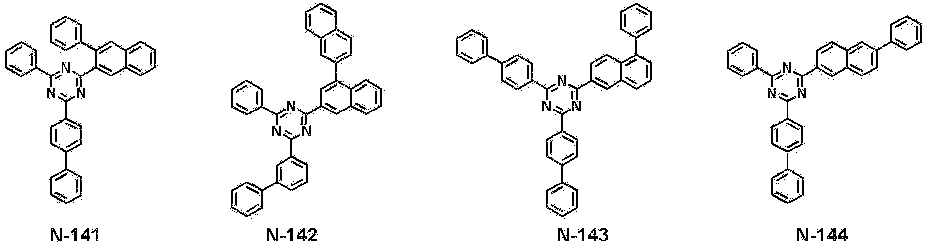

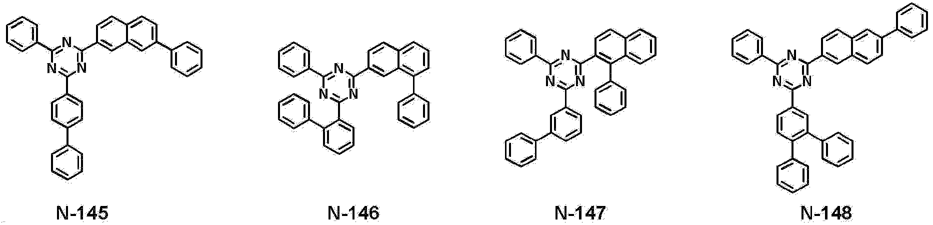

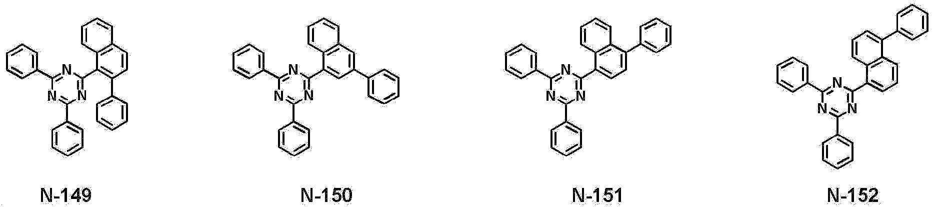

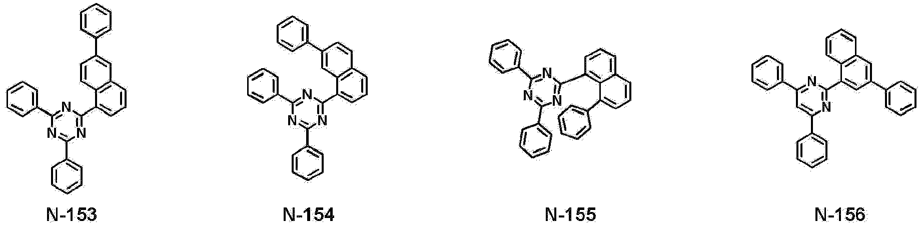

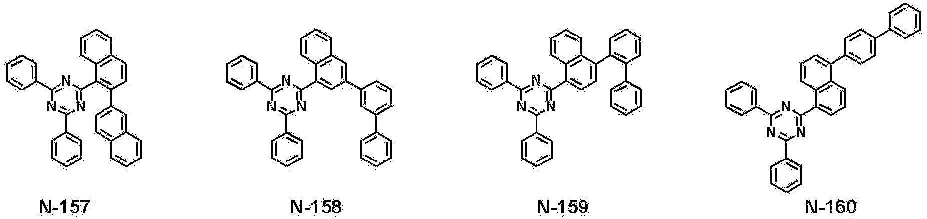

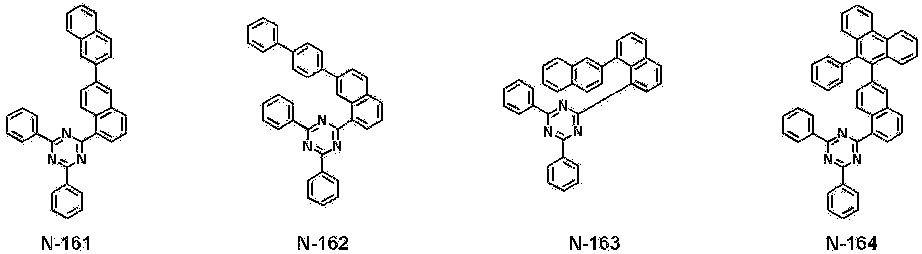

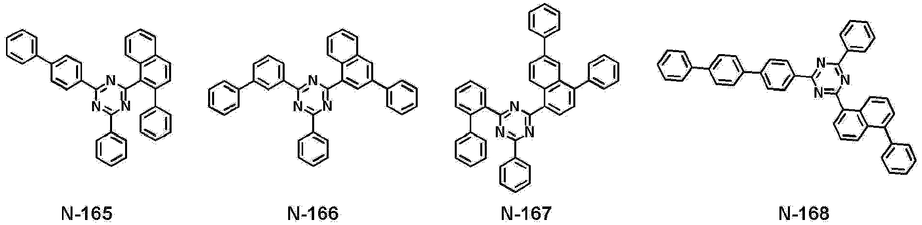

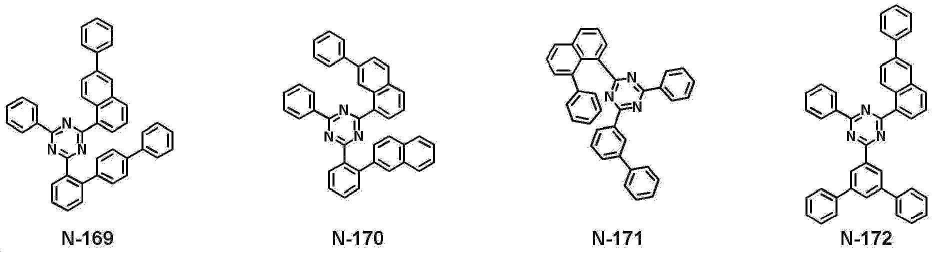

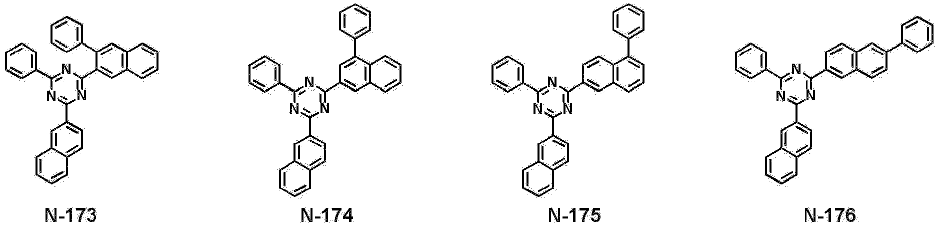

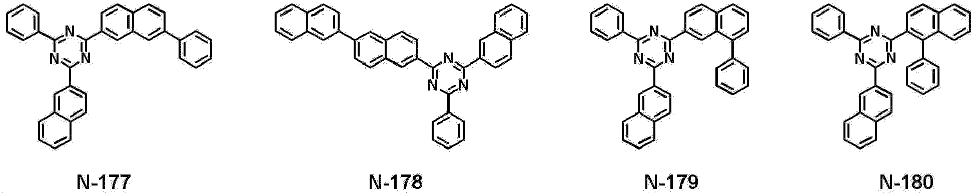

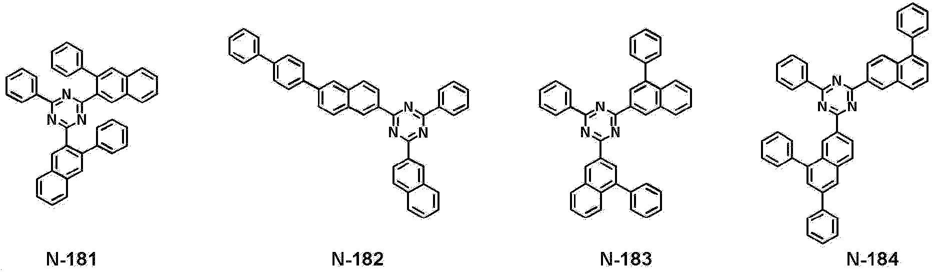

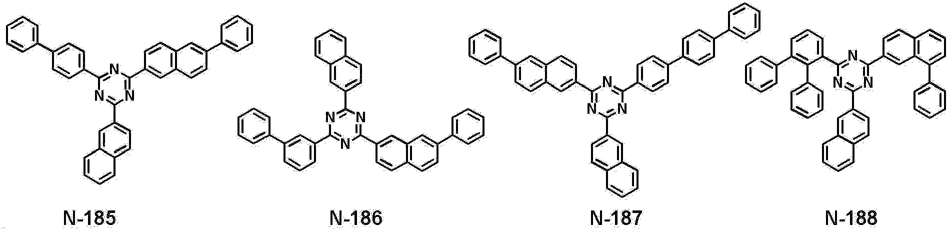

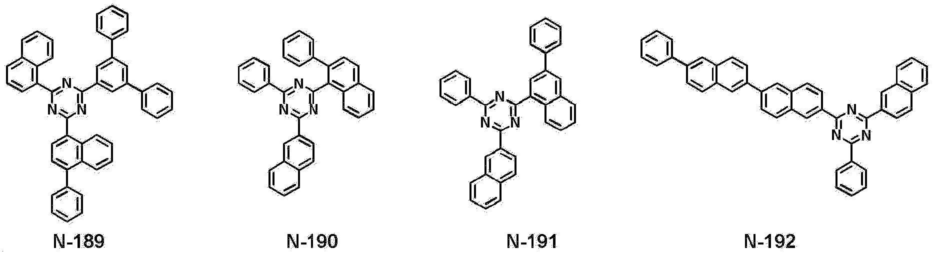

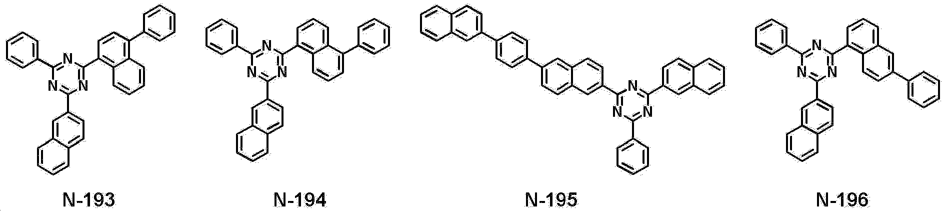

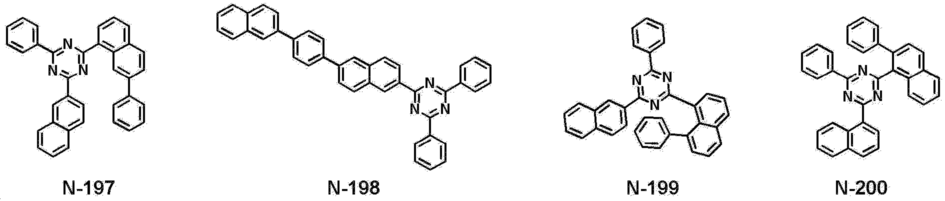

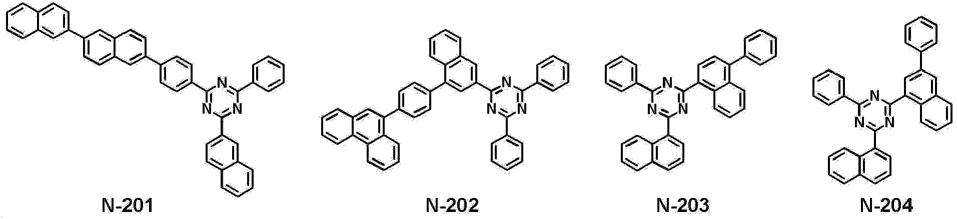

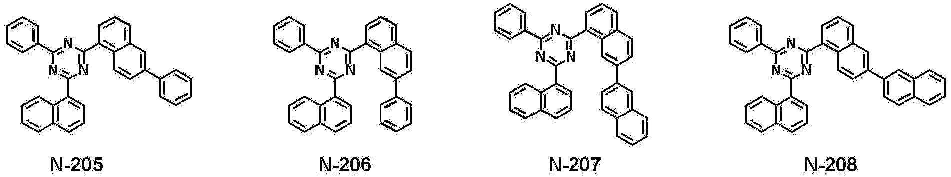

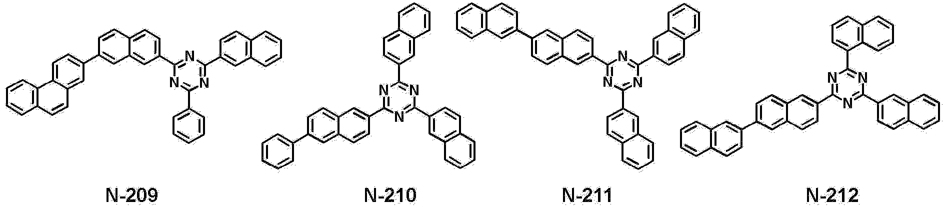

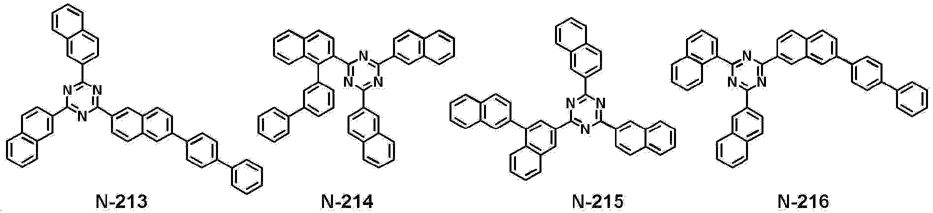

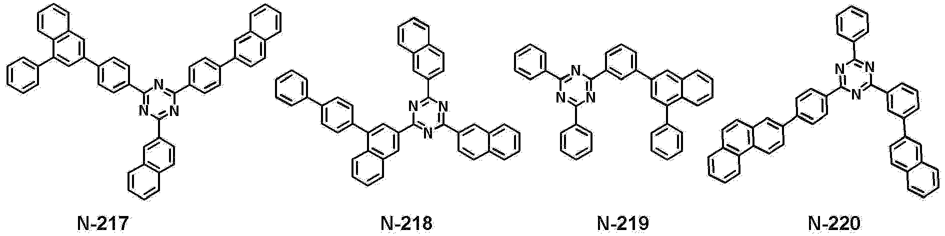

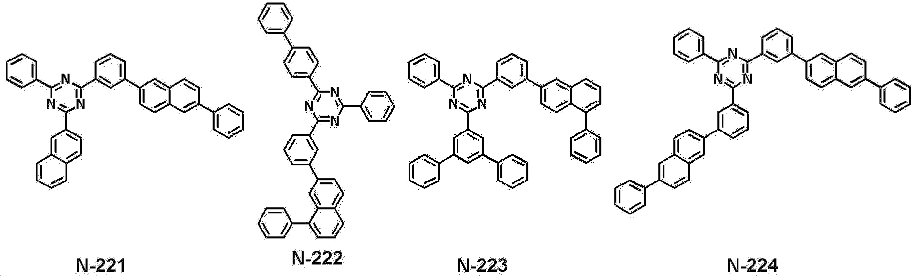

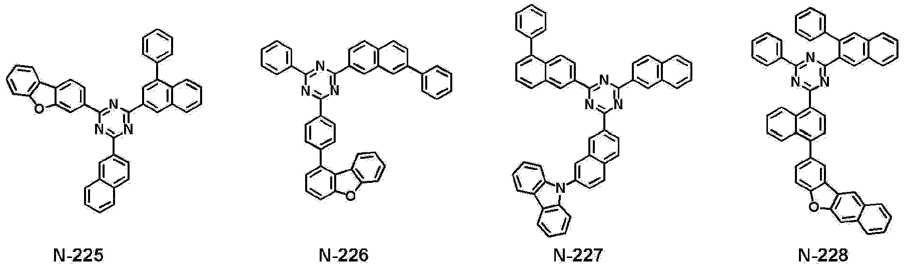

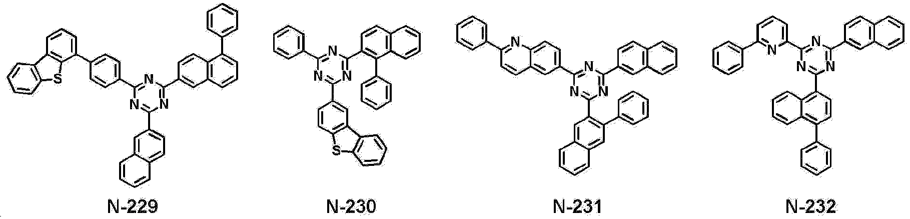

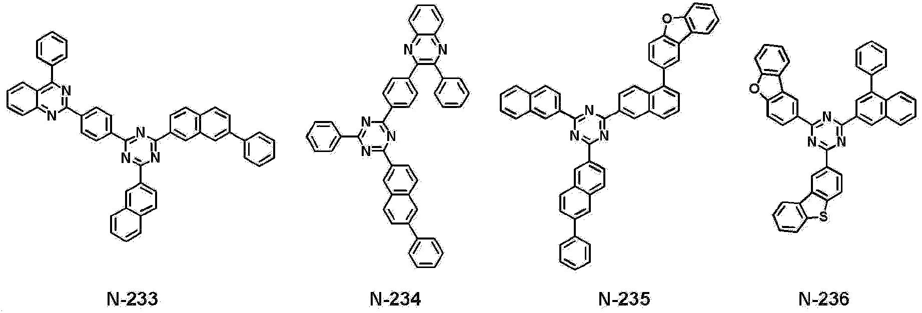

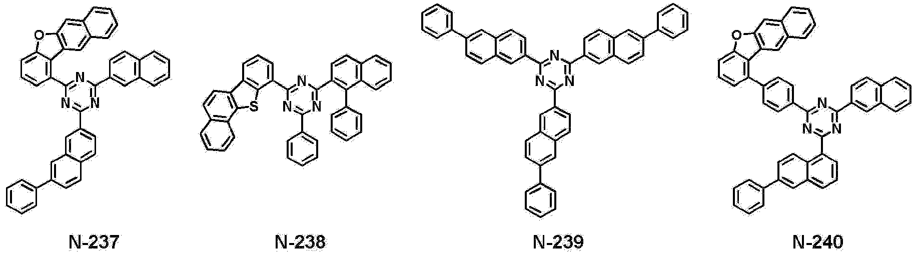

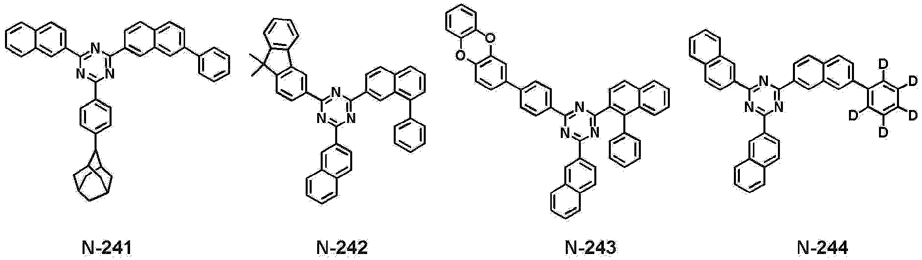

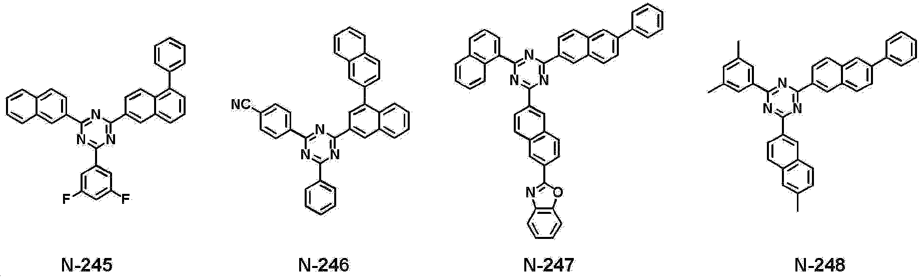

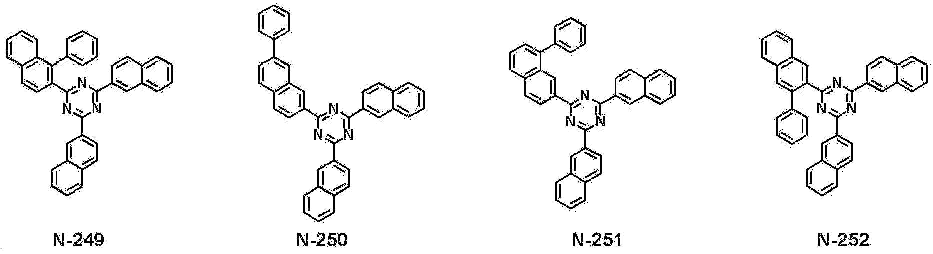

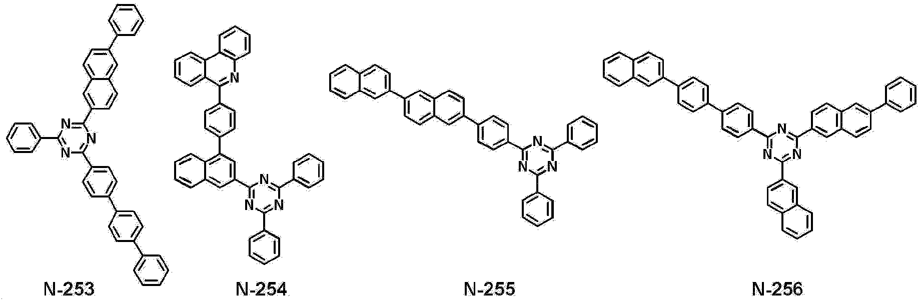

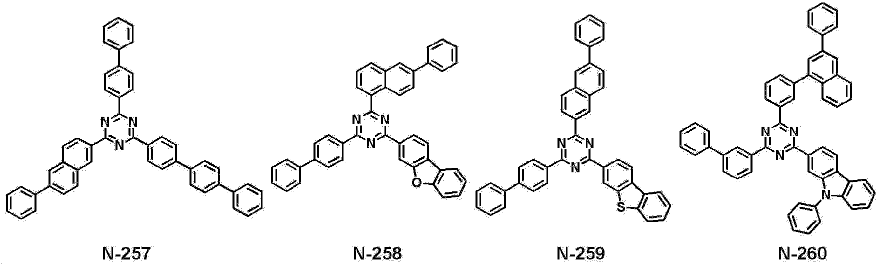

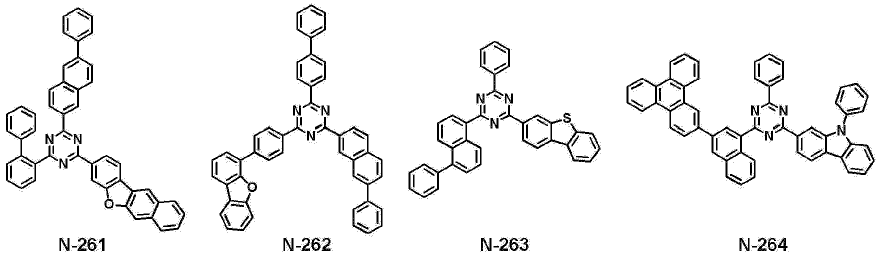

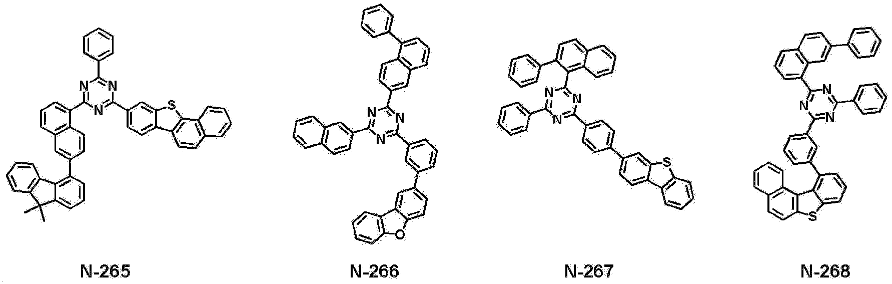

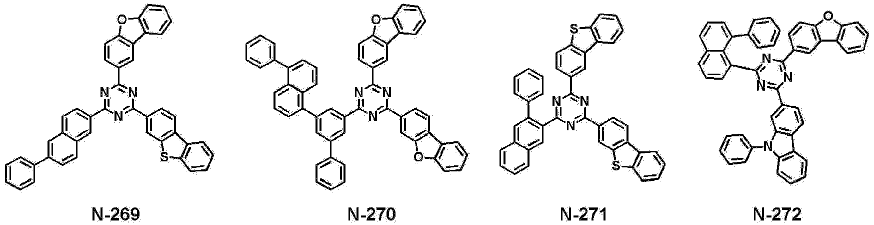

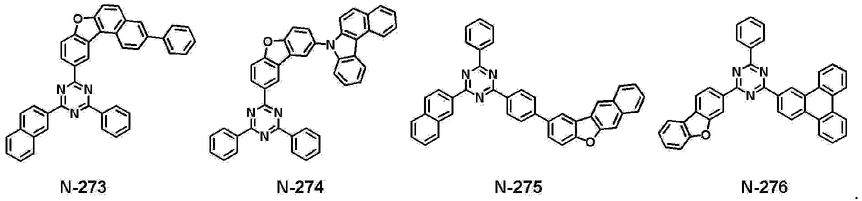

In another aspect, the present invention provides an electronic apparatus comprising a display device comprising an organic electronic device, and a control unit configured to drive the display device, wherein the organic electronic device comprises a compound represented by Formula 1 or compounds represented by Formula 1 and Formula I. Preferably, these compounds are included in an emission layer, and more preferably, they are used as hosts in the emission layer.

In another aspect, the present invention provides a compound represented by Formula 1, which is obtained by recovering and purifying a material of an organic layer from a deposition apparatus after deposition of the organic layer in a process for manufacturing an organic electronic device. The compound obtained through the recovery and purification process has a purity of 99.9% or higher.

In another aspect, the present invention provides a method for recovering a compound represented by Formula 1, the method comprising: depositing an organic layer material comprising the compound represented by Formula 1; recovering the organic layer material that is attached to a deposition apparatus; and purifying the recovered organic layer material to obtain the compound represented by Formula 1 having a purity of 99.9% or higher.

The purification step may comprise: a recrystallization step in which the recovered material of an organic layer is recrystallized using a recrystallization solvent; an adsorption and separation step using an adsorbent; and a sublimation and purification step.

The recrystallization step may comprise a preliminary purification process in which a compound represented by Formula 1 having a purity of 98% is obtained using a recrystallization solvent.

As the recrystallization solvent, a polar solvent having a polarity index (PI) of 5.5 to 7.2 is preferably used, or a mixed solvent comprising a polar solvent having a polarity index of 5.5 to 7.2 and a non-polar solvent having a polarity index of 2.0 to 4.7 may be used.

When a mixed solvent of a polar solvent and a non-polar solvent is used as the recrystallization solvent, the non-polar solvent may be used in an amount of 15% (v/v) or less relative to the volume of the polar solvent.

As the recrystallization solvent, a single solvent of methylpyrrolidone (N-methylpyrrolidone; NMP) is preferably used. Alternatively, a mixed polar solvent in which methylpyrrolidone is mixed with at least one solvent selected from the group consisting of 1,3-dimethyl-2-imidazolidinone, 2-pyrrolidone, N,N-dimethylformamide, dimethylacetamide, and dimethyl sulfoxide may be used. In addition, a single or mixed non-polar solvent selected from the group consisting of toluene, dichloromethane (DCM), dichloroethane (DCE), tetrahydrofuran (THF), chloroform, ethyl acetate, and butanone, or a mixture of the polar solvent and the non-polar solvent may also be used.

The preliminary purification process may comprise dissolving the unpurified organic electroluminescent material, which has been recovered from a deposition apparatus, in a polar solvent at a temperature of 90°C to 120°C, and cooling the solution to a temperature of 0°C to 5°C to precipitate crystals.

The preliminary purification process may comprise dissolving the unpurified organic electroluminescent material, recovered from a deposition apparatus, in a polar solvent at a temperature of 90°C to 120°C; cooling the solution to a temperature of 35°C to 40°C; adding a non-polar solvent to the cooled solution; and further cooling the resulting mixture to a temperature of 0°C to 5°C to precipitate crystals.

The preliminary purification process may comprise dissolving the unpurified organic electroluminescent material, recovered from a deposition apparatus, in a non-polar solvent, and concentrating the solution to remove the non-polar solvent while precipitating crystals.

The preliminary purification process may comprise a step of recrystallization using a polar solvent, followed by a subsequent recrystallization step using a non-polar solvent.

In the adsorption separation step using an adsorbent, the adsorbent may be selected from the group consisting of activated carbon, silica gel, alumina, and other materials known for use in adsorption.

Hereinafter, the present invention will be described in further detail with reference to specific examples regarding the synthesis of compounds represented by Formula 1 and Formula I and the fabrication of an organic electronic device. However, the present invention is not limited to the following examples.

### [Synthesis example 1]

The compound(final products) represented by Formula 1 according to the present invention can be synthesized by reacting Sub 1 and Sub 2 as shown in Reaction Formula 1 below, but is not limited thereto.

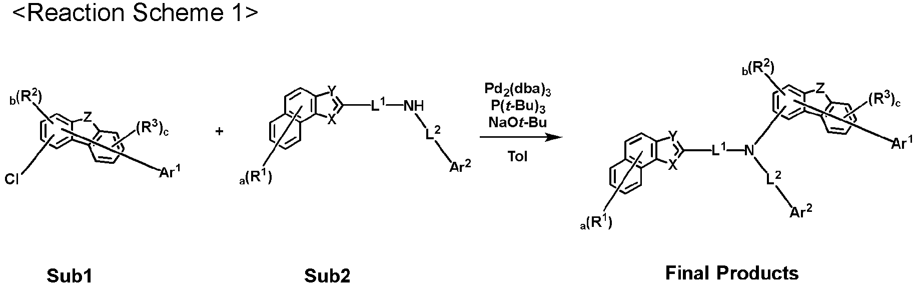

### I. Synthesis of Sub1

Sub1 of the above Reaction Scheme 1 can be synthesized as shown in Reaction Scheme 2 below, but is not limited thereto.

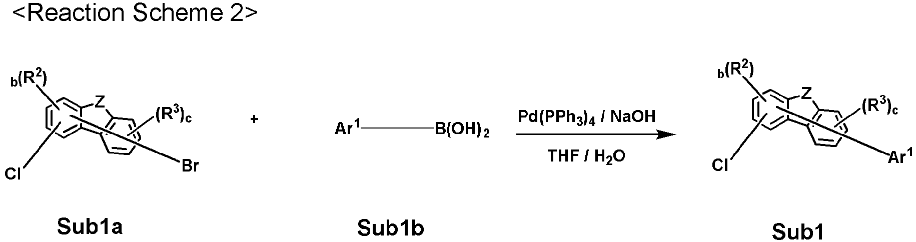

### 1. Synthesis example of Sub1-1

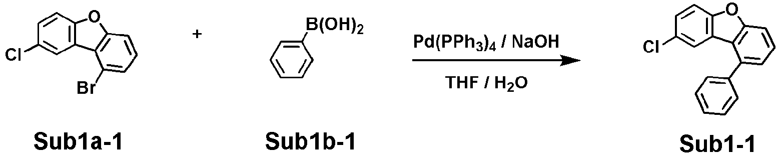

Sub1a-1 (70.00 g, 248.64 mmol) and Sub1b-1 (30.32 g, 248.64 mmol) were added to a round-bottom flask, followed by the addition of THF (tetrahydrofuran) (1200 mL) to dissolve the mixture. Then, NaOH (19.89 g, 497.28 mmol), Pd(PPh₃)₄ (8.62 g, 7.46 mmol), and water (400 mL) were added, and the reaction mixture was stirred at 80 °C. Upon completion of the reaction, the reaction product was extracted with toluene and water. The organic layer was dried over MgSO₄ and concentrated. The concentrate was purified by silica gel column chromatography and recrystallized to afford the product 56.14 g (yield: 81.0%).

### 2. Synthesis example of Sub1-12

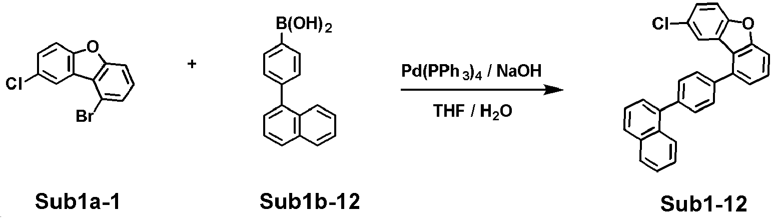

Sub1a-1 (50.00 g, 177.60 mmol) and Sub1b-12 (44.06 g, 177.60 mmol) were added to a round-bottom flask and dissolved in THF (tetrahydrofuran) (900 mL). Then, NaOH (14.21 g, 355.20 mmol), Pd(PPh₃)₄ (6.16 g, 5.33 mmol), and water (300 mL) were added, and the reaction was carried out in the same manner as in the synthesis example of Sub1-1 to obtain 56.8 g(yield: 79.1%) of the product.

### 3. Synthesis example of Sub1-23

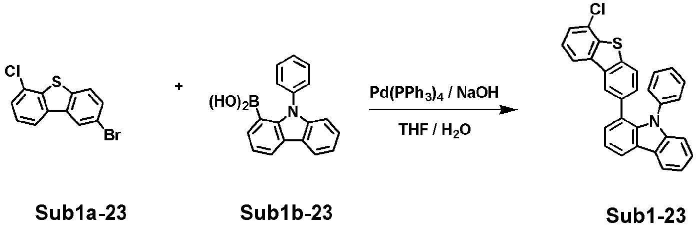

Sub1a-23 (30.00 g, 100.81 mmol) and Sub1b-23 (28.95 g, 100.81 mmol) were added to a round-bottom flask and dissolved in THF (tetrahydrofuran) (500 mL). Then, NaOH (8.06 g, 201.62 mmol), Pd(PPh₃)₄ (3.49 g, 3.02 mmol), and water (150 mL) were added, and the reaction was carried out in the same manner as in the synthesis example of Sub1-1 to obtain 34.55 g (yield: 74.5%) of the product.

### 4. Synthesis example of Sub1-33

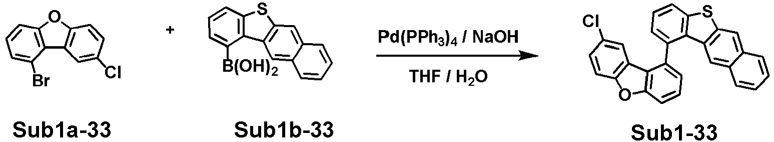

Sub1a-33 (30.00 g, 106.56 mmol) and Sub1b-33 (29.64 g, 106.56 mmol) were added to a round-bottom flask and dissolved in THF (tetrahydrofuran) (530 mL). Then, NaOH (8.52 g, 106.56 mmol), Pd(PPh₃)₄ (3.69 g, 3.20 mmol), and water (170 mL) were added, and the reaction was carried out in the same manner as in the synthesis example of Sub1-1 to obtain 9.53 g (yield: 85.3%) of the product.

### 5. Synthesis example of Sub1-55

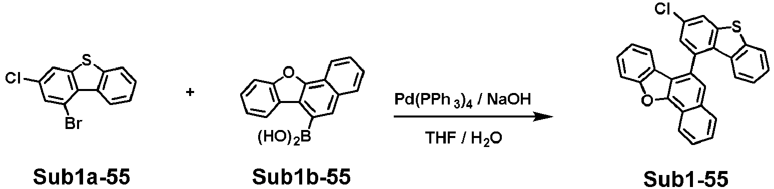

Sub1a-55 (30.00 g, 100.81 mmol) and Sub1b-55 (26.42 g, 100.81 mmol) were added to a round-bottom flask and dissolved in THF (tetrahydrofuran) (500 mL). Then, NaOH (8.06 g, 201.62 mmol), Pd(PPh₃)₄ (3.49 g, 3.02 mmol), and water (150 mL) were added, and the reaction was carried out in the same manner as in the synthesis example of Sub1-1 to obtain 34.86 g (yield: 79.5%) of the product.

### 6. Synthesis example of Sub1-72

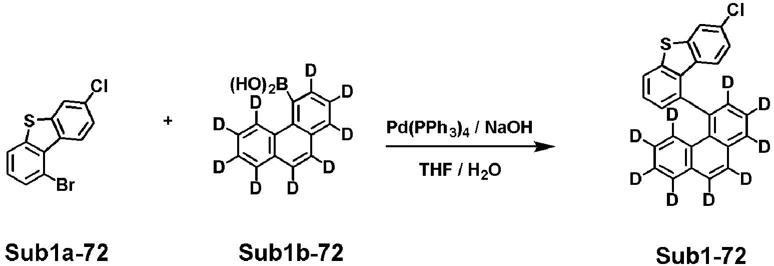

Sub1a-72 (30.00 g, 100.81 mmol) and Sub1b-72 (23.30 g, 100.81 mmol) were added to a round-bottom flask and dissolved in THF (tetrahydrofuran) (500 mL). Then, NaOH (8.06 g, 201.62 mmol), Pd(PPh₃)₄ (3.49 g, 3.02 mmol), and water (150 mL) were added, and the reaction was carried out in the same manner as in the synthesis example of Sub1-1 to obtain 33.76 g (yield: 82.9%) of the product.

### 7. Synthesis example of Sub1-76

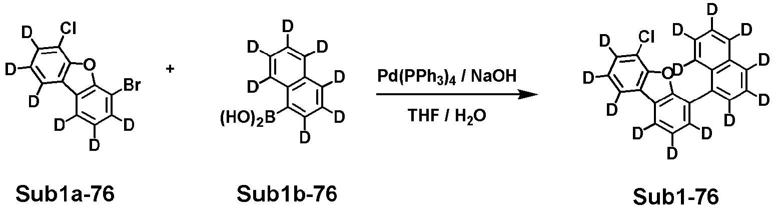

Sub1a-76 (30.00 g, 104.32 mmol) and Sub1b-76 (18.68 g, 104.32 mmol) were added to a round-bottom flask and dissolved in THF (tetrahydrofuran) (510 mL). Then, NaOH (8.35 g, 208.64 mmol), Pd(PPh₃)₄ (3.62 g, 3.13 mmol), and water (170 mL) were added, and the reaction was carried out in the same manner as in the synthesis example of Sub1-1 to obtain 29.00 g (yield: 81.3%) of the product.

The compound belonging to Sub 1 may be the following compound, but is not limited thereto, and the FD-MS (Field Desorption-Mass Spectrometry) values of the following compound are shown in Table 1.

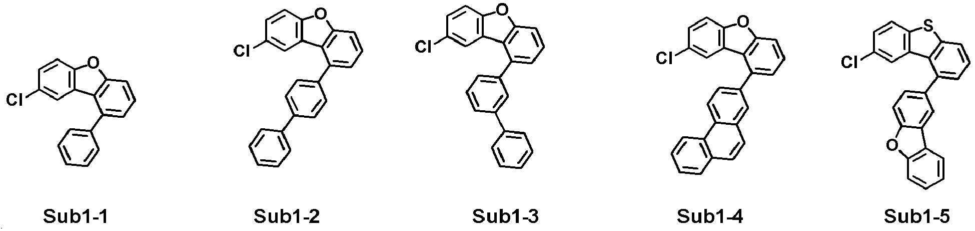

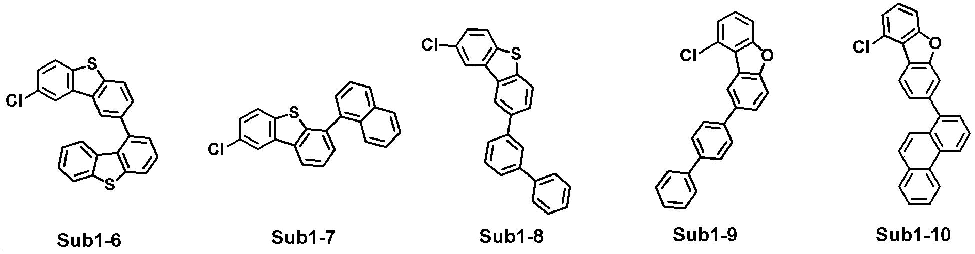

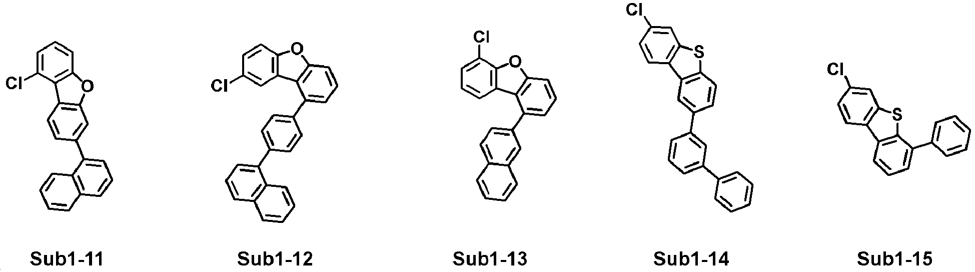

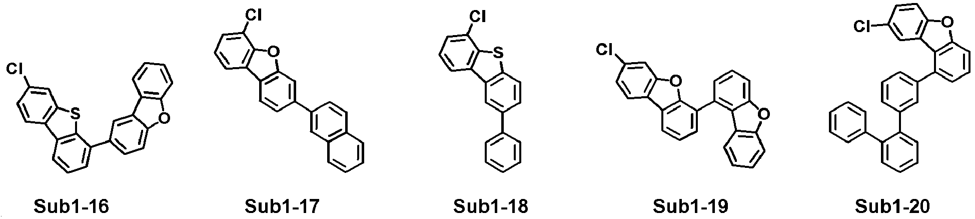

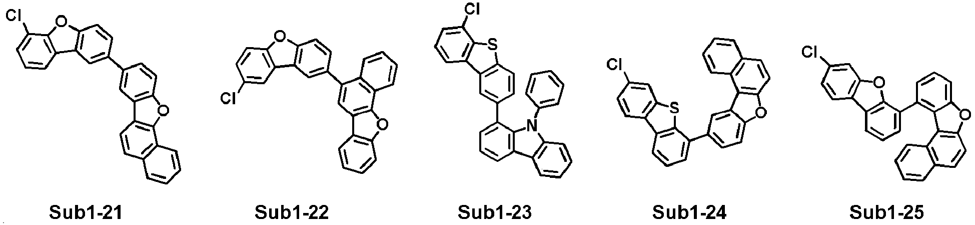

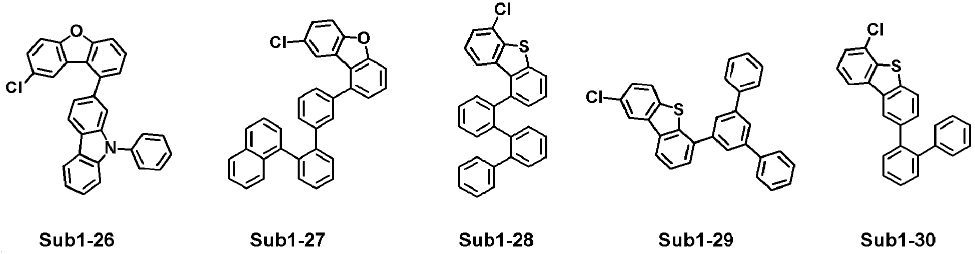

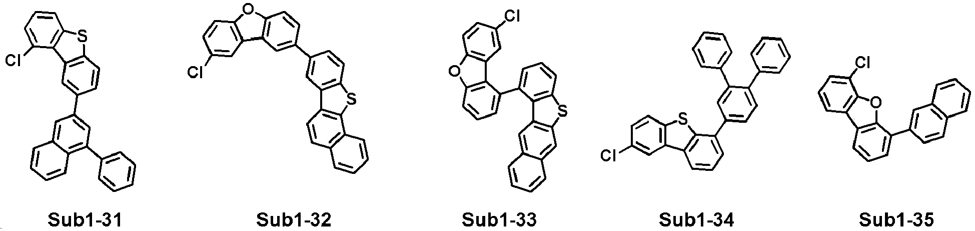

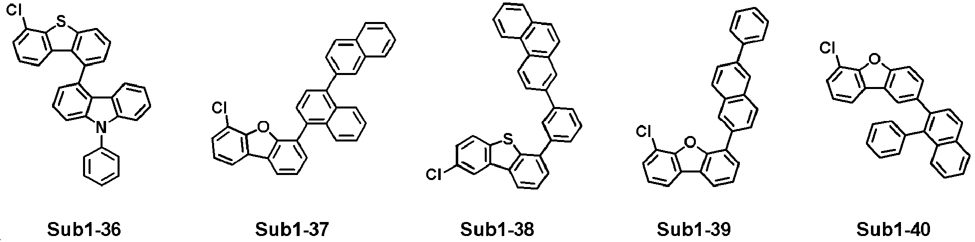

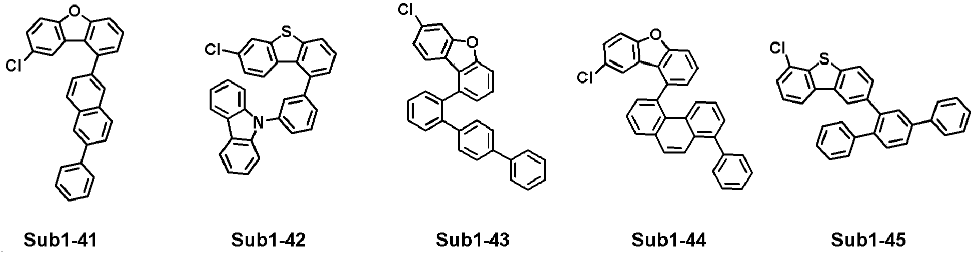

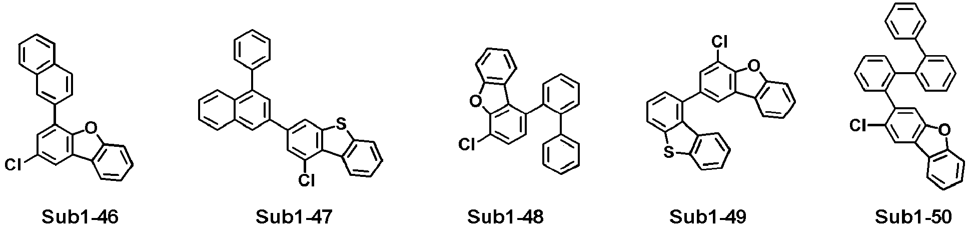

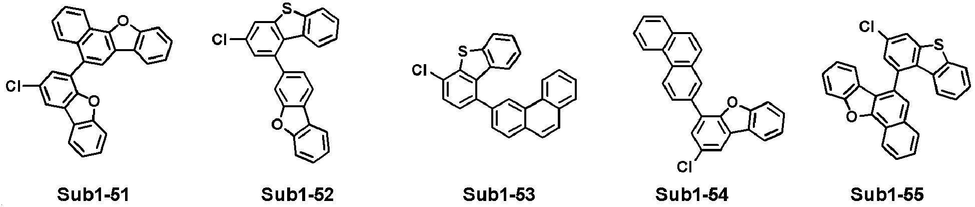

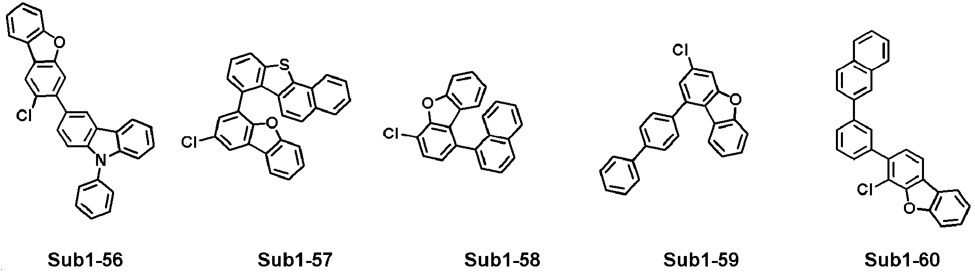

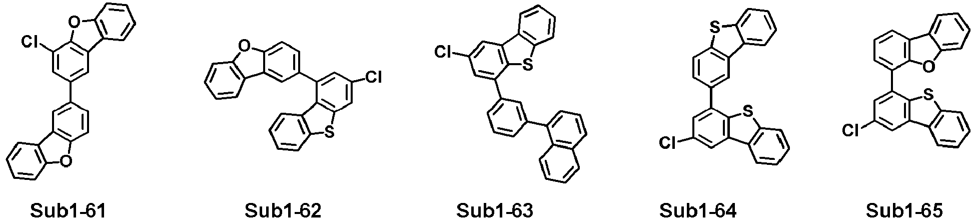

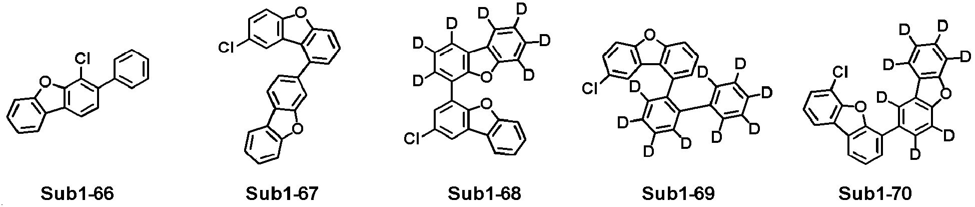

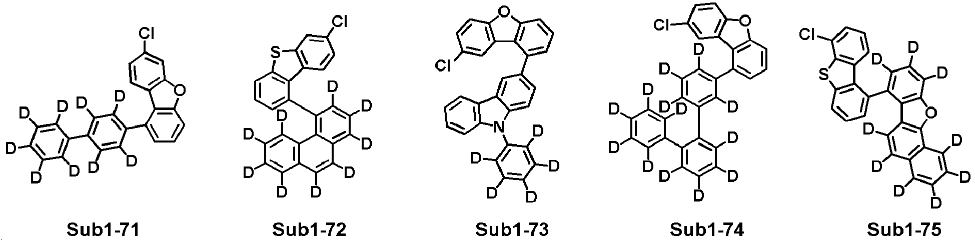

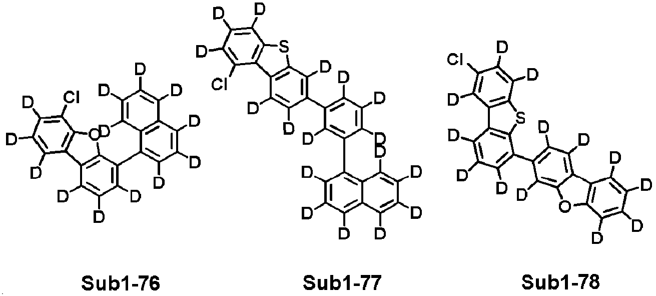

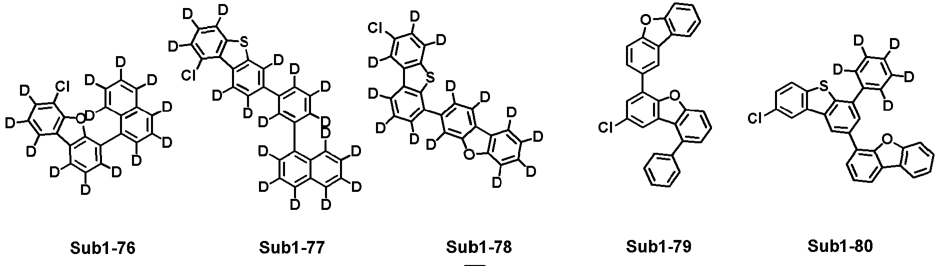

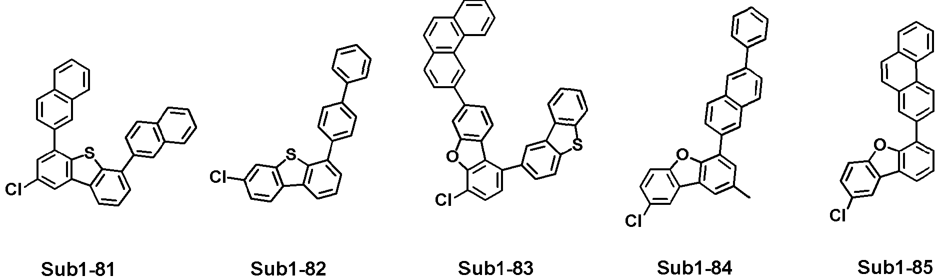

**[Table 1]**

| Compound | FD-MS | Compound | FD-MS |
|---|---|---|---|
| Sub1-1 | m/z=278.05(C₁₈H₁₁ClO=278.74) | Sub1-2 | m/z=354.08(C₂₄H₁₅ClO=354.83) |
| Sub1-3 | m/z=354.08(C₂₄H₁₅ClO=354.83) | Sub1-4 | m/z=378.08(C₂₆H₁₅ClO=378.86) |
| Sub1-5 | m/z=384.04(C₂₄H₁₃ClOS=384.88) | Sub1-6 | m/z=400.01(C₂₄H₁₃ClS₂=400.94) |
| Sub1-7 | m/z=344.04(C₂₂H₁₃ClS=344.86) | Sub1-8 | m/z=370.06(C₂₄H₁₅ClS=370.89) |
| Sub1-9 | m/z=354.08(C₂₄H₁₅ClO=354.83) | Sub1-10 | m/z=378.08(C₂₆H₁₅ClO=378.86) |
| Sub1-11 | m/z=328.07(C₂₂H₁₃ClO=328.79) | Sub1-12 | m/z=404.10(C₂₈H₁₇ClO=404.89) |
| Sub1-13 | m/z=328.07(C₂₂H₁₃ClO=328.79) | Sub1-14 | m/z=370.06(C₂₄H₁₅ClS=370.89) |
| Sub1-15 | m/z=294.03(C₁₈H₁₁ClS=294.80) | Sub1-16 | m/z=384.04(C₂₄H₁₃ClOS=384.88) |
| Sub1-17 | m/z=328.07(C₂₂H₁₃ClO=328.79) | Sub1-18 | m/z=294.03(C₁₈H₁₁ClS=294.80) |
| Sub1-19 | m/z=368.06(C₂₄H_{1 3}ClO₂=368.82) | Sub1-20 | m/z=430.11(C₃₀H₁₉ClO=430.93) |
| Sub1-21 | m/z=418.08(C₂₈H₁₅ClO₂=418.88) | Sub1-22 | m/z=418.08(C₂₈H₁₅ClO₂=418.88) |
| Sub1-23 | m/z=459.08(C₃₀H₁₈ClNS=459.99) | Sub1-24 | m/z=434.05(C₂₈H₁₅ClOS=434.94) |
| Sub1-25 | m/z=418.08(C₂₈H₁₅ClO₂=418.88) | Sub1-26 | m/z=443.11(C₃₀H₁₈ClNO=443.93) |
| Sub1-27 | m/z=480.13(C₃₄H₂₁ClO=480.99) | Sub1-28 | m/z=446.09(C₃₀H₁₉ClS=446.99) |
| Sub1-29 | m/z**=**446.09(C₃₀H₁₉ClS=446.99) | Sub1-30 | m/z=370.06(C₂₄H₁₅ClS=370.89) |
| Sub1-31 | m/z=420.07(C₂₈H₁₇ClS=420.95) | Sub1-32 | m/z=434.05(C₂₈H₁₅ClOS=434.94) |
| Sub1-33 | m/z=434.05(C₂₈H₁₅ClOS=434.94) | Sub1-34 | m/z=446.09(C₃₀H₁₉ClS=446.99) |
| Sub1-35 | m/z=328.07(C₂₂H₁₃ClO=328.79) | Sub1-36 | m/z=459.08(C₃₀H₁₈ClNS=459.99) |
| Sub1-37 | m/z=454.11(C₃₂H₁₉ClO=454.95) | Sub1-38 | m/z=470.09(C₃₂H₁₉ClS=471.01) |
| Sub1-39 | m/z=404.10(C₂sH₁₇ClO=404.89) | Sub1-40 | m/z=404.10(C₂₈H₁₇ClO=404.89) |
| Sub1-41 | m/z=404.10(C₂₈H₁₇ClO=404.89) | Sub1-42 | m/z=459.08(C₃₀H₁₈ClNS=459.99) |
| Sub1-43 | m/z=430.11(C₃₀H₁₉ClO=430.93) | Sub1-44 | m/z=454.11(C₃₂H₁₉ClO=454.95) |
| Sub1-45 | m/z=446.09(C₃₀H₁₉ClS=446.99) | Sub1-46 | m/z=328.07(C₂₂H₁₃ClO=328.79) |
| Sub1-47 | m/z=420.07(C₂₈H₁₇ClS=420.95) | Sub1-48 | m/z=354.08(C₂₄H₁₅ClO=354.83) |
| Sub1-49 | m/z=354.08(C₂₄H₁₅ClO=354.83) | Sub1-50 | m/z=430.11(C₃₀H₁₉ClO=430.93) |
| Sub1-51 | m/z=418.08(C₂₈H₁₅ClO₂=418.88) | Sub1-52 | m/z=384.04(C₂₄H₁₃ClOS=384.88) |
| Sub1-53 | m/z=394.06(C₂₆H₁₅ClS=394.92) | Sub1-54 | m/z=378.08(C₂₆H₁₅ClO=378.86) |
| Sub1-55 | m/z=434.05(C₂₈H₁₅ClOS=434.94) | Sub1-56 | m/z=443.11(C₃₀H₁₈ClNO=443.93) |
| Sub1-57 | m/z=434.05(C₂₈H₁₅ClOS=434.94) | Sub1-58 | m/z=328.07(C₂₂H₁₃ClO=328.79) |
| Sub1-59 | m/z=354.08(C₂₄H₁₅ClO=354.83) | Sub1-60 | m/z=404.10(C₂₈H₁₇ClO=404.89) |
| Sub1-61 | m/z=368.05(C₂₄H₁₃ClO₂=368.82) | Sub1-62 | m/z=384.04(C₂₄H₁₃ClOS=384.88) |
| Sub1-63 | m/z=420.07(C₂₈H₁₇ClS=420.95) | Sub1-64 | m/z**=**400.01(C₂₄H₁₃ClS₂=400.94) |
| Sub1-65 | m/z=384.04(C₂₄H₁₃ClOS=384.88) | Sub1-66 | m/z=278.05(C₁₈H₁₁ClO=278.74) |
| Sub1-67 | m/z=368.05(C₂₄H₁₃ClO₂=368.82) | Sub1-68 | m/z=37510(C₂₄H₆D₇ClO₂=37586) |
| Sub1-69 | m/z=363.14(C₂₄H₆D₉ClO=36389) | Sub1-70 | m/z=375.10(C₂₄H₆D₇ClO₂=375.86) |
| Sub1-71 | m/z=363.14(C₂₄H₆D₉ClO=363.89) | Sub1-72 | m/z=403.11(C₂₆H₆D₉ClS=403.97) |
| Sub1-73 | m/z=448.14(C₃₀H₁₃D₅ClNO=448.96) | Sub1-74 | m/z=443.19(C₃₀H₆D₁₃ClO=444.01) |
| Sub1-75 | m/z=443.11(C₂₈H₆D₉ClOS=443.99) | Sub1-76 | m/z=341.15(C₂₂D₁₃CIO=341.87) |
| Sub1-77 | m/z=437.18(C₂₈D₁₇ClS=438.06) | Sub1-78 | m/z=397.12(C₂₄D₁₃ClOS=397.96) |
| Sub1-79 | m/z=444.09(C₃₀H₁₇ClO₂=444.91) | Sub1-80 | m/z=465.10(C₃₀H₁₂D₅ClOS=466.01) |
| Sub1-81 | m/z=470.09(C₃₂H₁₉ClS=471.01) | Sub1-82 | m/z=370.06(C₂₄H₁₅ClS=370.89) |
| Sub1-83 | m/z=560.10(C₃₈H₂₁ClOS=561.10) | Sub1-84 | m/z=418.11(C₂₉H₁₉ClO=418.92) |
| Sub1-85 | m/z=378.08(C₂₆H₁₅ClO=378.86) | | |

### Synthesis example of Sub2

Sub2 of the above Reaction Scheme 1 may be synthesized as shown in Reaction Scheme 3 below, but is not limited thereto.

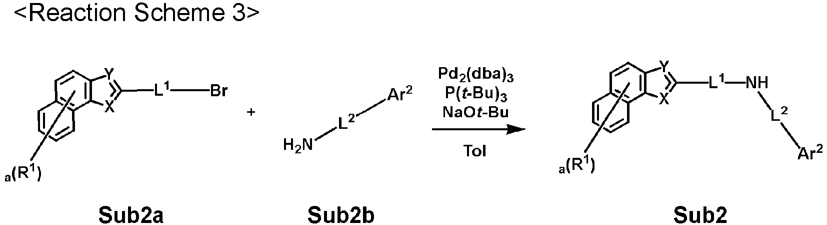

### 1. Synthesis example of Sub2-1

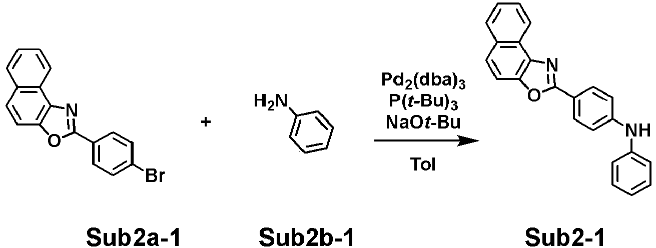

Sub2a-1 (70.00 g, 215.93 mmol) was dissolved in toluene (720 mL), followed by the addition of Sub2b-1 (22.12 g, 237.52 mmol), Pd₂(dba)₃ (5.93 g, 6.48 mmol), P(t-Bu)₃ (2.62 g, 12.96 mmol), and NaOt-Bu (41.51 g, 431.86 mmol). The mixture was stirred at 130 °C. Upon completion of the reaction, the reaction product was extracted with CH₂Cl₂ and water. The organic layer was dried over MgSO₄ and concentrated. The concentrate was purified by silica gel column chromatography and recrystallized to obtain 51.57 g (yield: 71.0%) of the product.

### 2. Synthesis example of Sub2-10

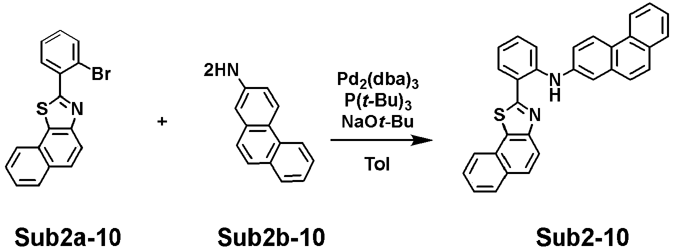

Sub2a-10 (30 g, 88.17 mmol) was dissolved in toluene (300 mL), followed by the addition of Sub2b-10 (18.74 g, 96.99 mmol), Pd₂(dba)₃ (2.42 g, 2.65 mmol), P(t-Bu)₃ (1.07 g, 5.29 mmol), and NaOt-Bu (16.95 g, 176.35 mmol). The reaction was carried out in the same manner as in the synthesis example of Sub2-1 to obtain 29.93 g (yield: 75.0%) of the product.

### 3. Synthesis example of Sub2-24

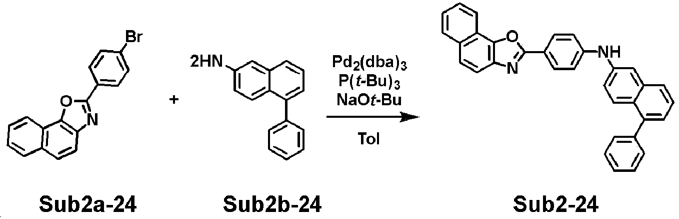

Sub2a-24 (30 g, 92.54 mmol) was dissolved in toluene (310 mL), followed by the addition of Sub2b-24 (22.32 g, 101.80 mmol), Pd₂(dba)₃ (2.54 g, 2.78 mmol), P(*t*-Bu)₃ (1.12 g, 5.55 mmol), and NaOt-Bu (17.79 g, 185.08 mmol). The reaction was carried out in the same manner as in the synthesis example of Sub2-1 to obtain 34.37 g (yield: 80.3%) of the product.

### 4. Synthesis example of Sub2-45

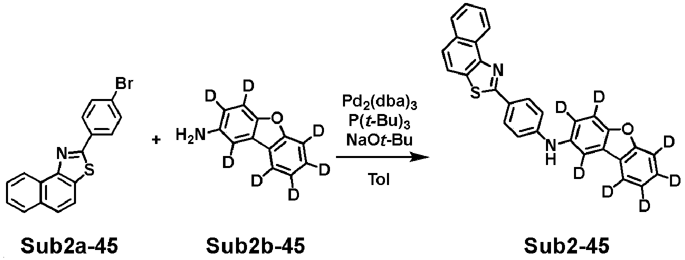

Sub2a-45 (30 g, 88.17 mmol) was dissolved in toluene (300 mL), followed by the addition of Sub2b-45 (18.45 g, 96.99 mmol), Pd₂(dba)₃ (2.42 g, 2.65 mmol), P(*t*-Bu)₃ (1.07 g, 5.29 mmol), and NaOt-Bu (16.95 g, 176.35 mmol). The reaction was carried out in the same manner as in the synthesis example of Sub2-1 to obtain 31.40 g (yield: 79.2) of the product.

### 5. Synthesis example of Sub2-51

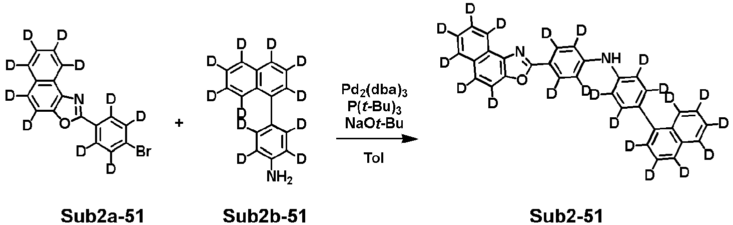

Sub2a-51 (30 g, 89.76 mmol) was dissolved in toluene (300 mL), followed by the addition of Sub2b-51 (22.74 g, 98.73 mmol), Pd₂(dba)₃ (2.47 g, 2.69 mmol), P(*t*-Bu)₃ (1.09 g, 5.39 mmol), and NaOt-Bu (17.25 g, 179.51 mmol). The reaction was carried out in the same manner as in the synthesis example of Sub2-1 to obtain 33.04 g (yield: 76.1%) of the product.

The compound belonging to Sub 2 may be the following compound, but is not limited thereto, and the FD-MS (Field Desorption-Mass Spectrometry) values of the following compound are shown in Table 2.

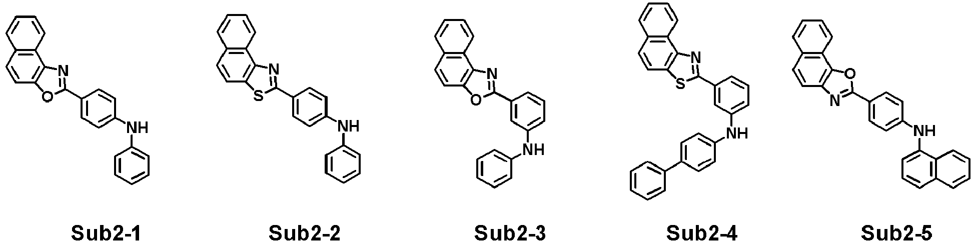

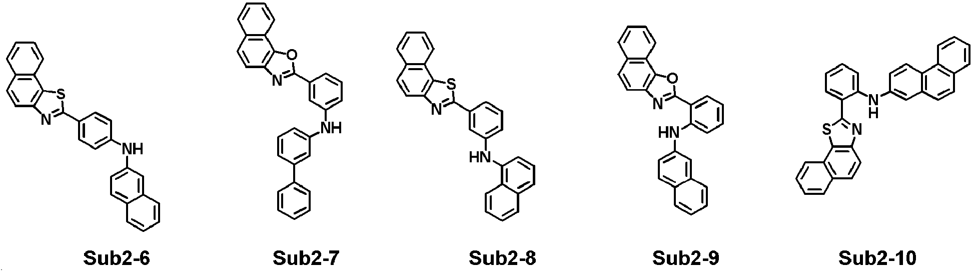

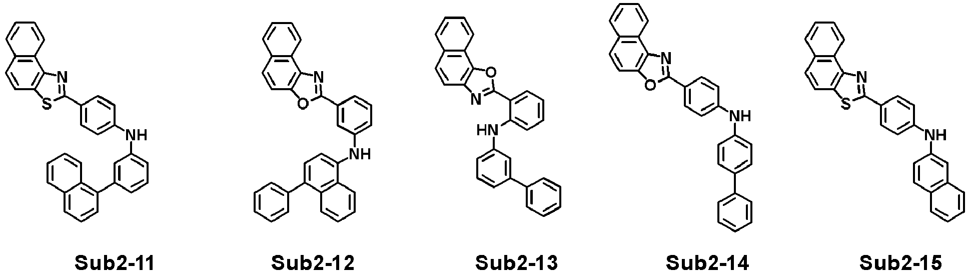

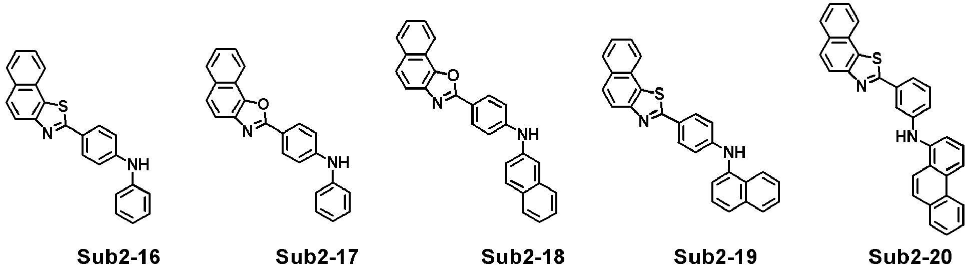

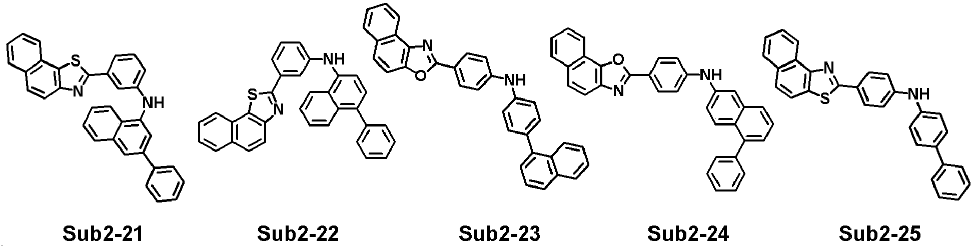

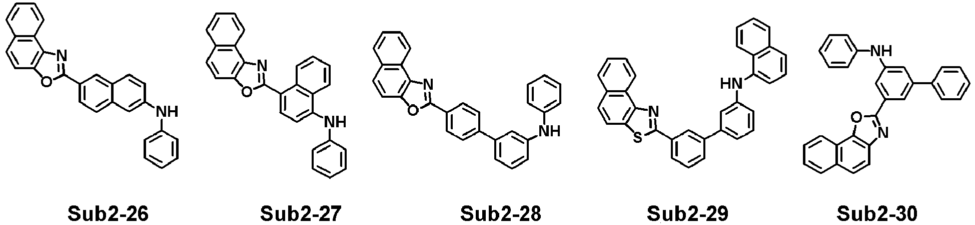

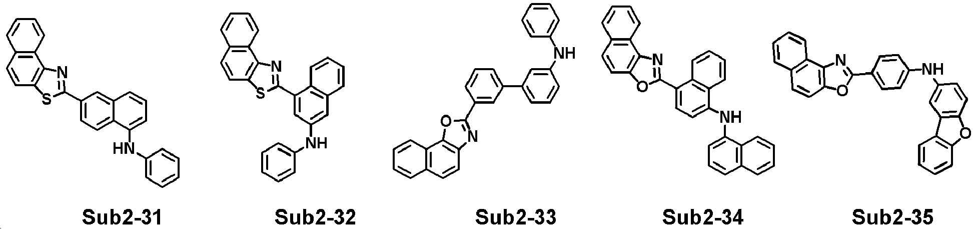

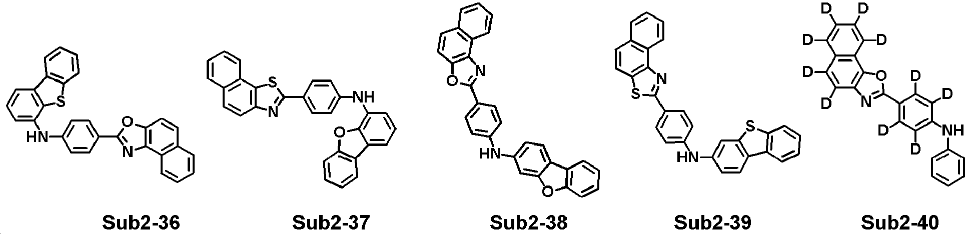

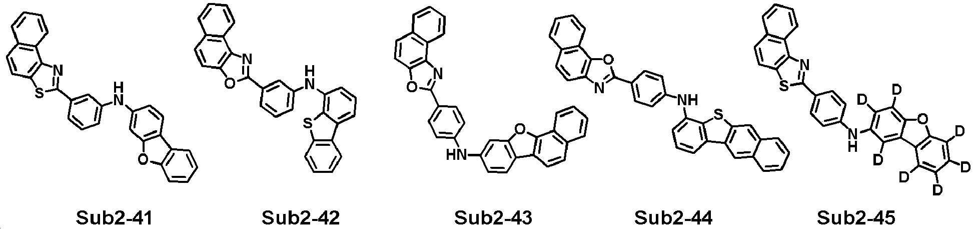

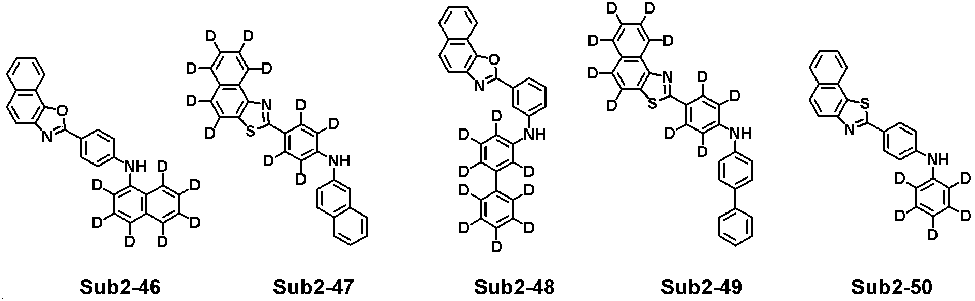

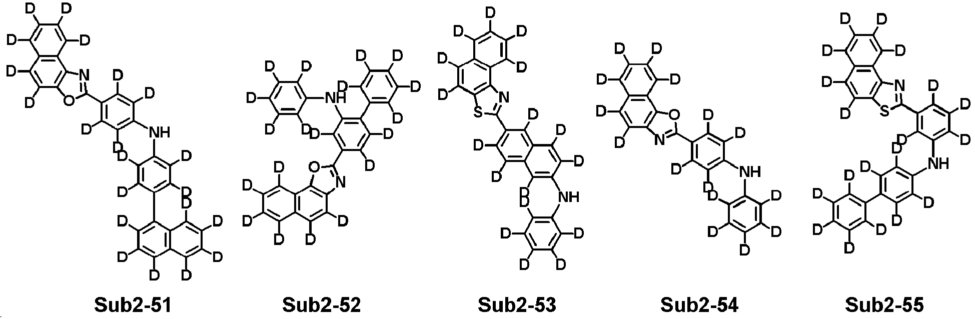

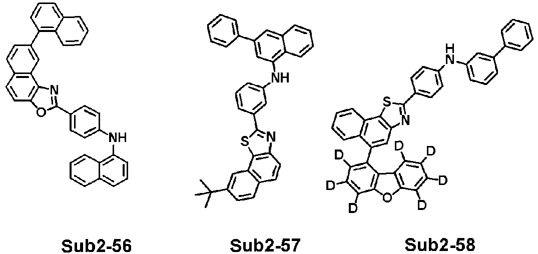

**[Table 2]**

| Compound | FD-MS | Compound | FD-MS |
|---|---|---|---|
| Sub2-1 | m/z=336.13(C₂₃H₁₆N₂O=336.39) | Sub2-2 | m/z=352.10(C₂₃H₁₆N₂S=352.46) |
| Sub2-3 | m/z=336.13(C₂₃H₁₆N₂O=336.39) | Sub2-4 | m/z=428.13(C₂₉H₂₀N₂S=428.55) |
| Sub2-5 | m/z=386.14(C₂₇H₁₈N₂O=386.45) | Sub2-6 | m/z=402.12(C₂₇H₁₈N₂S=402.52) |
| Sub2-7 | m/z=412.16(C₂₉H₂₀N₂O=412.49) | Sub2-8 | m/z=402.12(C₂₇H₁₈N₂S=402.52) |
| Sub2-9 | m/z=386.14(C₂₇H₁₈N₂O=386.45) | Sub2-10 | m/z=452.13(C₃₁H₂₀N₂S=452.58) |
| Sub2-11 | m/z=478.15(C₃₃H₂₂N₂S=478.61) | Sub2-12 | m/z=462.17(C₃₃H₂₂N₂O=462.55) |
| Sub2-13 | m/z=412.16(C₂₉H₂₀N₂O=412.49) | Sub2-14 | m/z=412.16(C₂₉H₂₀N₂O=412.49) |
| Sub2-15 | m/z=402.12(C₂₇H₁₈N₂S=402.52) | Sub2-16 | m/z=352.10(C₂₃H₁₆N₂S=352.46) |
| Sub2-17 | m/z=336.13(C₂₃H₁₆N₂O=336.39) | Sub2-18 | m/z=386.14(C₂₇H₁₈N₂O=386.45) |
| Sub2-19 | m/z=402.12(C₂₇H₁₈N₂S=402.52) | Sub2-20 | m/z=452.13(C₃₁H₂₀N₂S=452.58) |
| Sub2-21 | m/z=478.15(C₃₃H₂₂N₂S=478.61) | Sub2-22 | m/z=478.15(C₃₃H₂₂N₂S=478.61) |
| Sub2-23 | m/z=462.17(C₃₃H₂₂N₂O=462.55) | Sub2-24 | m/z=462.17(C₃₃H₂₂N₂O=462.55) |
| Sub2-25 | m/z=428.13(C₂₉H₂₀N₂S=428.55) | Sub2-26 | m/z=386.14(C₂₇H₁₈N₂O=386.45) |
| Sub2-27 | m/z=386.14(C₂₇H₁₈N₂O=386.45) | Sub2-28 | m/z=412.16(C₂₉H₂₀N₂O=412.49) |
| Sub2-29 | m/z=478.15(C₃₃H₂₂N₂S=478.61) | Sub2-30 | m/z=412.16(C₂₉H₂₀N₂O=412.49) |
| Sub2-31 | m/z=402.12(C₂₇H₁₈N₂S=402.52) | Sub2-32 | m/z=402.12(C₂₇H₁₈N₂S=402.52) |
| Sub2-33 | m/z=412.16(C₂₉H₂₀N₂O=412.49) | Sub2-34 | m/z=436.16(C₃₁H₂₀N₂O=436.51) |
| Sub2-35 | m/z=426.14(C₂₉H₁₈N₂O₂=426.48) | Sub2-36 | m/z=442.11(C₂₉H₁₈N₂OS=442.54) |
| Sub2-37 | m/z=442.11(C₂₉H₁₈N₂OS=442.54) | Sub2-38 | m/z=426.14(C₂₉H₁₈N₂O₂=426.48) |
| Sub2-39 | m/z=458.09(C₂₉H₁₈N₂S₂=458.60) | Sub2-40 | m/z=346.19(C₂₃H₆D₁₀N₂O=346.46) |
| Sub2-41 | m/z=442.11(C₂₉H₁₈N₂OS=442.54) | Sub2-42 | m/z=442.11(C₂₉H₁₈N₂OS=442.54) |
| Sub2-43 | m/z=476.15(C₃₃H₂₀N₂O₂=476.54) | Sub2-44 | m/z=492.13(C₃₃H₂₀N₂OS=492.60) |
| Sub2-45 | m/z=449.16(C₂₉H₁₁D₇N₂OS=449.58) | Sub2-46 | m/z=393.19(C₂₇H₁₁D₇N₂O=393.50) |
| Sub2-47 | m/z=412.18(C₂₇H₈D₁₀N₂S=412.58) | Sub2-48 | m/z=438.20(C₂₉H₁₀D₁₀N₂S=438.61) |
| Sub2-49 | m/z=421.21(C₂₉H₁₁D₉N₂O=421.55) | Sub2-50 | m/z=357.13(C₂₃H₁₁D₅N₂S=357.49) |
| Sub2-51 | m/z=483.31(C₃₃HD₂₁N₂O=483.68) | Sub2-52 | m/z=431.28(C₂₉HD₁₉N₂O=431.61 ) |
| Sub2-53 | m/z=419.23(C₂₇HD₁₇N₂S=419.62) | Sub2-54 | m/z=351.22(C₂₃HD₁₅N₂O=351.49) |
| Sub2-55 | m/z=447.25(C₂₉HD₁₉N₂S=447.67) | Sub2-56 | m/z=512.19(C₃₇H₂₄N₂O=512.61) |
| Sub2-57 | m/z=534.21(C₃₇H₃₀N₂S=534.72) | Sub2-58 | m/z=601.22(C₄₁H₁₉D₇N₂OS=601.77) |

### Synthesis example of final compound

### 1. Synthesis example of P-1

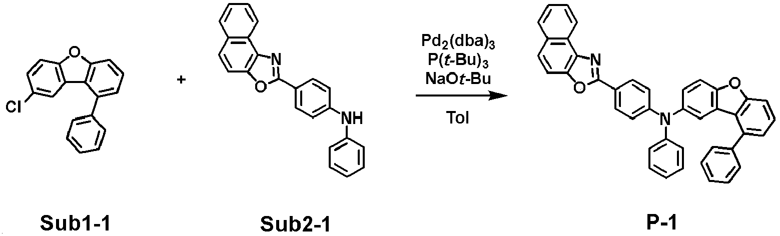

Sub1-1 (20 g, 71.75 mmol) was dissolved in toluene (240 mL), followed by the addition of Sub2-1 (24.14 g, 71.75 mmol), Pd₂(dba)₃ (1.97 g, 2.15 mmol), P(t-Bu)₃ (0.87 g, 4.31 mmol), and NaOt-Bu (13.79 g, 143.50 mmol). The mixture was stirred at 120 °C. Upon completion of the reaction, the reaction product was extracted with CH₂Cl₂ and water. The organic layer was dried over MgSO₄ and concentrated. The concentrate was purified by silica gel column chromatography and recrystallized to obtain 32.10 g (yield: 77.3%) of the product.

### 2. Synthesis example of P-12

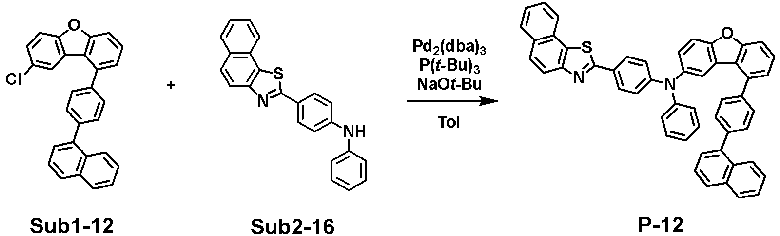

Sub1-12 (20 g, 49.40 mmol) was dissolved in toluene (165 mL), followed by the addition of Sub2-16 (17.41 g, 49.40 mmol), Pd₂(dba)₃ (1.36 g, 1.48 mmol), P(t-Bu)₃ (0.60 g, 2.96 mmol), and NaOt-Bu (9.49 g, 98.79 mmol). The reaction was carried out in the same manner as in the synthesis example of P-1 to obtain 26.92 g (yield: 75.6%) the product.

### 3. Synthesis example of P-28

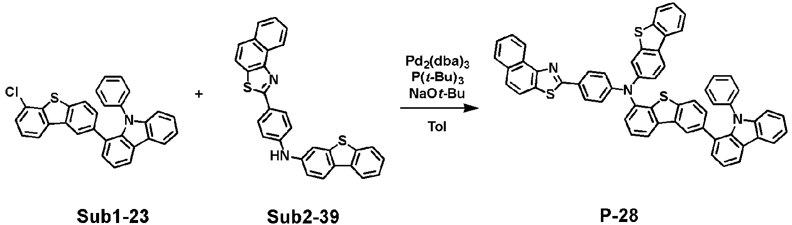

Sub1-23 (20 g, 43.48 mmol) was dissolved in toluene (145 mL), followed by the addition of Sub2-39 (19.94 g, 43.48 mmol), Pd₂(dba)₃ (1.19 g, 1.30 mmol), P(*t*-Bu)₃ (0.53 g, 2.61 mmol), and NaOt-Bu (8.36 g, 86.96 mmol). The reaction was carried out in the same manner as in the synthesis example of P-1 to obtain 32.60 g (yield: 85.0%) the product.

### 4. Synthesis example of P-44

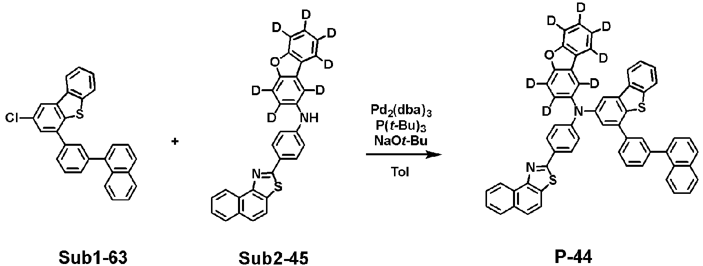

Sub1-63 (20 g, 47.51 mmol) was dissolved in toluene (150 mL), followed by the addition of Sub2-45 (21.36 g, 47.51 mmol), Pd₂(dba)₃ (1.31 g, 1.43 mmol), P(*t*-Bu)₃ (0.58 g, 2.85 mmol), and NaOt-Bu (9.13 g, 95.02 mmol). The reaction was carried out in the same manner as in the synthesis example of P-1 to obtain 31.31 g (yield: 79.0%) the product.

### 5. Synthesis example of P-56

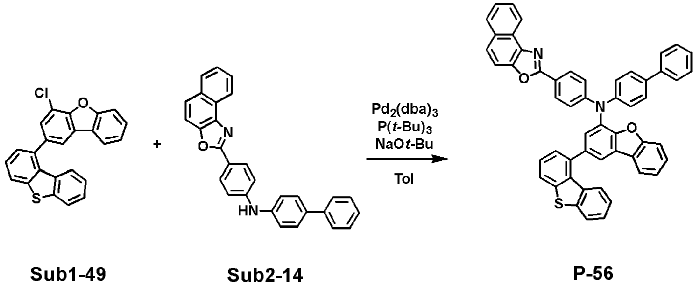

Sub1-49 (20 g, 51.96 mmol) was dissolved in toluene (140 mL), followed by the addition of Sub2-14 (21.43 g, 51.96 mmol), Pd₂(dba)₃ (1.43 g, 1.56 mmol), P(*t*-Bu)₃ (0.63 g, 3.12 mmol), and NaOt-Bu (9.99 g, 103.93 mmol). The reaction was carried out in the same manner as in the synthesis example of P-1 to obtain 30.56 g (yield: 77.3%) the product.

### 6. Synthesis example of P-75

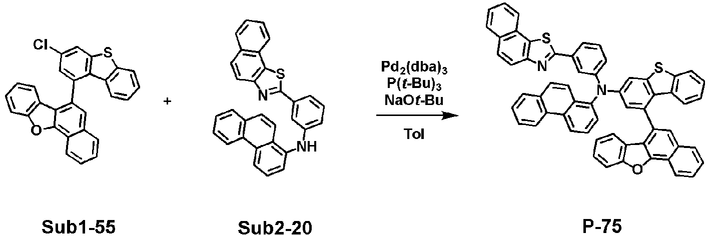

Sub1-55 (20 g, 45.98 mmol) was dissolved in toluene (153 mL), followed by the addition of Sub2-20 (20.81 g, 45.98 mmol), Pd₂(dba)₃ (1.26 g, 1.38 mmol), P(*t*-Bu)₃ (0.56 g, 2.76 mmol), and NaOt-Bu (8.84 g, 91.97 mmol). The reaction was carried out in the same manner as in the synthesis example of P-1 to obtain 27.43 g (yield: 70.1%) the product.

### 7. Synthesis example of P-78

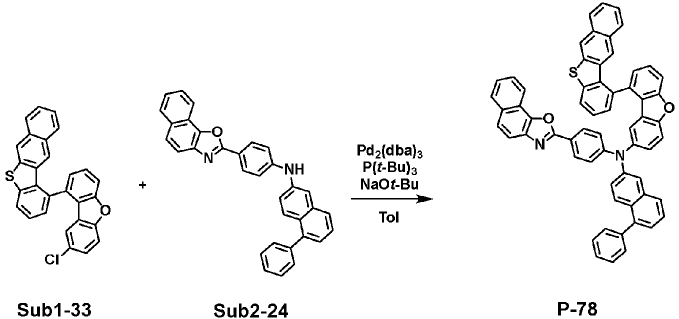

Sub1-33 (20 g, 45.98 mmol) was dissolved in toluene (153 mL), followed by the addition of Sub2-24 (21.27 g, 45.98 mmol), Pd₂(dba)₃ (1.26 g, 1.38 mmol), P(*t*-Bu)₃ (0.56 g, 2.76 mmol), and NaOt-Bu (8.84 g, 91.97 mmol). The reaction was carried out in the same manner as in the synthesis example of P-1 to obtain 28.70 g (yield: 72.5%) the product.

### 8. Synthesis example of P-82

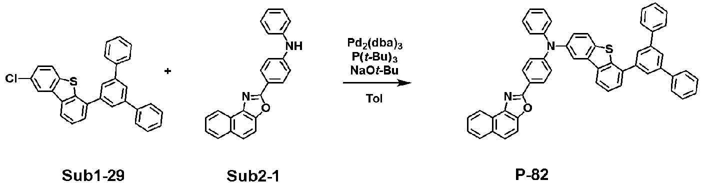

Sub1-29 (20 g, 44.74 mmol) was dissolved in toluene (150 mL), followed by the addition of Sub2-1 (15.05 g, 44.74 mmol), Pd₂(dba)₃ (1.23 g, 1.34 mmol), P(*t*-Bu)₃ (0.54 g, 2.68 mmol), and NaOt-Bu (8.60 g, 89.49 mmol). The reaction was carried out in the same manner as in the synthesis example of P-1 to obtain 25.60 g (yield: 76.6%) the product.

### 9. Synthesis example of P-105

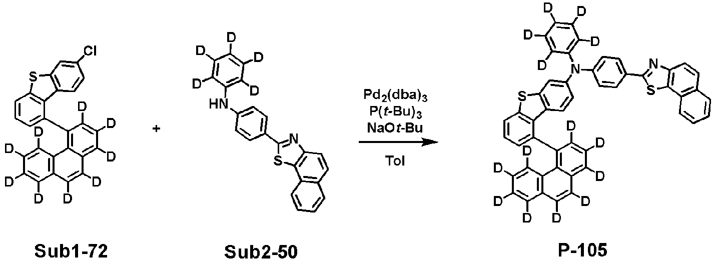

Sub1-72 (20 g, 49.51 mmol) was dissolved in toluene (165 mL), followed by the addition of Sub2-50 (17.70 g, 49.51 mmol), Pd₂(dba)₃ (1.36 g, 1.49 mmol), P(*t*-Bu)₃ (0.60 g, 2.97 mmol), and NaOt-Bu (9.52 g, 99.02 mmol). The reaction was carried out in the same manner as in the synthesis example of P-1 to obtain 29.11 g (yield: 81.1%) the product.

### 10. Synthesis example of P-106

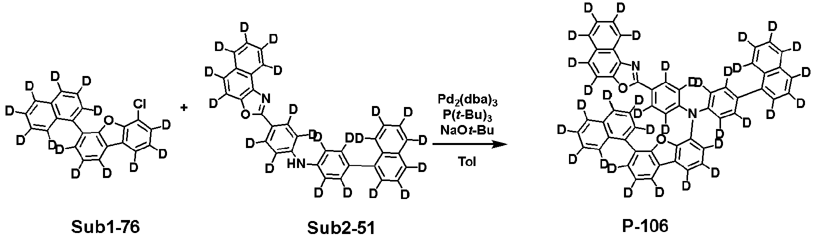

Sub1-76 (20 g, 58.50 mmol) was dissolved in toluene (195 mL), followed by the addition of Sub2-51 (28.30 g, 58.50 mmol), Pd₂(dba)₃ (1.61 g, 1.76 mmol), P(*t*-Bu)₃ (0.71 g, 3.51 mmol), and NaOt-Bu (11.25 g, 117.00 mmol). The reaction was carried out in the same manner as in the synthesis example of P-1 to obtain 32.31 g (yield: 70.0%) the product.

The FD-MS (Field Desorption-Mass Spectrometry) values of the compound represented by Formula 1 of the present invention, prepared according to the synthesis examples described above, are shown in Table 3 below.

**[Table 3]**

| Compound | FD-MS | Compound | FD-MS |
|---|---|---|---|
| P-1 | m/z=578.20(C₄₁H₂₆N₂O₂=578.67) | P-2 | m/z=654.23(C₄₇H₃₀N₂O₂=654.77) |
| P-3 | m/z=654.23(C₄₇H₃₀N₂O₂=654.77) | P-4 | m/z=678.23(C₄₉H₃₀N₂O₂=678.79) |
| P-5 | m/z=684.19(C₄₇H₂₈N₂O₂S=684.81) | P-6 | m/z=700.16(C₄₇H₂₈N₂OS₂=700.87) |
| P-7 | m/z=644.19(C₄₅H₂₈N₂OS=644.79) | P-8 | m/z=670.21(C₄₇H₃₀N₂OS=670.83) |
| P-9 | m/z=670.21(C₄₇H₃₀N₂OS=670.83) | P-10 | m/z=694.21(C₄₉H₃₀N₂OS=694.85) |
| P-11 | m/z=644.19(C₄₅H₂₈N₂OS=644.79) | P-12 | m/z=720.22(C₅₁H₃₂N₂OS=720.89) |
| P-13 | m/z=790.17(C₅₃H₃₀N₂O₂S₂=790.96) | P-14 | m/z=746.24(C₅₃H₃₄N₂OS=746.93) |
| P-15 | m/z=734.20(C₅₁H₃₀N₂O₂S=734.87) | P-16 | m/z=72022(C₅₁H₃₂N₂OS=720.89) |
| P-17 | m/z=720.22(C₅₁H₃₂N₂OS=720.89) | P-18 | m/z=736.20(C₅₁H₃₂N₂S₂=736.95) |
| P-19 | m/z=710.19(C₄₉H₃₀N₂S₂=710.91) | P-20 | m/z=804.28(C₅₉H₃₆N₂O₂=804.95) |
| P-21 | m/z=750.18(C₅₁H₃₀N₂OS₂=750.93) | P-22 | m/z=704.25(C₅₁H₂₁N₂O₂=704.83) |
| P-23 | m/z=660.17(C₄₅H₂₈N₂S₂=660.85) | P-24 | m/z=718.23(C₅₁H₃₀N₂O₃=718.81) |
| P-25 | m/z=730.26(C₅₃H₃₄N₂O₂=730.87) | P-26 | m/z=718.23(C₅₁H₃₀N₂O₃=718.81) |
| P-27 | m/z=817.28(C₅₇H₂₇D₇N₂O₂S=818.01) | P-28 | m/z=881.20(C₅₉H₃₅N₃S₃=882.13) |
| P-29 | m/z=824.21(C₅₇H₃₂N₂O₃S=824.95) | P-30 | m/z=784.22(C₅₅H₃₂N₂O₂S=784.93) |
| P-31 | m/z=849.24(C₅₉H₃₅N₃O₂S=850.01) | P-32 | m/z=780.28(C₅₇H₃₆N₂O₂=780.93) |
| P-33 | m/z=720.22(C₅₁H₃₂N₂OS=720.89) | P-34 | m/z=800.20(C₅₅H₃₂N₂OS₂=800.99) |
| P-35 | m/z=796.25(C₅₇H₃₆N₂OS=796.99) | P-36 | m/z=670.21(C₄₇H₃₀N₂OS=670.83) |
| P-37 | m/z=720.22(C₅₁H₃₂N₂OS=720.89) | P-38 | m/z=780.28(C₅₇H₃₆N₂O₂=780.93) |
| P-39 | m/z=846.27(C₆₁H₃₈N₂OS=847.05) | P-40 | m/z=744.22(C₅₃H₃₂N₂OS=744.91) |
| P-41 | m/z=750.18(C₅₁H₃₀N₂OS₂=750.93) | P-42 | m/z=770.24(C₅₅H₃₄N₂OS=770.95) |
| P-43 | m/z=770.24(C₅₅H₃₄N₂OS=770.95) | P-44 | m/z=815.30(C₅₈H₂₉O₇N₂OS=816.04) |
| P-45 | m/z=750.18(C₅₁H₃₀N₂OS₂=750.93) | P-46 | m/z=694.21(C₄₉H₃₀N₂OS=694.85) |
| P-47 | m/z=734.20(C₅₁H₃₀N₂O₂S=734.87) | P-48 | m/z=858.25(C₆₁H₃₄N₂O₄=858.95) |
| P-49 | m/z=776.20(C₅₃H₃₂N₂OS₂=776.97) | P-50 | m/z=796.25(C₅₇H₃₆N₂OS=796.99) |
| P-51 | m/z=770.24(C₅₅H₃₄N₂OS=770.95) | P-52 | m/z=796.25(C₅₇H₃₆N₂OS=796.99) |
| P-53 | m/z=874.23(C₆₁H₃₄N₂O₃S=875.01) | P-54 | m/z=718.23(C₅₁H₃₀N₂O₃=718.81) |
| P-55 | m/z=809.25(C₅₇H₃₅N₂OS=809.99) | P-56 | m/z=760.22(C₅₃H₃₂N₂O₂S=760.91) |
| P-57 | m/z=770.24(C₅₅H₃₄N₂OS=770.95) | P-58 | m/z=800.20(C₅₅H₃₂N₂OS₂=800.99) |
| P-59 | m/z=875.24(C₆₁H₃₇N₃S₂=876.11) | P-60 | m/z=896.29(C₆₅H₄₀N₂OS=897.11) |
| P-61 | m/z=744.22(C₅₃H₃₂N₂OS=744.91) | P-62 | m/z=901.26(C₆₃H₃₉N₃S₂=902.15) |
| P-63 | m/z=804.28(C₅₉H₃₆N₂O₂=804.95) | P-64 | m/z=744.22(C₅₃H₃₂N₂OS=744.91) |
| P-65 | m/z=578.27(C₄₇H₁₈D₁₀N₂O₃=678.81) | P-66 | m/z=750.18(C₅₁H₃₀N₂OS₂=750.93) |
| P-67 | m/z=776.20(C₅₃H₃₂N₂OS₂=776.97) | P-68 | m/z= 796.25(C₅₇H₃₆N₂OS=796.99) |
| P-69 | m/z=736.20(C₅₁H₃₂N₂S₂=736.95) | P-70 | m/z=744.22(C₅₃H₃₂N₂OS=744.91) |
| P-71 | m/z=680.27(C₄₇H₂₀D₁₀N₂OS=680.89) | P-72 | m/z=761.31(C₅₅H₂₇D₇N₂O₂=761.93) |
| P-73 | m/z=578.20(C₄₁H₂₆N₂O₂=578.67) | P-74 | m/z=825.23(C₅₇H₃₅N₃S₂=826.05) |
| P-75 | m/z=850.21(C₅₉H₃₄N₂OS₂=851.05) | P-76 | m/z=720.22(C₅₁H₃₂N₂OS=720.89) |
| P-77 | m/z=860.25(C₆₁H₃₆N₂O₂S=861.03) | P-78 | m/z=860.25(C₆₁H₃₆N₂O₂S=861.03) |
| P-79 | m/z=838.25(C₅₉H₃₈N₂S₂=839.09) | P-80 | m/z=678.23(C₄₉H₃₀N₂O₂=678.79) |
| P-81 | m/z=796.25(C₅₇H₃₆N₂OS=796.99) | P-82 | m/z=746.24(C₅₃H₃₄N₂OS=746.93) |
| P-83 | m/z=812.23(C₅₇H₃₆N₂S₂=813.05) | P-84 | m/z= 796.25(C₅₇H₃₆N₂OS=796.99) |
| P-85 | m/z=704.25(C₅₁H₂₁N₂O₂=704.83) | P-86 | m/z=870.36(C₅₉H₁₈D₁₉N₃S₂=871.20) |
| P-87 | m/z=896.29(C₆₅H₄₀N₂OS=897.11) | P-88 | m/z=846.27(C₆₁H₃₈N₂OS=847.05) |
| P-89 | m/z=798.30(C₅₇H₃₀D₅N₃O₂=798.96) | P-90 | m/z= 707.29(C₄₉H₁₇D₁₃N₂OS=707.93) |
| P-91 | m/z=813.28(C₅₅H₁₉D₁₃N₂OS₂=814.07) | P-92 | m/z=805.31(C₅₇H₂₇D₉N₂OS=806.04) |
| P-93 | m/z=770.24(C₅₅H₃₄N₂OS=770.95) | P-94 | m/z=770.24(C₅₅H₃₄N₂OS=770.95) |
| P-95 | m/z=780.28(C₅₇H₃₆N₂O₂=780.93) | P-96 | m/z=859.27(C₆₁H₃₇N₃OS=860.05) |
| P-97 | m/z=774.20(C₅₃H₃₀N₂O₃S=774.89) | P-98 | m/z=668.21(C₄₇H₂₈N₂O₃=668.75) |
| P-99 | m/z=805.35(C₅₇H₂₃D₁₂N₃O₂=806.00) | P-100 | m/z=819.40(C₅₇H₁₃D₂₃N₂OS= 820.13) |
| P-101 | m/z=673.35(C₄₇H₁₁D₁₉N₂O₂=673.88) | P-102 | m/z=690.35(C₄₇H₆D₂₂N₂O₃=690.89) |
| P-103 | m/z=828.35(C₅₇H₁₆D₁₈N₂O₂S=829.08) | P-104 | m/z=765.36(C₅₃H₁₅D₁₉N₂OS=766.04) |
| P-105 | m/z=724.27(C₄₉H₁₆D₁₄N₂S₂=725.00) | P-106 | m/z=788.48(C₅₅H₃₄N₂O₂=789.10) |
| P-107 | m/z=832.48(C₅₇D₃₆N₂OS=833.21) | P-108 | m/z=780.37(C₅₁H₃₀N₂OS₂=781.12) |
| P-109 | m/z=830.29(C₆₁H₃₈N₂O₂=830.99) | P-110 | m/z=760.22(C₅₃H₃₂N₂O₂S=760.91) |
| P-111 | m/z=765.25(C₅₃H₂₇D₅N₂O₂S=765.94) | P-112 | m/z=786.22(C₅₅H₃₄N₂S₂=787.01) |
| P-113 | m/z=868.29(C₆₁H₄₄N₂S₂=869.16) | P-114 | m/z=876.23(C₆₁H₃₆N₂OS₂=877.09) |
| P-115 | m/z=784.25(C₅₆H₃₆N₂OS=784.98) | P-116 | m/z=943.32(C₆₇H₃₃D₇N₂O₂S=944.17) |

### Synthesis example of Formula I

The compound represented by Formula I may be prepared with reference to known synthetic methods (named reactions) or disclosed patent publications, such as Korean Patent Publication Nos. 2020-0129334, 2022-0055392, and 2023-0000502, but are not limited thereto.

The FD-MS (Field Desorption-Mass Spectrometry) values of the compounds N-1 to N-276 represented by Formula I are shown in Table 4 below.

**[Table 4]**

| Compound | FD-MS | Compound | FD-MS |
|---|---|---|---|
| N-1 | m/z=399.14(C₂₇H₁₇N₃O=399.45) | N-2 | m/z=415.11(C₂₇H₁₇N₃S=415.51) |
| N-3 | m/z=474.18(C₃₃H₂₂N₄=474.57) | N-4 | m/z=449.15(C₃₁H₁₉N₃O=449.51) |
| N-5 | m/z=449.15(C₃₁H₁₉N₃O=449.51) | N-6 | m/z=515.15(C₃₅H₂₁N₃S=515.63) |
| N-7 | m/z=600.23(C₄₃H₂₈N₄=600.73) | N-8 | m/z=499.17(C₃₅H₂₁N₃O=499.57) |
| N-9 | m/z=551.20(C₃₉H₂₅N₃O=551.65) | N-10 | m/z=567.18(C₃₉H₂₅N₃S=567.71) |
| N-11 | m/z=702.28(C₅₁H₃₄N₄=702.86) | N-12 | m/z=657.22(C₄₆H₃₁N₃S=657.84) |
| N-13 | m/z=551.20(C₃₉H₂₅N₃O=551.65) | N-14 | m/z=541.16(C₃₇H₂₃N₃S=541.67) |
| N-15 | m/z=700.26(C₅₁H₃₂N₄=700.85) | N-16 | m/z=703.21(C₅₀H₂₉N₃S=703.86) |
| N-17 | m/z=525.18(C₃₇H₂₃N₃O=525.61) | N-18 | m/z=591.18(C₄₁H₂₅N₃S=591.73) |
| N-19 | m/z=627.24(C₄₄H₂₉N₅=627.75) | N-20 | m/z=524.20(C₃₇H₂₄N₄=524.63) |
| N-21 | m/z=551.20(C₃₉H₂₅N₃O=551.65) | N-22 | m/z=567.18(C₃₉H₂₅N₃S=567.71) |
| N-23 | m/z=702.28(C₅₁H₃₄N₄=702.86) | N-24 | m/z=474.18(C₃₃H₂₂N₄=474.57) |
| N-25 | m/z= 779.29(C₅₇H₃₇N₃O=779.94) | N-26 | m/z=731.24(C₅₂H₃₃N₃S=731.92) |
| N-27 | m/z=601.23(C₄₂H₂₇N₅=601.71) | N-28 | m/z=475.17(C₃₃H₂₁N₃O=475.55) |
| N-29 | m/z=641.21(C₄₅H₂₇N₃O₂=641.73) | N-30 | m/z=746.21(C₅₁H₃₀N₄OS=746.89) |
| N-31 | m/z=716.26(C₅₁H₃₂N₄₀O=716.84) | N-32 | m/z=681.19(C₄₇H₂₇N₃OS=681.81) |
| N-33 | m/z=475.17(C₃₃H₂₁N₃O=475.55) | N-34 | m/z=491.15(C₃₃H₂₁N₃S=491.61) |
| N-35 | m/z=550.22(C₃₉H₂₆N₄=550.67) | N-36 | m/z=525.18(C₃₇H₂₃N₃O=525.61) |
| N-37 | m/z=475.17(C₃₃H₂₁N₃O=475.55) | N-38 | m/z=491.15(C₃₃H₂₁N₃S=491.61) |
| N-39 | m/z=704.27(C₄₉H₃₂N₆=704.84) | N-40 | m/z=541.16(C₃₇H₂₃N₃S=541.67) |
| N-41 | m/z=551.20(C₃₉H₂₅N₃O=551.65) | N-42 | m/z=541.16(C₃₇H₂₃N₃S=541.67) |
| N-43 | m/z=626.25(C₄₅H₃₀N₄=626.76) | N-44 | m/z=676.26(C₄₉H₃₂N₄=676.82) |
| N-45 | m/z=551.2(C₃₉H₂₅N₃O=551.65) | N-46 | m/z=567.18(C₃₉H₂₅N₃S=567.71) |
| N-47 | m/z=614.25(C₄₄H₃₀N₄=614.75) | N-48 | m/z=575.17(C₃₉H₂₁N₅O=575.63) |
| N-49 | m/z=525.18(C₃₇H₂₃N₃O=525.61) | N-50 | m/z=541.16(C₃₇H₂₃N₃S=541.67) |
| N-51 | m/z=600.23(C₄₃H₂₈N₄=600.73) | N-52 | m/z=625.22(C₄₅H₂₇N₃O=625.73) |
| N-53 | m/z=525.18(C₃₇H₂₃N₃O=525.61) | N-54 | m/z=591.18(C₄₁H₂₅N₃S=591.73) |
| N-55 | m/z=600.23(C₄₃H₂₈N₄=600.73) | N-56 | m/z=693.22(C₄₉H₃₁N₃S=693.87) |
| N-57 | m/z=505.12(C₃₃H₁₉N₃OS=505.60) | N-58 | m/z=641.21(C₄₅H₂₇N₃O₂=641.73) |
| N-59 | m/z=571.12(C₃₇H₂₁N₃S₂=571.72) | N-60 | m/z=564.20(C₃₉H₂₄N₄O=564.65) |
| N-61 | m/z=581.16(C₃₉H₂₃N₃OS=581.69) | N-62 | m/z=521.10(C₃₃H₁₉N₃S₂=521.66) |
| N-63 | m/z=489.15(C₃₃H₁₉N₃O₂=489.53) | N-64 | m/z=640.23(C₄₅H₂₈N₄O=640.75) |
| N-65 | m/z=489.15(C₃₃H₁₉N₃O₂=489.53) | N-66 | m/z=505.12(C₃₃H₁₉N₃OS=505.60) |
| N-67 | m/z=580.17(C₃₉H₂₄N₄S=580.71) | N-68 | m/z=564.20(C₃₉H₂₄N₄O=564.65) |
| N-69 | m/z=489.15(C₃₃H₁₉N₃O₂=489.53) | N-70 | m/z=505.12(C₃₃H₁₉N₃OS=505.60) |
| N-71 | m/z=505.12(C₃₃H₁₉N₃OS=505.60) | N-72 | m/z=639.24(C₄₅H₂₉N₅=639.76) |
| N-73 | m/z=607.21(C₄₂H₂₉N₃S=607.78) | N-74 | m/z=715.26(C₅₂H₃₃N₃O=715.86) |
| N-75 | m/z=640.23(C₄₅H₂₈N₄O=640.75) | N-76 | m/z=707.20(C₄₉H₂₉N₃OS=707.85) |
| N-77 | m/z=591.23(C₄₂H₂₉N₃O=591.71) | N-78 | m/z=617.28(C₄₅H₃₅N₃=617.80) |
| N-79 | m/z=653.25(C₄₇H₃₁N₃O=653.79) | N-80 | m/z=733.22(C₅₁H₃₁N₃OS=733.89) |
| N-81 | m/z=615.19(C₄₃H₂₅N₃O₂=615.69) | N-82 | m/z=681.19(C₄₇H₂₇N₃OS=681.81) |
| N-83 | m/z=716.29(C₅₂H₃₆N₄=716.89) | N-84 | m/z=690.24(C₄₉H₃₀N₄O=690.81) |
| N-85 | m/z=641.25(C₄₆H₃₁N₃O=641.77) | N-86 | m/z=693.22(C₄₉H₃₁N₃S=693.87) |
| N-87 | m/z=690.24(C₄₉H₃₀N₄O=690.81) | N-88 | m/z=631.17(C₄₃H₂₅N₃OS=631.75) |
| N-89 | m/z=595.14(C₃₉H₂₁N₃O₂S=595.68) | N-90 | m/z=659.24(C₄₅H₂₁D₅N₄O₂=659.76) |
| N-91 | m/z=637.16(C₄₂H₂₇N₃S₂=637.82) | N-92 | m/z=729.25(C₅₁H₃₁N₅O=729.84) |
| N-93 | m/z=578.17(C₃₉H₂₂N₄O₂=578.63) | N-94 | m/z=746.21(C₅₁H₃₀N₄OS=746.89) |
| N-95 | m/z=681.24(C₄₈H₃₁N₃O₂=681.80) | N-96 | m/z=762.19(C₅₁H₃₀N₄S₂=762.95) |
| N-97 | m/z=436.17(C₃₀H₂₀N₄=436.52) | N-98 | m/z=437.16(C₂₉H₁₉N₅=437.51) |
| N-99 | m/z=513.20(C₃₅H₂₃N₅=513.60) | N-100 | m/z=589.23(C₄₁H₂₇N₅=589.70) |
| N-101 | m/z=486.18(C₃₄H₂₂N₄=486.58) | N-102 | m/z=527.17(C₃₅H₂₁N₅O=527.59) |
| N-103 | m/z=589.23(C₄₁H₂₇N₅=589.70) | N-104 | m/z=502.18(C₃₄H₂₂N₄O=502.58) |
| N-105 | m/z=511.20(C₃₇H₂₅N₃=511.63) | N-106 | m/z=563.21(C₃₉H₂₅N₅=563.66) |
| N-107 | m/z=511.20(C₃₇H₂₅N₃=511.63) | N-108 | m/z=589.23(C₄₁H₂₇N₅=589.70) |
| N-109 | m/z=513.20(C₃₅H₂₃N₅=513.60) | N-110 | m/z=462.16(C₃₀H₁₈N₆=462.52) |
| N-111 | m/z=612.21(C₄₂H₂₄N₆=612.70) | N-112 | m/z=499.20(C₃₆H₂₅N₃=499.62) |
| N-113 | m/z=569.17(C₃₇H₂₃N₅S=569.69) | N-114 | m/z=629.22(C₄₃H₂₇N₅O=629.72) |
| N-115 | m/z=629.22(C₄₃H₂₇N₅O=629.72) | N-116 | m/z=563.21(C₃₉H₂₅N₅=563.66) |
| N-117 | m/z=565.2(C₃₇H₂₃N₇=565.64) | N-118 | m/z=630.22(C₄₂H₂₆N₆O=630.71) |
| N-119 | m/z=611.24(C₄₅H₂₉N₃=611.75) | N-120 | m/z=803.29(C₅₉H₃₇N₃O=803.97) |
| N-121 | m/z=563.20(C₄₀H₂₅N₃O=563.66) | N-122 | m/z=549.22(C₄₀H₂₇N₃=549.68) |
| N-123 | m/z=449.15(C₃₁H₁₉N₃O=449.51) | N-124 | m/z=579.18(C₄₀H₂₅N₃S=579.72) |
| N-125 | m/z=435.17(C₃₁H₂₁N₃=435.53) | N-126 | m/z=435.17(C₃₁H₂₁N₃=435.53) |
| N-127 | m/z=435.17(C₃₁H₂₁N₃=435.53) | N-128 | m/z=435.17(C₃₁H₂₁N₃=435.53) |
| N-129 | m/z=435.17(C₃₁H₂₁N₃=435.53) | N-130 | m/z=435.17(C₃₁H₂₁N₃=435.53) |
| N-131 | m/z=435.17(C₃₁H₂₁N₃=435.53) | N-132 | m/z=434.18(C₃₂H₂₂N₂=434.54) |
| N-133 | m/z=511.20(C₃₇H₂₅N₃=511.63) | N-134 | m/z=611.24(C₄₅H₂₉N₃=611.75) |
| N-135 | m/z=485.19(C₃₅H₂₃N₃=485.59) | N-136 | m/z=511.2(C₃₇H₂₅N₃=511.63) |
| N-137 | m/z=511.20(C₃₇H₂₅N₃=511.63) | N-138 | m/z=485.19(C₃₅H₂₃N₃=485.59) |
| N-139 | m/z=434.18(C₃₂H₂₂N₂=434.54) | N-140 | m/z=434.18(C₃₂H₂₂N₂=434.54) |
| N-141 | m/z=511.20(C₃₇H₂₅N₃=511.63) | N-142 | m/z=561.22(C₄₁H₂₇N₃=561.69) |
| N-143 | m/z=587.24(C₄₃H₂₉N₃=587.73) | N-144 | m/z=511.20(C₃₇H₂₅N₃=511.63) |
| N-145 | m/z=511.20(C₃₇H₂₅N₃=511.63) | N-146 | m/z=511.20(C₃₇H₂₅N₃=511.63) |
| N-147 | m/z=511.20(C₃₇H₂₅N₃=511.63) | N-148 | m/z=587.24(C₄₃H₂₉N₃=587.73) |
| N-149 | m/z=435.17(C₃₁H₂₁N₃=435.53) | N-150 | m/z=435.17(C₃₁H₂₁N₃=435.53) |
| N-151 | m/z=435.17(C₃₁ H₂₁ N₃=435.53) | N-152 | m/z=435.17(C₃₁ H₂₁N₃=435.53) |
| N-153 | m/z=435.17(C₃₁H₂₁N₃=435.53) | N-154 | m/z=435.17(C₃₁H₂₁N₃=435.53) |
| N-155 | m/z=435.17(C₃₁H₂₁N₃=435.53) | N-156 | m/z=434.18(C₃₂H₂₂N₂=434.54) |
| N-157 | m/z=485.19(C₃₅H₂₃N₃=485.59) | N-158 | m/z=511.2(C₃₇H₂₅N₃=511.63) |
| N-159 | m/z=511.20(C₃₇H₂₅N₃=511.63) | N-160 | m/z=511.20(C₃₇H₂₅N₃=511.63) |
| N-161 | m/z=485.19(C₃₅H₂₃N₃=485.59) | N-162 | m/z=511.20(C₃₇H₂₅N₃=511.63) |
| N-163 | m/z=485.19(C₃₅H₂₃N₃=485.59) | N-164 | m/z=611.24(C₄₅H₂₉N₃=611.75) |
| N-165 | m/z=511.20(C₃₇H₂₅N₃=511.63) | N-166 | m/z=511.20(C₃₇H₂₅N₃=511.63) |
| N-167 | m/z=587.24(C₄₃H₂₉N₃=587.73) | N-168 | m/z=587.24(C₄₃H₂₉N₃=587.73) |
| N-169 | m/z=587.24(C₄₃H₂₉N₃=587.73) | N-170 | m/z=56122(C₄₁H₂₇N₃=56169) |
| N-171 | m/z=511.20(C₃₇H₂₅N₃=511.63) | N-172 | m/z=587.24(C₄₃H₂₉N₃=587.73) |
| N-173 | m/z=485.19(C₃₅H₂₃N₃=485.59) | N-174 | m/z=485.19(C₃₅H₂₃N₃=485.59) |
| N-175 | m/z=485.19(C₃₅H₂₃N₃=485.59) | N-176 | m/z=485.19(C₃₅H₂₃N₃=485.59) |
| N-177 | m/z=485.19(C₃₅H₂₃N₃=485.59) | N-178 | m/z=535.20(C₃₉H₂₅N₃=535.65) |
| N-179 | m/z=485.19(C₃₅H₂₃N₃=485.59) | N-180 | m/z=485.19(C₃₅H₂₃N₃=485.59) |
| N-181 | m/z=561.22(C₄₁H₂₇N₃=561.69) | N-182 | m/z=561.22(C₄₁H₂₇N₃=561.69) |
| N-183 | m/z=561.22(C₄₁H₂₇N₃=561.69) | N-184 | m/z=637.25(C₄₇H₃₁N₃=637.79) |
| N-185 | m/z=561.22(C₄₁H₂₇N₃=561.69) | N-186 | m/z=561.22(C₄₁H₂₇N₃=561.69) |
| N-187 | m/z=637.25(C₄₇H₃₁N₃=637.79) | N-188 | m/z=63 7 25(C₄₇H₃₁N₃=637.79) |
| N-189 | m/z=637.25(C₄₇H₃₁N₃=637.79) | N-190 | m/z=485.19(C₃₅H₂₃N₃=485.59) |
| N-191 | m/z=485.19(C₃₅H₂₃N₃=485.59) | N-192 | m/z=611.24(C₄₅H₂₉N₃=611.75) |
| N-193 | m/z=485.19(C₃₅H₂₃N₃=485.59) | N-194 | m/z=485.19(C₃₅H₂₃N₃=485.59) |
| N-195 | m/z=611.24(C₄₅H₂₉N₃=611.75) | N-196 | m/z=485.19(C₃₅H₂₃N₃=485.59) |
| N-197 | m/z=485.19(C₃₅H₂₃N₃=485.59) | N-198 | m/z=561.22(C₄₁H₂₇N₃=561.69) |
| N-199 | m/z=485.19(C₃₅H₂₃N₃=485.59) | N-200 | m/z=485.19(C₃₅H₂₃N₃=485.59) |
| N-201 | m/z=611.24(C₄₅H₂₉N₃=611.75) | N-202 | m/z=611.24(C₄₅H₂₉N₃=611.75) |
| N-203 | m/z=485.19(C₃₅H₂₃N₃=485.59) | N-204 | m/z=485.19(C₃₅H₂₃N₃=485.59) |
| N-205 | m/z=485.19(C₃₅H₂₃N₃=485.59) | N-206 | m/z=485.19(C₃₅H₂₃N₃=485.59) |
| N-207 | m/z=535.20(C₃₉H₂₅N₃=535.65) | N-208 | m/z=535.2(C₃₉H₂₅N₃=535 65) |
| N-209 | m/z=585.22(C₄₃H₂₇N₃=585.71) | N-210 | m/z=535.2(C₃₉H₂₅N₃=535.65) |
| N-211 | m/z=585.22(C₄₃H₂₇N₃=585.71) | N-212 | m/z=585.22(C₄₃H₂₇N₃=585.71) |
| N-213 | m/z=611.24(C₄₅H₂₉N₃=611.75) | N-214 | m/z=611.24(C₄₅H₂₉N₃=611.75) |
| N-215 | m/z=585.22(C₄₃H₂₇N₃=58571) | N-216 | m/z=611.24(C₄₅H₂₉N₃=611.75) |
| N-217 | m/z=687.27(C₅₁H₃₃N₃=687.85) | N-218 | m/z=611.24(C₄₅H₂₉N₃=611.75) |
| N-219 | m/z=511.20(C₃₇H₂₅N₃=511.63) | N-220 | m/z=611.24(C₄₅H₂₉N₃=611.75) |
| N-221 | m/z=561.22(C₄₁H₂₇N₃=561.69) | N-222 | m/z=587.24(C₄₃H₂₉N₃=587.73) |
| N-223 | m/z=663.27(C₄₉H₃₃N₃=663.82) | N-224 | m/z=713.28(C₅₃H₃₅N₃=713.88) |
| N-225 | m/z=575.20(C₄₁H₂₅N₃O=575.67) | N-226 | m/z=601.22(C₄₃H₂₇N₃O=601.71) |
| N-227 | m/z=700.26(C₅₁H₃₂N₄=700.85) | N-228 | m/z=701.25(C₅₁H₃₁N₃O=701.83) |
| N-229 | m/z=667.21(C₄₇H₂₉N₃S=667.83) | N-230 | m/z=541.16(C₃₇H₂₃N₃S=541.67) |
| N-231 | m/z=612.23(C₄₄H₂₈N₄=612.74) | N-232 | m/z=562.22(C₄₀H₂₆N₄=562.68) |
| N-233 | m/z=689.26(C₄₉H₃₁N₅=689.82) | N-234 | m/z=639.24(C₄₅H₂₉N₅=639.76) |
| N-235 | m/z=701.25(C₅₁H₃₁N₃O=701.83) | N-236 | m/z=631.17(C₄₃H₂₅N₃OS=631.75) |
| N-237 | m/z=625.22(C₄₅H₂₇N₃O=625.73) | N-238 | m/z=59118(C₄₁H₂₅N₃S=59173) |
| N-239 | m/z=687.27(C₅₁H₃₃N₃=687.85) | N-240 | m/z=701.25(C₅₁H₃₁N₃O=701.83) |
| N-241 | m/z=619.30(C₄₅H₃₇N₃=619.81) | N-242 | m/z=601.25(C₄₄H₃₁N₃=601.75) |
| N-243 | m/z=667.23(C₄₇H₂₉N₃O₂=667.77) | N-244 | m/z=540.24(C₃₉H₂OD₅N₃=540.68) |
| N-245 | m/z=521.17(C₃₅H₂₁F₂N₃=521.57) | N-246 | m/z=510.18(C₃₆H₂₂N₄=510.60) |
| N-247 | m/z=652₂3(C₄₆H_{2S}N₄O=652 76) | N-248 | m/z=527.24(C₃₈H₂₉N₃=527.67) |
| N-249 | m/z=535.20(C₃₉H₂₅N₃=535.65) | N-250 | m/z=535.20(C₃₉H₂₅N₃=535.65) |
| N-251 | m/z=535.20(C₃₉H₂₅N₃=535.65) | N-252 | m/z=535.20(C₃₉H₂₅N₃=535.65) |
| N-253 | m/z=587.24(C₄₃H₂₉N₃=587.73) | N-254 | m/z=612.23(C₄₄H₂₈N₄=612.74) |
| N-255 | m/z=561.22(C₄₁H₂₇N₃=561.69) | N-256 | m/z=687.27(C₅₁H₃₃N₃=687.85) |
| N-257 | m/z=663.27(C₄₉H₃₃N₃=663.82) | N-258 | m/z=601.22(C₄₃H₂₇N₃O=601.71) |
| N-259 | m/z=617.19(C₄₃H₂₇N₃S=617.77) | N-260 | m/z=752.29(C₅₅H₃₆N₄=752.92) |
| N-261 | m/z=651.23(C₄₇H₂₉N₃O=651.77) | N-262 | m/z=677.25(C₄₉H₃₁N₃O=677.81) |
| N-263 | m/z=541.16(C₃₇H₂₃N₃S=541.67) | N-264 | m/z=750.28(C₅₅H₃₄N₄=750.91) |
| N-265 | m/z=707.24(C₅₀H₃₃N₃S=707.90) | N-266 | m/z=651.23(C₄₇H₂₀N₃O=651.77) |
| N-267 | m/z=617.19(C₄₃H₂₇N₃S=617.77) | N-268 | m/z=667.21(C₄₇H₂₉N₃S=667.83) |
| N-269 | m/z=631.17(C₄₃H₂₅N₃OS=631.75) | N-270 | m/z=767.26(C₅₅H₃₃N₃O₂=767.89) |
| N-271 | m/z=647.15(C₄₃H₂₅N₃S₂=647.81) | N-272 | m/z=690.24(C₄₉H₃₀N₄O=690.81) |
| N-273 | m/z=575.2(C₄₁H₂₅N₃O=575.67) | N-274 | m/z=614.21(C₄₃H₂₆N₄O=614.71 |
| N-275 | m/z=575.2(C₄₁H₂₅N₃O=575.67) | N-276 | m/z=549.18(C₃₉H₂₃N₃O=549.63) |

Although the above description provides synthesis examples of the compounds represented by Formula 1 and Formula I, these are all based on reactions such as the Buchwald-Hartwig cross-coupling reaction, Miyaura boration reaction, Suzuki cross-coupling reaction, intramolecular acid-induced cyclization (J. Mater. Chem. 1999, 9, 2095), Pd(II)-catalyzed oxidative cyclization (Org. Lett. 2011, 13, 5504), and PPh₃-mediated reductive cyclization (J. Org. Chem. 2005, 70, 5014). It will be readily understood by those skilled in the art that these reactions may proceed even when substituents other than those specifically described in the synthesis examples are introduced, as long as they fall within the scope defined for Formula 1 or Formula I.

### Manufacturing and Evaluation of organic electronic device

### [Test Example 1] Red organic electroluminescent device (phosphorescent host)

A hole injection layer with a thickness of 10 nm is formed by vacuum-depositing N-([1,1'-biphenyl]-4-yl)-9,9-dimethyl-N-(4-(9-phenyl-9H-carbazol-3-yl)phenyl)-9H-fluoren-2-amine (hereinafter referred to as Compound A) and 4,4',4"-((1E,1'E,1"E)-cyclopropane-1,2,3-triylidenetris(cyanomethaneylylidene))tris(2,3,5,6-tetrafluorobenzonitrile) (hereinafter referred to as Compound B) on an ITO layer (anode) formed on a glass substrate. At this time, Compound B is doped so that the weight ratio of Compound A to Compound B is 98:2.

Next, a hole transport layer with a thickness of 110 nm is formed by vacuum-depositing Compound A on the hole injection layer.

Subsequently, an emission-auxiliary layer with a thickness of 15 nm is formed by vacuum-depositing *N*⁷-(dibenzo[b,d]thiophen-2-yl)-*N*²,*N*²,*N*⁷-triphenyldibenzo[b,d]thiophene-2,7-diamine on the hole transport layer.

Next, an emission layer with a thickness of 30 nm is formed by vacuum-depositing host and dopant on the emission-auxiliary layer, wherein the host is a mixture of Compound P-2 (a first host) and Compound N-210 (a second host) in a 5:5 weight ratio, and the dopant is bis-(1-phenylisoquinolyl)iridium(III) acetylacetonate (hereinafter abbreviated as '(piq)₂Ir(acac)'). At this time, the dopant is doped such that the weight ratio of the host to the dopant is 95:5.

Next, a hole blocking layer with a thickness of 10 nm is formed by vacuum-depositing 2-(4'-(9,9-dimethyl-9H-fluoren-2-yl)-[1,1'-biphenyl]-3-yl)-4,6-diphenyl-1,3,5-triazine on the emission layer.

Subsequently, an electron transport layer with a thickness of 30 nm is formed by vacuum-depositing a mixture of 2,7-bis(4-(4,6-diphenyl-1,3,5-triazin-2-yl)phenyl)naphthalene and (8-quinolinolato)lithium in a 5:5 weight ratio on the hole blocking layer.

Then, an electron injection layer with a thickness of 0.2 nm is formed by vacuum-depositing (8-quinolinolato)lithium on the electron transport layer, followed by deposition of Al to form a cathode with a thickness of 150 nm.

### [Test Examples 2] to [Test Example 28]

Organic electroluminescent devices were fabricated in the same manner as in Example 1, except that the compounds listed in Table 5 below were used as a first host and a second host of the emission layer.

### [Comparative Examples 1] to [Comparative Examples 4]

Organic electroluminescent devices were fabricated in the same manner as in Example 1, except that one of the Comparative Compounds A to F below was used as a first host of the emission layer.

### <Comparative Compd A> <Comparative Compd B> <Comparative Compd C>

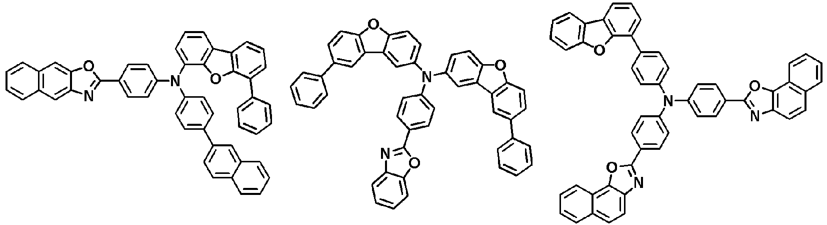

### <Comparative Compd D> <Comparative Compd E> <Comparative Compd F>

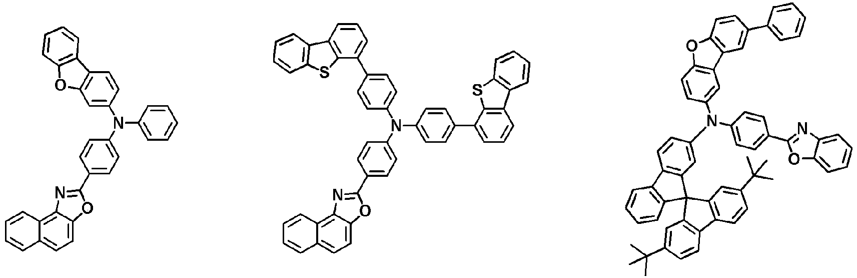

The electroluminescent (EL) characteristics of the organic electroluminescent devices fabricated according to the Examples and Comparative Examples of the present invention were measured by applying a forward DC voltage using a PR-650 photometer from Photo Research. The T95 lifetime was measured at a standard luminance of 2500 cd/m² using a lifetime measurement system manufactured by Mc Science. The measurement results are shown in Table 5 below.

The measuring device is independent from possible day-to-day variations of deposition rates, vacuum quality or other tool performance parameters, and allows assessing performance of new material in comparison with comparative compound under the same conditions. At the time of assessment, each batch contained 4 identically prepared OLEDs including a comparative compound, and since the performance of each of a total of 12 OLEDs in 3 batchs is evaluated, the statistical evaluation of the obtained experimental results unequivocally showed the statistical significance.

**[Table 5]**

| | Compound 1 | Compound 2 | Voltage (V) | Current Density (mA/cm²) | Efficiency (cd/A) | T(95) |
|---|---|---|---|---|---|---|
| comp.Ex1 | Comp. compd A | Com.(N-210) | 5.1 | 10.4 | 24.1 | 102.3 |
| comp.Ex2 | Comp. compd B | Com.(N-210) | 5.1 | 10.2 | 24.5 | 103.4 |
| comp.Ex3 | Comp. compd C | Com.(N-210) | 4.9 | 10.0 | 25.1 | 104.6 |
| comp.Ex4 | Comp. compd D | Com.(N-210) | 4.8 | 9.8 | 25.6 | 105.2 |
| comp.Ex5 | Comp. compd E | Com.(N-210) | 4.7 | 9.5 | 26.3 | 106.1 |
| comp.Ex6 | Comp. compd F | Com.(N-210) | 5.0 | 10.5 | 23.9 | 91.5 |
| Test Ex.1 | Com.(P-2) | Com.(N-210) | 4.3 | 5.6 | 44.5 | 144.0 |
| Test Ex.2 | Com.(P-5) | Com.(N-210) | 4.3 | 5.7 | 44.1 | 143.4 |
| Test Ex.3 | Com.(P-9) | Com.(N-210) | 4.4 | 6.4 | 39.1 | 135.3 |
| Test Ex.4 | Com.(P-11) | Com.(N-210) | 4.4 | 6.4 | 38.9 | 134.3 |
| Test Ex.5 | Com.(P-17) | Com.(N-210) | 4.4 | 6.4 | 39.2 | 135.6 |
| Test Ex.6 | Com.(P-19) | Com.(N-210) | 4.5 | 7.0 | 35.5 | 124.6 |
| Test Ex.7 | Com.(P-26) | Com.(N-210) | 4.5 | 7.1 | 35.0 | 122.9 |
| Test Ex.8 | Com.(P-73) | Com.(N-210) | 4.5 | 7.2 | 34.6 | 121.2 |
| Test Ex.9 | Com.(P-77) | Com.(N-210) | 4.4 | 6.7 | 37.1 | 131.6 |
| Test Ex.10 | Com.(P-85) | Com.(N-210) | 4.4 | 6.9 | 36.3 | 127.6 |
| Test Ex.11 | Com.(P-92) | Com.(N-210) | 4.3 | 6.1 | 41.3 | 142.9 |
| Test Ex.12 | Com.(P-104) | Com.(N-210) | 4.4 | 6.2 | 40.3 | 137.2 |
| Test Ex.13 | Com.(P-2) | Com.(N-274) | 4.3 | 5.7 | 43.5 | 147.2 |
| Test Ex.14 | Com.(P-5) | Com.(N-274) | 4.3 | 5.8 | 43.1 | 146.6 |
| Test Ex.15 | Com.(P-9) | Com.(N-274) | 4.5 | 6.5 | 38.2 | 138.3 |
| Test Ex.16 | Com.(P-11) | Com.(N-274) | 4.5 | 6.6 | 38.0 | 137.3 |
| Test Ex.17 | Com.(P-17) | Com.(N-274) | 4.4 | 6.5 | 38.3 | 138.5 |
| Test Ex.18 | Com.(P-19) | Com.(N-274) | 4.5 | 7.2 | 34.7 | 127.3 |
| Test Ex.19 | Com.(P-26) | Com.(N-274) | 4.5 | 7.3 | 34.2 | 125.6 |
| Test Ex.20 | Com.(P-73) | Com.(N-274) | 4.6 | 7.4 | 33.8 | 123.9 |
| Test Ex.21 | Com.(P-77) | Com.(N-274) | 4.5 | 6.9 | 36.3 | 134.5 |
| Test Ex.22 | Com.(P-85) | Com.(N-274) | 4.5 | 7.0 | 35.5 | 130.4 |
| Test Ex.23 | Com.(P-92) | Com.(N-274) | 4.4 | 6.2 | 40.4 | 146.1 |
| Test Ex.24 | Com.(P-104) | Com.(N-274) | 4.4 | 6.3 | 39.4 | 140.2 |

From Table 5, it can be confirmed that when the material for an organic electroluminescent device of the present invention is used as a phosphorescent host material, the driving voltage, efficiency, and lifetime are significantly improved, compared to the use of any one of Comparative Compounds A to F.

This shows that in the case of a mixed host, the device characteristics vary significantly depending on the types of compounds combined.

The compound of Formula 1 of the present invention is substituted at the nitrogen of a monoamine with naphthooxazole or naphthothiazole, and with dibenzofuran or dibenzothiophene, wherein Ar¹ is substituted on the dibenzofuran or dibenzothiophene moiety.

In contrast, Comparative Compounds B and F differ from the compound of the present invention in that benzoxazole, rather than naphthooxazole, is substituted, and although Comparative compound A is identical to the present invention in that the naphthoxazole structure is substituted, but the structure of the naphthooxazole differs from that of the compound of the present invention.

In addition, Comparative Compounds C to E differ in that the substituent corresponding to Ar¹ of the present invention is not substituted on the dibenzofuran or dibenzothiophene.

Due to these structural differences, the physical properties of the compounds vary, which in turn appears to affect the performance of the organic electroluminescent device when used as a host.

To find out the effect of these structural differences on device characteristics, the energy levels of Compound P-2 of the present invention and Comparative Compounds A to E were measured using the DFT method (B3LYP/6-31g(D)) with the Gaussian program. The measurement results are shown in Table 6 below.

**[Table 6]**

| | HOMO (eV) |
|---|---|
| P-2 | -4.93 |
| Comp. compd A | -5.08 |
| Comp. compd B | -5.04 |
| Comp. compd C | -5.03 |
| Comp. compd D | -5.01 |
| Comp. compd E | -5.00 |

As can be seen from the results in Table 6, the HOMO level of Compound P-2 of the present invention is lower than those of Comparative Compounds A to E.

Compared to Comparative Compound B, which is substituted with a benzoxazole moiety, the compound of the present invention substituted with a naphthooxazole moiety has a lower HOMO value, and even among compounds substituted with a naphthooxazole moiety, the compound of the present invention exhibits a lower HOMO value than Comparative Compound A, which has a different structure.

In addition, Comparative Compounds C to E and the present invention differ in the presence or absence of substituent on dibenzofuran or dibenzothiophene. It can be seen that when Ar¹ is substituted on dibenzofuran or dibenzothiophene, a synergistic effect with other substituents such as naphthooxazole or naphthothiazole results in a lower HOMO value.

Therefore, when the compound of the present invention is used as a host, holes can be more smoothly transported as a host in the emission-auxiliary layer, and the hole transport performance from the host to the dopant is also excellent, resulting in improved efficiency of device.

These results suggest that even monoamine compounds with similar structures exhibit different physical properties depending on the presence and type of substituents, as well as the bonding pattern of naphthooxazole, and therefore unpredictable effects may occur when such different compounds are used in organic electronic devices. Therefore, even those skilled in the art of organic electronic devices would find it difficult to modify a structure similar to compound of the present invention into the structure of the present invention, and it can be understood that significant and unpredictable effects may arise due to structural differences.

The foregoing description is merely illustrative of the present invention and various modifications may be made by those of ordinary skill in the art without departing from the essential characteristics of the invention. The scope of protection of the present invention should be interpreted based on the following claims, and all technical equivalents thereof should be construed as falling within the scope of the invention.

**[Description of Reference Numerals]**

| | | | |
|---|---|---|---|
| 100, 200, 300: | organic electronic device | 110: | first electrode |
| 120: | hole injection layer | 130: | hole transport layer |
| 140: | emission layer | 150: | electron transport layer |
| 160: | electron injection layer | 170: | second electrode |
| 180: | layer for improving light efficiency | 210: | buffer layer |
| 220: | emission-auxiliary layer | 320: | first hole injection layer |
| 330: | first hole transport layer | 340: | first emission layer |
| 350: | first electron transport layer | 360: | first charge-generation layer |
| 361: | second charge-generation layer | 420: | second hole injection layer |
| 430: | second hole transport layer | 440: | second emission layer |
| 450: | second electron transport layer | CGL: | charge generation layer |
| ST1: | first stack | ST2: | second stack |

## Claims

1. A compound of Formula 1: 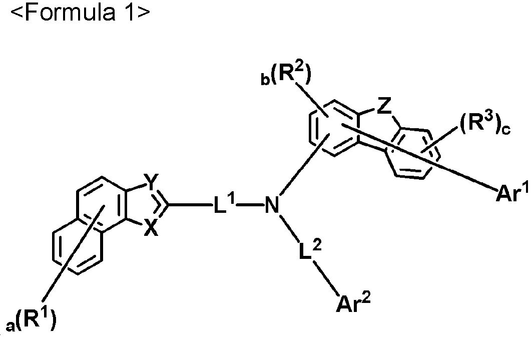 wherein,
one of X and Y is N, and the other is O or S,
Z is O or S,
Ar¹ and Ar² are each independently selected from the group consisting of a C₆-C₆₀ aryl group, a fluorenyl group, C₂-C₆₀ heterocyclic group containing at least one heteroatom of O, N, S, Si and P, a C₃-C₆₀ aliphatic ring group, a fused ring of a C₆-C₆₀ aromatic ring and a C₃-C₆₀ aliphatic ring, and a C₁-C₂₀ alkyl group,
R¹ to R³ are each independently selected from the group consisting of hydrogen, deuterium, halogen, a cyano group, a nitro group, a C₆-C₆₀ aryl group, a fluorenyl group, a C₂-C₆₀ heterocyclic group containing at least one heteroatom of O, N, S, Si and P, a C₃-C₆₀ aliphatic ring group, a fused ring of a C₆-C₆₀ aromatic ring and a C₃-C₆₀ aliphatic ring, a C₁-C₂₀ alkyl group, a C₂-C₂₀ alkenyl group, a C₂-C₂₀ alkynyl group, a C₁-C₂₀ alkoxyl group, and a C₆-C₆₀ aryloxy group, and adjacent groups may be bonded to each other to form a ring,
a is an integer of 0 to 6, b is an integer of 0 to 3, c is an integer of 0 to 4,
L¹ is selected from the group consisting of a C₆-C₆₀ arylene group, a fluorenylene group, a C₂-C₆₀ heterocyclic group containing at least one heteroatom of O, N, S, Si and P, a C₃-C₆₀ aliphatic ring group, and a fused ring of a C₆-C₆₀ aromatic ring and a C₃-C₆₀ aliphatic ring,
L² is selected from the group consisting of a single bond, a C₆-C₆₀ arylene group, a fluorenylene group, a C₂-C₆₀ heterocyclic group containing at least one heteroatom of O, N, S, Si and P, a C₃-C₆₀ aliphatic ring group, and a fused ring of a C₆-C₆₀ aromatic ring and a C₃-C₆₀ aliphatic ring, and
the aryl group, the arylene group, the fluorenyl group, the fluorenylene group, the heterocyclic group, the aliphatic ring group, the fused ring, the alkyl group, the alkenyl group, the alkynyl group, the alkoxyl group, the aryloxyl group, and the ring formed by adjacent groups may be each substituted with one or more substituents selected from the group consisting of deuterium, halogen, a silane group unsubstituted or substituted with a C₁-C₂₀ alkyl group or a C₆-C₂₀ aryl group, a phosphine oxide substituted or unsubstituted with a C₁-C₂₀ alkyl group or a C₆-C₂₀ aryl group, a cyano group, a nitro group, C₁-C₂₀ alkylthio group, C₁-C₂₀ alkoxy group, C₆-C₃₀ aryloxy group, C₆-C₃₀ arylthio group, a C₁-C₂₀ alkyl group, a C₂-C₂₀ alkenyl group, a C₂-C₂₀ alkynyl group, a C₆-C₃₀ aryl group, a C₆-C₃₀ aryl group substituted with deuterium, a C₃-C₃₀ aliphatic ring, a fused ring of a C₆-C₃₀ aromatic ring and a C₃-C₃₀ aliphatic ring, and a C₂-C₃₀ heterocyclic group comprising at least one heteroatom selected from the group consisting of O, N, S, Si and P, the adjacent substituents may be bonded to each other to form a ring, and hydrogen of the substituents may be replaced with deuterium.

2. The compound of claim 1, wherein Formula 1 is represented by one of Formula 1-1 to Formula 1-8: 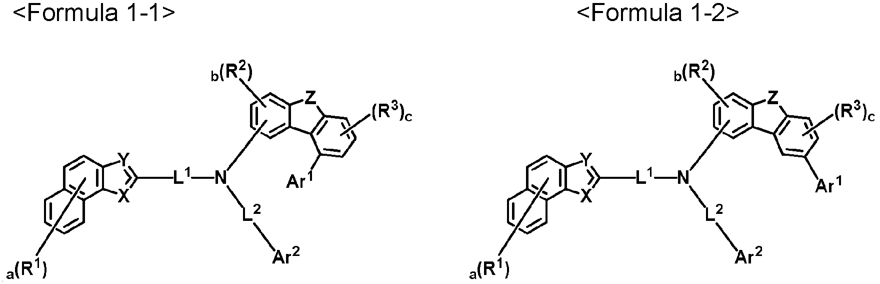 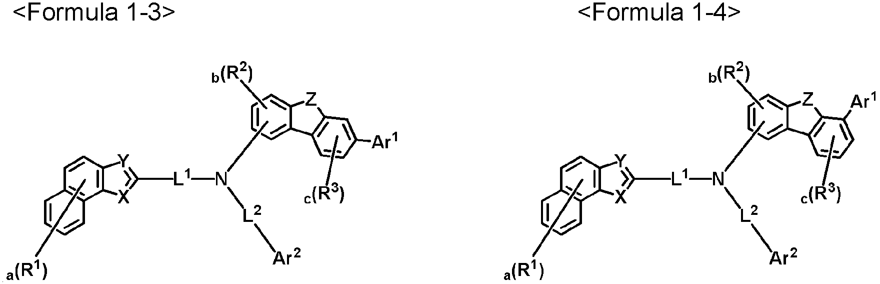 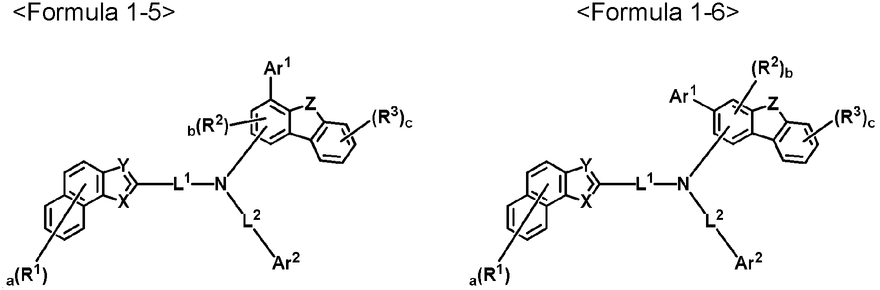 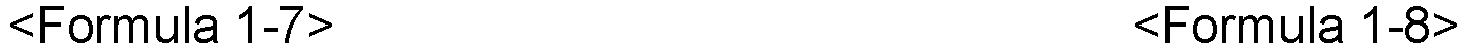 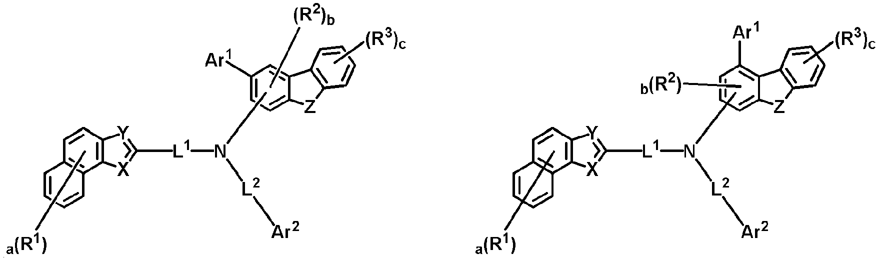 in Formula 1-1 to Formula 1-8, X, Y, Z, R¹ to R³, L¹, L², Ar¹, Ar², a are the same as defined in claim 1, in Formula 1-1 to Formula 1-4, b and c are each an integer of 0 to 3, and in Formula 1-5 to Formula 1-8, b is an integer of 0 to 2, c is an integer of 0 to 4.

3. The compound of claim 1, wherein at least one of L¹ or L² is selected from the group consisting of Formula a-1 to Formula a-12: 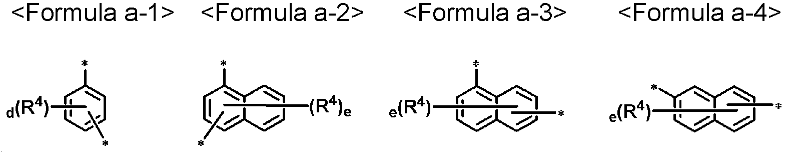 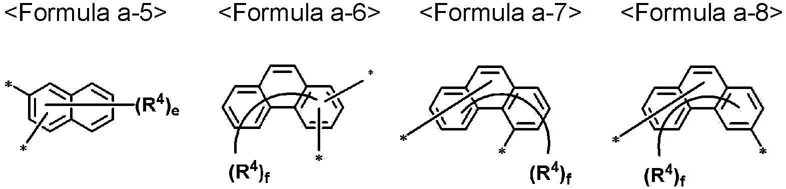 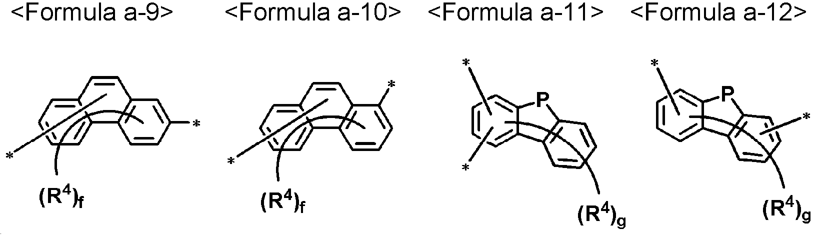 in Formula a-1 to Formula a-12,
P is O or S,
R⁴ is selected from the group consisting of hydrogen, deuterium, halogen, a silane group unsubstituted or substituted with a C₁-C₂₀ alkyl group or a C₆-C₂₀ aryl group, a phosphine oxide substituted or unsubstituted with a C₁-C₂₀ alkyl group or a C₆-C₂₀ aryl group, a cyano group, a nitro group, C₁-C₂₀ alkylthio group, C₁-C₂₀ alkoxy group, C₆-C₃₀ aryloxy group, C₆-C₃₀ arylthio group, a C₁-C₂₀ alkyl group, a C₂-C₂₀ alkenyl group, a C₂-C₂₀ alkynyl group, a C₆-C₃₀ aryl group, a C₆-C₃₀ aryl group substituted with deuterium, a C₃-C₃₀ aliphatic ring, a fused ring of a C₆-C₃₀ aromatic ring and a C₃-C₃₀ aliphatic ring, and a C₂-C₃₀ heterocyclic group comprising at least one heteroatom selected from the group consisting of O, N, S, Si and P, the adjacent groups may be bonded to each other to form a ring, and
d is an integer of 0 to 4, e and g are each an integer of 0 to 6, f is an integer of 0 to 8.

4. The compound of claim 1, wherein at least one of Ar¹ and Ar² is selected from the group consisting of Formula b-1 to Formula b-11: 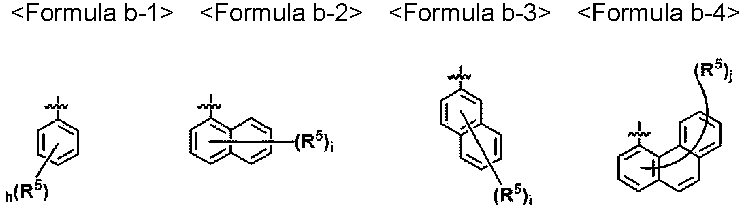 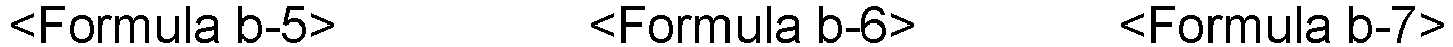 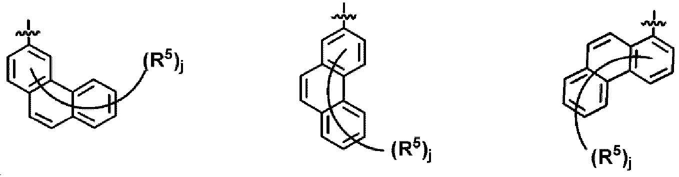 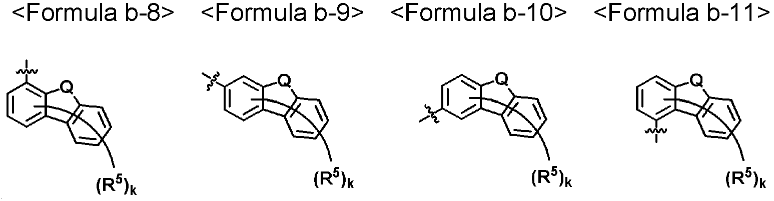 in Formula b-1 to Formula b-11,
Q is O or S,
R⁵ is selected from the group consisting of hydrogen, deuterium, halogen, a silane group unsubstituted or substituted with a C₁-C₂₀ alkyl group or a C₆-C₂₀ aryl group, a phosphine oxide substituted or unsubstituted with a C₁-C₂₀ alkyl group or a C₆-C₂₀ aryl group, a cyano group, a nitro group, C₁-C₂₀ alkylthio group, C₁-C₂₀ alkoxy group, C₆-C₃₀ aryloxy group, C₆-C₃₀ arylthio group, a C₁-C₂₀ alkyl group, a C₂-C₂₀ alkenyl group, a C₂-C₂₀ alkynyl group, a C₆-C₃₀ aryl group, a C₆-C₃₀ aryl group substituted with deuterium, a C₃-C₃₀ aliphatic ring, a fused ring of a C₆-C₃₀ aromatic ring and a C₃-C₃₀ aliphatic ring, and a C₂-C₃₀ heterocyclic group comprising at least one heteroatom selected from the group consisting of O, N, S, Si and P, the adjacent groups may be bonded to each other to form a ring, and
h is an integer of 0 to 5, i and k are each an integer of 0 to 7, j is an integer of 0 to 9.

5. The compound of claim 1, wherein the compound represented by Formula 1 is one of the following compounds:

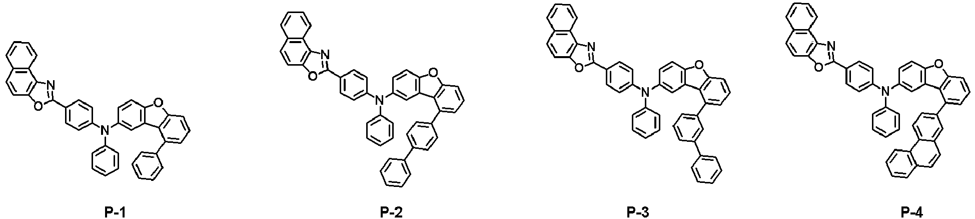

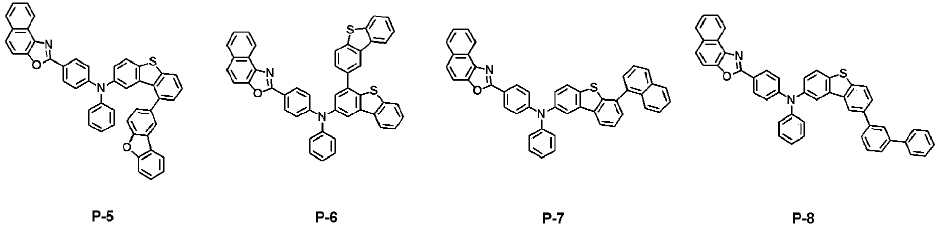

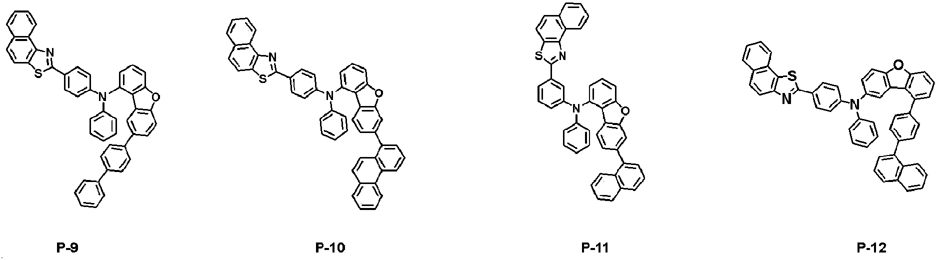

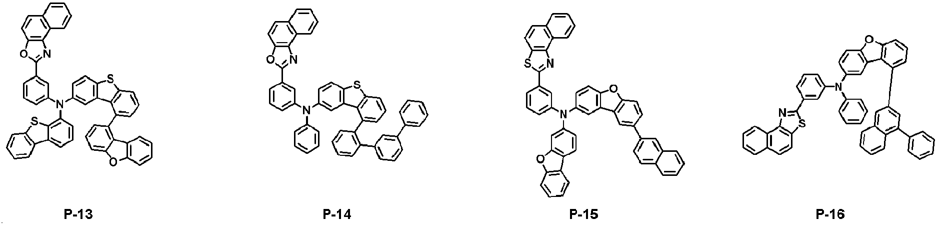

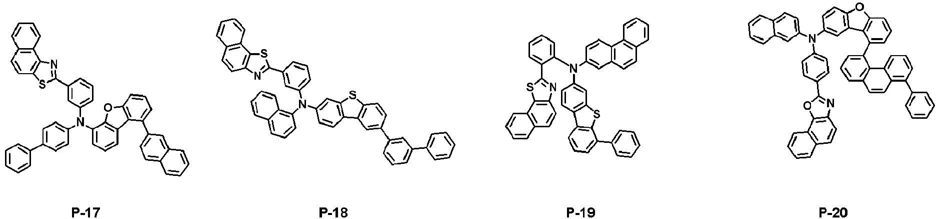

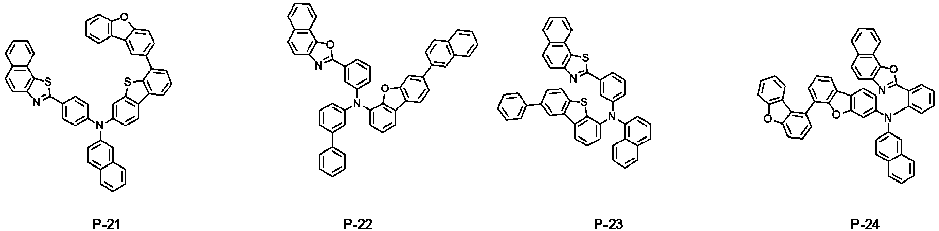

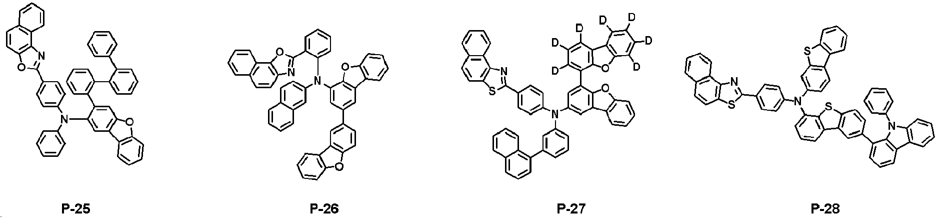

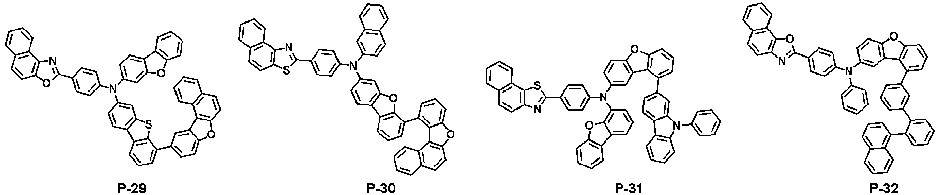

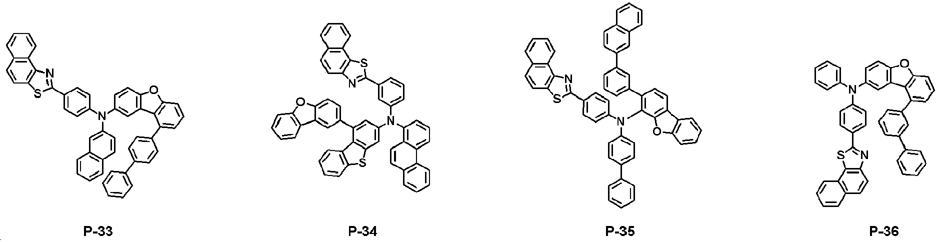

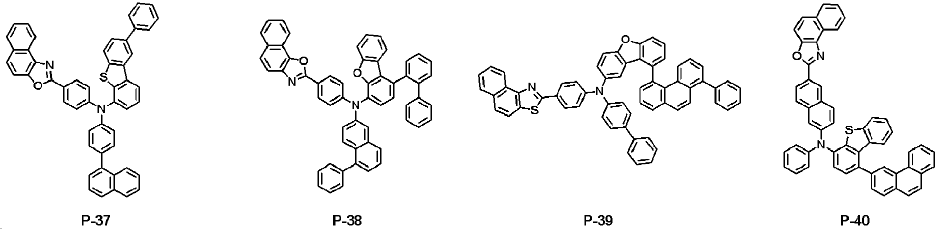

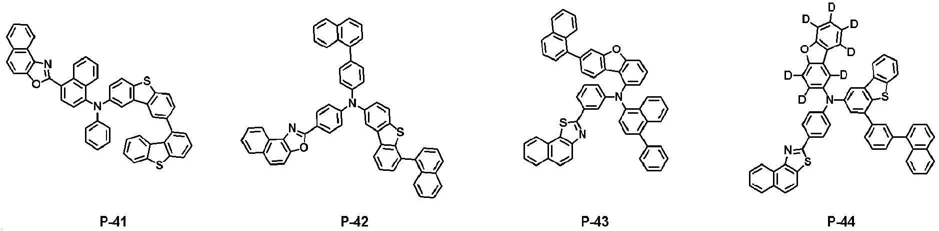

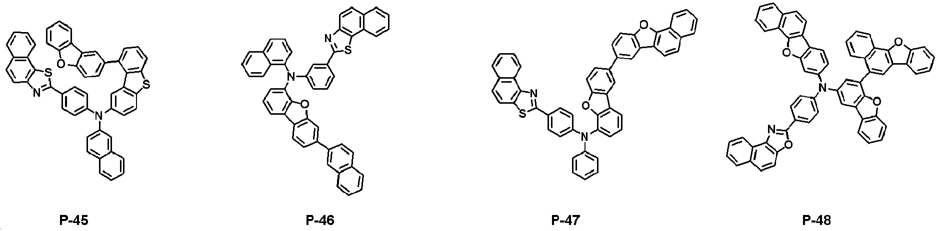

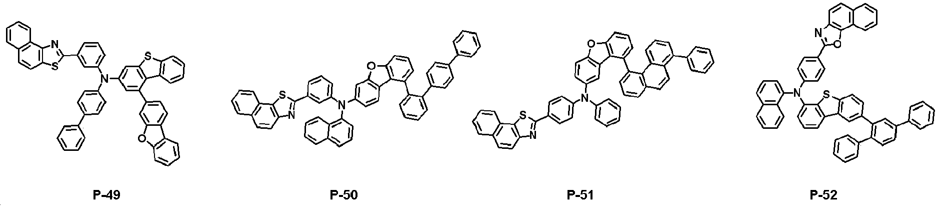

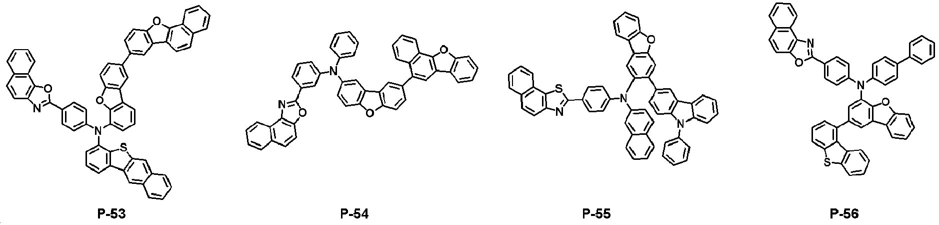

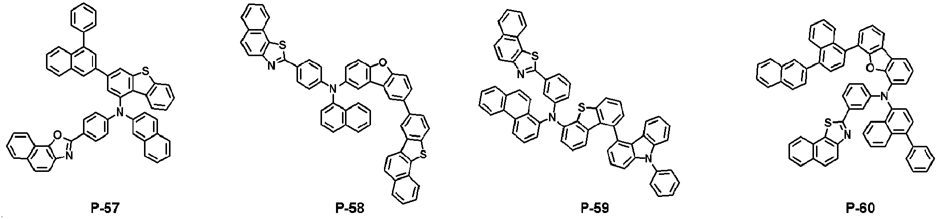

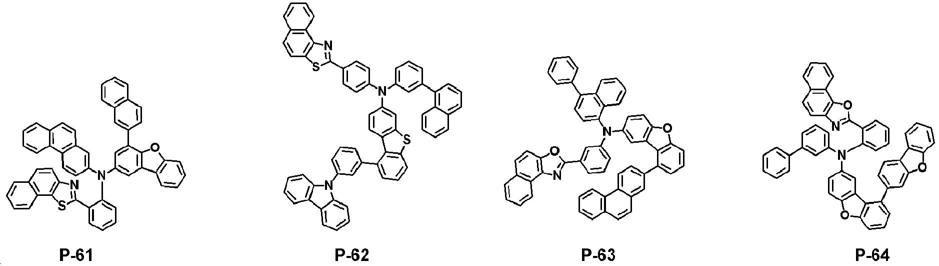

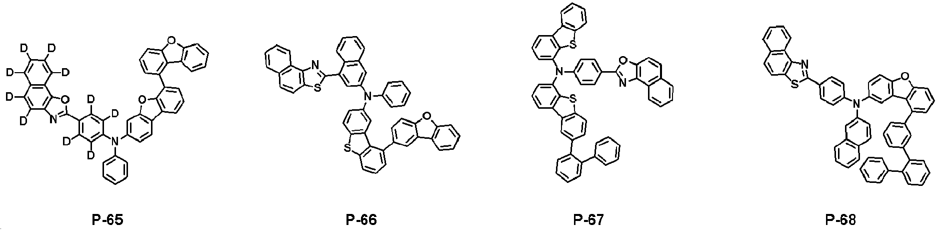

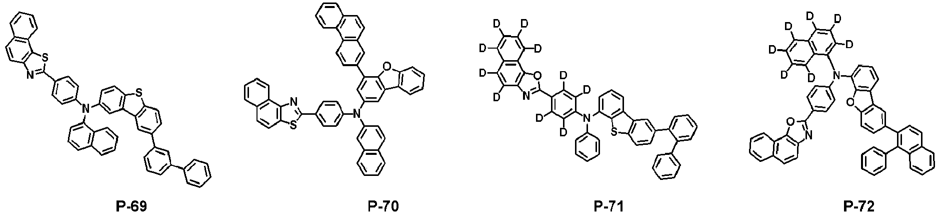

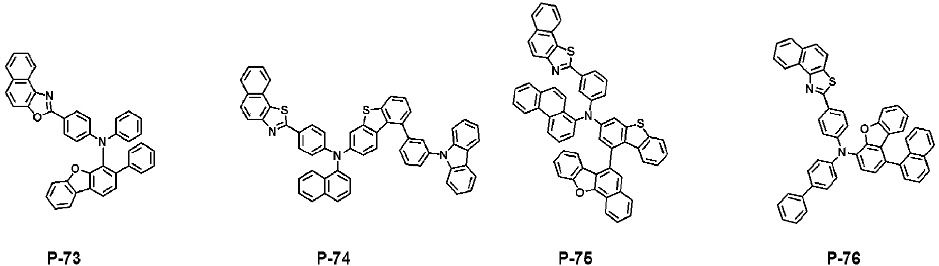

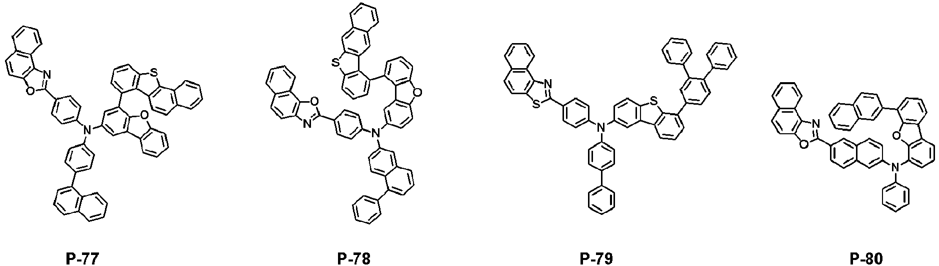

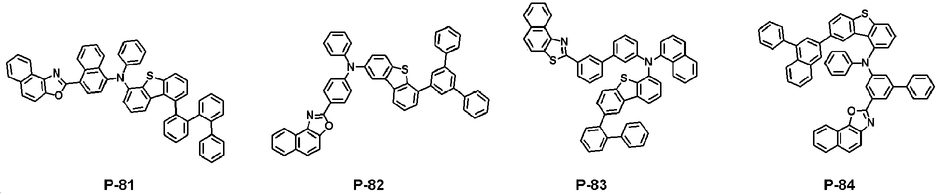

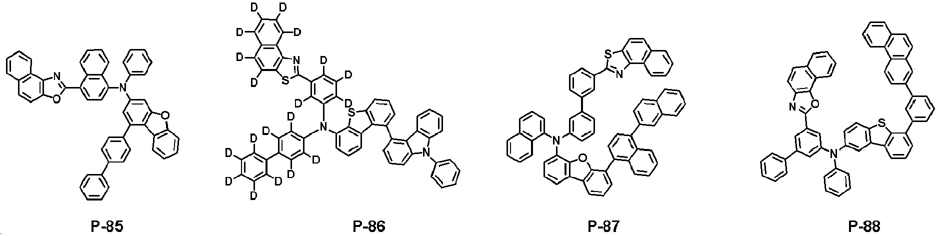

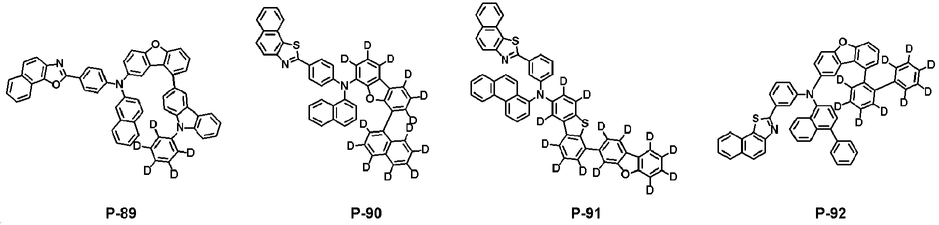

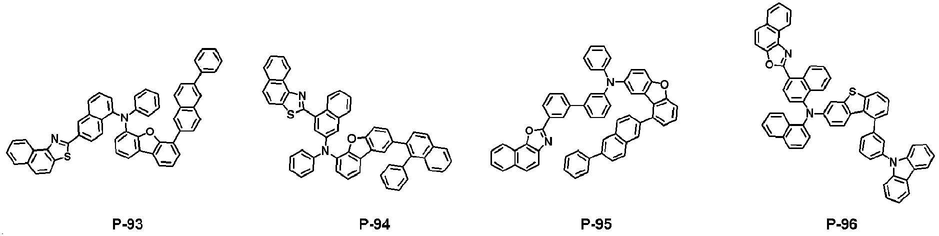

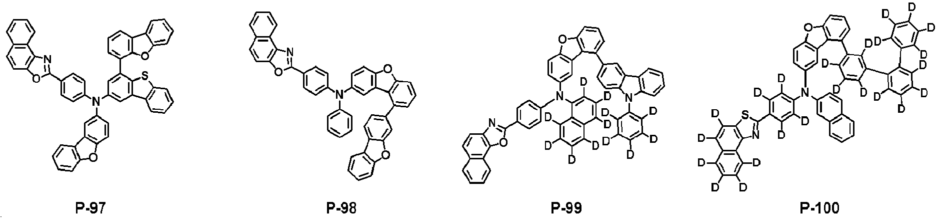

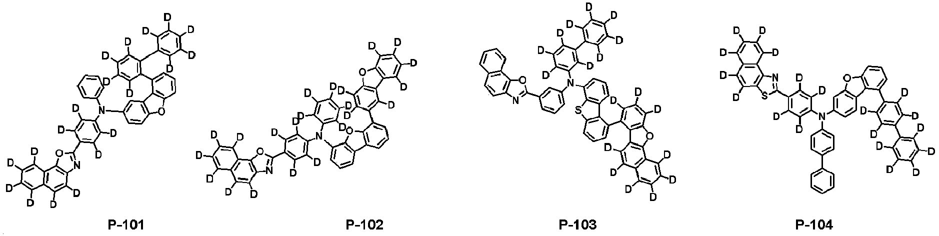

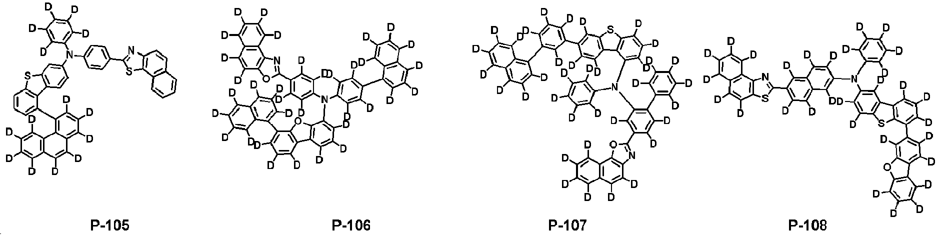

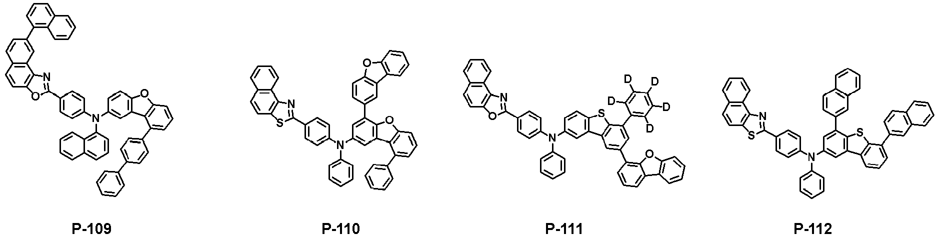

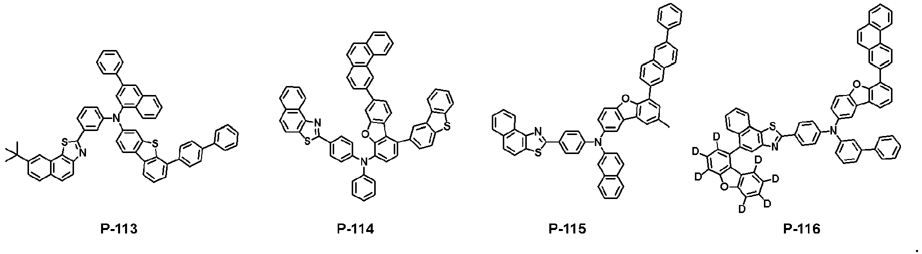

6. A material for an organic electronic device comprising the compound of claim 1 and a compound represented by Formula I: 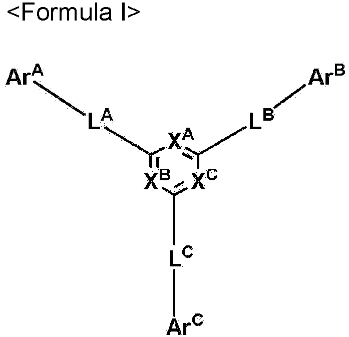 wherein,
X^{A} to X^{C} are each N or C(R'), and at least two of them are N,
Ar^{A} to Ar^{C} are each independently selected from the group consisting of a C₆-C₆₀ aryl group, a fluorenyl group, C₂-C₆₀ heterocyclic group containing at least one heteroatom of O, N, S, Si and P, a C₃-C₆₀ aliphatic ring group, a fused ring of a C₃-C₆₀ aliphatic ring and a C₆-C₆₀ aromatic ring, and a C₁-C₃₀ alkyl group,
L^{A} to L^{C} are each independently selected from the group consisting of a single bond, a C₆-C₆₀ arylene group, a fluorenylene group, a C₂-C₆₀ heterocyclic group containing at least one heteroatom of O, N, S, Si and P, a C₃-C₆₀ aliphatic ring group, and a fused ring of a C₃-C₆₀ aliphatic ring and a C₆-C₆₀ aromatic ring,
R' is selected from the group consisting of hydrogen, deuterium, halogen, a cyano group, a nitro group, a C₆-C₆₀ aryl group, a fluorenyl group, a C₂-C₆₀ heterocyclic group containing at least one heteroatom of O, N, S, Si and P, a C₃-C₆₀ aliphatic ring group, a fused ring of a C₆-C₆₀ aromatic ring and a C₃-C₆₀ aliphatic ring, a C₁-C₂₀ alkyl group, a C₂-C₂₀ alkenyl group, a C₂-C₂₀ alkynyl group, a C₁-C₂₀ alkoxyl group, and a C₆-C₆₀ aryloxy group, and
the aryl group, the arylene group, the fluorenyl group, the fluorenylene group, the heterocyclic group, the aliphatic ring group, the fused ring, the alkyl group, the alkenyl group, the alkynyl group, the alkoxyl group, and the aryloxyl group may be each substituted with one or more substituents selected from the group consisting of deuterium, halogen, a silane group unsubstituted or substituted with a C₁-C₂₀ alkyl group or a C₆-C₂₀ aryl group, a phosphine oxide substituted or unsubstituted with a C₁-C₂₀ alkyl group or a C₆-C₂₀ aryl group, a cyano group, a nitro group, C₁-C₂₀ alkylthio group, C₁-C₂₀ alkoxy group, C₆-C₃₀ aryloxy group, C₆-C₃₀ arylthio group, a C₁-C₂₀ alkyl group, a C₂-C₂₀ alkenyl group, a C₂-C₂₀ alkynyl group, a C₆-C₃₀ aryl group, a fluorenyl group, a C₃-C₃₀ aliphatic ring, a fused ring of a C₆-C₃₀ aromatic ring and a C₃-C₃₀ aliphatic ring, and a C₂-C₃₀ heterocyclic group comprising at least one heteroatom selected from the group consisting of O, N, S, Si and P, the adjacent substituents may be bonded to each other to form a ring, and hydrogen of the substituents may be replaced with deuterium.

7. The material of claim 6, wherein at least one of Ar^{A} to Ar^{C} is selected from the group consisting of Formula Ar-a to Formula Ar-d: 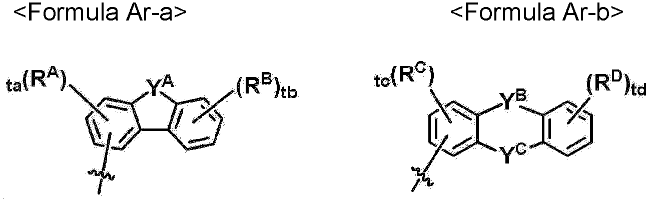 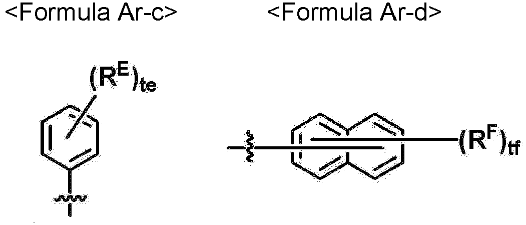 in Formula Ar-a to Formula Ar-d,
Y^{A} to Y^{C} are each independently O, S, C(R₁)(R₂) or N(Ar₁),
R^{A} to R^{F}, R₁ and R₂ are each independently selected from the group consisting of hydrogen, deuterium, halogen, a silane group unsubstituted or substituted with a C₁-C₂₀ alkyl group or a C₆-C₂₀ aryl group, a phosphine oxide substituted or unsubstituted with a C₁-C₂₀ alkyl group or a C₆-C₂₀ aryl group, a cyano group, a nitro group, C₁-C₂₀ alkylthio group, C₁-C₂₀ alkoxy group, C₆-C₃₀ aryloxy group, C₆-C₃₀ arylthio group, a C₁-C₂₀ alkyl group, a C₂-C₂₀ alkenyl group, a C₂-C₂₀ alkynyl group, a C₆-C₃₀ aryl group, a fluorenyl group, a C₃-C₃₀ aliphatic ring, a fused ring of a C₆-C₃₀ aromatic ring and a C₃-C₃₀ aliphatic ring, and a C₂-C₃₀ heterocyclic group comprising at least one heteroatom selected from the group consisting of O, N, S, Si and P, and adjacent groups may be bonded to each other to form a ring,
Ar¹ is selected from the group consisting of a C₁-C₂₀ alkyl group, a C₆-C₃₀ aryl group, a fluorenyl group, a C₃-C₃₀ aliphatic ring, a fused ring of a C₆-C₃₀ aromatic ring and a C₃-C₃₀ aliphatic ring, and a C₂-C₃₀ heterocyclic group comprising at least one heteroatom selected from the group consisting of O, N, S, Si and P,
ta and tc are each an integer of 0 to 3, tb and td are each an integer of 0 to 4, te is an integer of 0 to 5, tf is an integer of 0 to 7, and
R^{A} to R^{F}, R₁, R₂, Ar¹ may be each substituted with one or more substituents selected from the group consisting of deuterium, halogen, a silane group unsubstituted or substituted with a C₁-C₂₀ alkyl group or a C₆-C₂₀ aryl group, a phosphine oxide substituted or unsubstituted with a C₁-C₂₀ alkyl group or a C₆-C₂₀ aryl group, a cyano group, a nitro group, C₁-C₂₀ alkylthio group, C₁-C₂₀ alkoxy group, C₆-C₃₀ aryloxy group, C₆-C₃₀ arylthio group, a C₁-C₂₀ alkyl group, a C₂-C₂₀ alkenyl group, a C₂-C₂₀ alkynyl group, a C₆-C₃₀ aryl group, a fluorenyl group, a C₃-C₃₀ aliphatic ring, a fused ring of a C₆-C₃₀ aromatic ring and a C₃-C₃₀ aliphatic ring, and a C₂-C₃₀ heterocyclic group comprising at least one heteroatom selected from the group consisting of O, **N,** S, Si and **P,** the adjacent substituents may be bonded to each other to form a ring, and hydrogen of the substituents may be replaced with deuterium.

8. An organic electronic device comprising a first electrode, a second electrode, and an organic layer between the first electrode and the second electrode, wherein the organic layer comprises the compound of claim 1 or the material for an organic electronic device of claim 6.

9. The organic electronic device of claim 8, wherein the organic layer comprises an emission layer, and the emission layer comprises the compound of claim 1 or the material for an organic electronic device of claim 6.

10. The organic electronic device of claim 8, wherein the organic layer comprises two or more stacks, and the two or more stacks each comprise a hole transport layer, an emission layer and an electron transport layer formed sequentially on the first electrode.

11. An electronic apparatus comprising a display device and a control unit configured to drive the display device, wherein the display device comprises the organic electronic device of claim 8.

12. A method for recovering a compound represented by Formula 1 comprising:
a step of depositing material of an organic layer comprising a compound represented by Formula 1 of claim 1;
a step of recovering the material of organic layer attached to the deposition equipment; and
a step of purifying the recovered material of an organic layer to obtain a compound represented by Formula 1 having a purity of 99.9% or higher.
